# EUROPEAN PATENT APPLICATION

(11) **EP 0 922 767 A1**
(43) Date of publication of application: **16.06.1999**
(21) Application number: 97402925.8
(22) Date of filing: 03.12.1997
(51) Int. Cl.: C12N 15/82, C12N 9/00, A01H 5/00, C07H 21/02

(54) **Ribozymes capable of conferring resistance to potyvirus infection, and plants expressing said ribozymes**

(71) Applicant: Gene Shears Pty Limited, Canberra, ACT 2601 (AU)
(72) Inventor: Huttner, Eric, 91450 Soisy sur Seine (FR); Tucker, William, Turner, ACT 2601 (AU); Vermeulen, Agnès, 26000 Valence (FR); Ignart, Frédéric, 26800 Beauvallon (FR)
(74) Representative: Almond-Martin, Carol

(57) **Abstract**

The invention relates to a nucleic acid molecule, called 〈〈 ribozyme 〉〉, having endoribonuclease activity, comprising at least one hybridising region and at least one catalytic region, said hybridising region being complementary to one or more target regions of the genomic (+) RNA of a potyvirus, and/or to one or more target regions of the replicative (-) strand RNA of a potyvirus, with the proviso that the said target region does not consist of the GUC triplet located at position 8122-8124 of the PVYn strain or the equivalent triplet of other potyviruses.

## Description

The present invention relates to a class of 〈〈 ribozymes 〉〉, i.e. nucleic acid molecules having endoribonuclease activity, which are capable of conferring on plants, resistance to infection by potyviruses. The invention also relates to plants and plant cells expressing the ribozymes, and showing resistance or tolerance to potyvirus infection.

Potyviruses belong to the picorna-like supergroup of viruses, and within this supergroup, to the family of the potyviridae. Potyviruses are mostly aphid-, transmitted, although some are transmitted by mites, whiteflies or fungi. They and are one of the world's most widespread plant pathogens, causing especially severe disease in cucurbits in many countries.

Potyviruses include : Zucchini yellow mosaic virus (ZYMV), Watermelon mosaic virus 2 (WMV2), Tobacco vein mottling virus (TVMV), Tobacco etch virus (TEV), Potato virus Y(PVY), Pepper mottle virus (PepMoV), Soyabean mosaic virus (SbMV), Papaya ringspot virus (PRSV), Pea seed-borne mosaic virus (PSbMV), Turnip mosaic virus (TuMV), Johnson grass mosaic virus (JGMV), Plum pox virus (PPV, Maize Dwarf Virus (MDV), Bean common mosaic virus (BCMV), Bean yellow mosaic Virus (BYMV), Carnation vein mottle (CarVMV), Celery mosaic virius (CeMV), Clover yellow vein virus (ClYVV), Henbane mosaic virus (HMV), Lettuce mosaic virus (LtMV), Sugarcane mosaic (SCMV).

Potyviruses regarded as strains of the above potyviruses, or of uncertain relationship to the above viruses, and included in the definition of 〈〈 Potyviruses 〉〉 for the purposes of the present invention, include : Clover mosaic virus, Datura mosaic virus, Desmodium mosaic virus, Freesia mosaic virus, Mungbean mosaic virus, Passionfruit ringspot virus, Pea necrosis virus, Sweet potato A virus, Wild potato mosaic virus. Possible members of the Potyvirus family include : Carrot mosaic virus, Celery latent virus, Palm mosaic virus and fern virus.

Of these viruses, WMV2, ZYMV and PRSV are responsible for severe yield reduction in Cucurbit crops. Complete resistance to infection by potyviruses, rarely occurs in economically important crops, although tolerance, in which plants are susceptible to infection but either remain symptomless or only slowly develop inconspicuous symptoms and often support restricted virus replication, is more common.

The establishment of a means to impart resistance to potyvirus infection and/or disease development would clearly have considerable economic importance.

In addition to breeding for resistance, a number of approaches using genetic engineering have been explored in an attempt to provide resistance to potyviruses.

For example, Van der Vlugt (Plant Molecular Biology, 1992, vol. 20 : 631-639) produced transgenic tobacco plants expressing both translatable and non-translatable Coat Protein (CP) RNA from PVY. Full protection (i.e completely free of systemically spread virus) following mechanical inoculation of transformed tobacco plant was observed for some plants, whether or not the CP RNA was translated. The work described did not extend to testing for resistance against inoculation by aphids, or against other related and unrelated potyviruses.

Immunity to infection by ZYMV in transgenic muskmelon plants expressing the full-length ZYMV CP gene has been reported by Fang and Grumet (Mol. Plant-Microbe Interactions, 1993, vol. 6 n°3 : 358-367). Protection was also exhibited by plants expressing either a truncated, conserved core of the CP gene, or CP antisense RNA, although these constructs were reported to be less efficient then the full-length sense CP gene. These results are in contrast to those obtained by Lindbo and Dougherty (Mol. Plant-Microbe Interactions, 1992, vol. 5 : 144-153) where expression of antisense and translationally deficient sense TEV CP RNA resulted in much higher protection against TEV infection than that conferred by expression of the sense version of the full-length CP gene. The effectiveness of coat protein and antisense techniques in obtaining potyvirus resistance therefore appears to have met with varying success depending upon the precise constructs and viruses involved.

Another approach has been described by Van der Vlugt et al (Virus Research, 1993, vol. 27 : 185-200) who synthesized two ribozymes against a relatively conserved region of the RNA-dependent, RNA-polymeraseencoding cistron (also known as the NIb cistron) of PVY. The target triplet of these ribozymes is GUC at position 8122-8124 of PVYn as described in Robaglia (1989). These ribozymes were tested in vitro against an artificial PVY-specific target RNA. Whilst the 〈〈 long-arm 〉〉 ribozyme (total arm length 106 bases) cleaved the substrate RNA efficiently, the 〈〈 short-arm 〉〉 ribozyme (total arm length 19 bases) failed to cleave. This short-arm ribozyme was however capable of cleaving a much shorter PVY specific RNA transcript. The authors conclude that the cleavage activity of the ribozyme is in part determined by the substrate involved, long substrate RNA's such as full-length viral RNA's being likely to possess extensive secondary structure which prevents the formation of the correct ribozyme hammerhead structure and thus prevents cleavage. No in vivo tests are described for these ribozymes.

The technical problem underlying the present invention is to provide a means for conferring protection, on plants, to potyvirus infection, or disease development. Advantageously, the means provide protection to a large number of host plants against a particular potyvirus and to serologically related potyviruses, preferably in the heterozygous state.

The present invention provides a solution to the above technical problem by providing nucleic acid molecules or 〈〈 ribozymes 〉〉 having endoribonuclease activity which are designed to specifically cleave full-length potyviral RNA, (either (+) sense genomic RNA and/or the (-) sense replicative strand RNA).

The ribozymes of the present invention may comprise single or multiple catalytic regions (〈〈 mono-〉〉 or 〈〈 poly- 〉〉 ribozymes).

Specifically the present invention relates to a nucleic acid molecule, called 〈〈 ribozyme 〉〉, having endoribonuclease activity, comprising at least one hybridising region and at least one catalytic region, said hybridising region being complementary to one or more target regions of the genomic (+) RNA of a potyvirus, and/or to one or more target regions of the replicative (-) strand RNA of A potyvirus, with the proviso that the said target region does not contain the GUC triplet located at position 8122-8124 of the PVYn strain or the equivalent triplet of other potyviruses.

The resistance provided by the ribozymes of the present invention is, for a number of reasons, unexpected on the basis of previous knowledge. Specifically, it has been shown both by the present inventors and by other authors (Edington and Nelson, Gene Regulation - Biology of Antisense RNA and DNA : Ed. Erickson and Izant, Raven Press Ltd. New York, 1992), that in-vitro cleavage results obtained with ribozymes are not always predictive of in-vivo situations. In vitro data obtained by Van der Vlugt (1993 supra) can therefore not be reliably extrapolated to in vivo situations. Furthermore, use of ribozymes on full-length viral RNA could not be expected to work efficiently in view of the secondary structure associated with such long RNA, as pointed out by Van der Vlugt et al (1993 et al supra).

Moreover, some authors have postulated that the (+) strand RNA may be protected by a protein coat during the early stages of infection (〈〈 stripeosome 〉〉 hypothesis) until replication, and therefore would not be an appropriate target for ribozymes.

Many of the present ribozymes give rise to resistance in the heterozygous state, thereby facilitating transmission of the resistance characteristic to progeny of transgenic plants.

In addition, the polymerlc ribozymes of the invention are capable of conferring resistance in vivo to virus strains related to, but different from the particular targeted strains.

Finally, many of the polymeric ribozymes of the invention target sites which are either on different strands of RNA (i.e (+) and (-)), or have sites which are widely separated regions on the same strand. The ribozymes thus do not only act as a single molecule simultaneously targeting a plurality of sites, but rather can act as if the molecule were a plurality of monoribozymes each targeting a single site, thus increasing the probability that the substrate will be cleaved.

In the context of the present invention, the following terms have the following meanings : Potyvirus : A large group of PLANT VIRUSES in which the virions are flexuous filaments (ca. 680-900 x 11 mm) comprising one type of coat protein (MWt ca. 32,000-36,000) and one molecule of linear position-sense ssRNA (MWt ca. 3.0-3.5 x 10⁶) comprising a single large open reading frame (ORF) coding for a polyprotein of ca. 350.000 Da which is proteolytically processed by virally encoded proteinases.

As a group, the viruses infect a wide range of host plants, including monocots and dicots, but individual members typically have a narrow host range. Symptoms depend on plant cultivar, virus strain, and environmental conditions, but typically include mosaic or mottle in the leaves, colour-breaking in flowers, mottling and/or distortion in the fruits ; characteristic proteinaceous inclusion bodies (serologically unrelated to virus coat protein) are formed in the cytoplasm of infected plant cells, appearing as 〈〈 pinwheels 〉〉 in transverse section or as 〈〈 bundles 〉〉 in longitudinal section. A few members also form crystalline nuclear inclusions. Potyviruses are transmitted (non-persistently) by aphids and can be transmitted mechanically ; some viruses tentatively included in the group are transmitted by whitefly, mites or fungi.

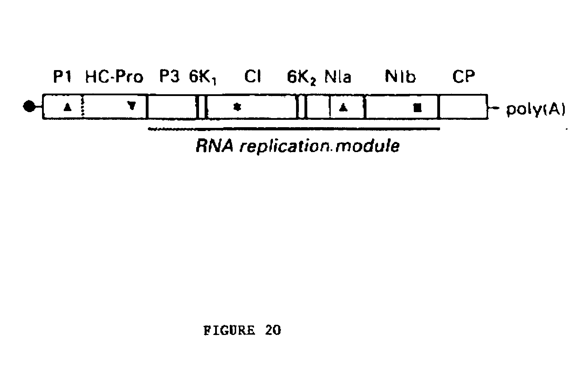

The replication cycle of potyviruses involves the following successive steps ; infection, uncoating, translation, polyprotein processing to yield functional, viral coded products, genome replication via a negative strand RNA template, progeny virus particle assembly, cell-to-cell movement within the infected plant and vector transmission from plant to plant.
Gene : In the context at potyviral genomic RNA, the term 〈〈 gene 〉〉 as used herein is synomynous with 〈〈 cistron 〉〉. All potyviral proteins are generated from a single large primary translation product (polyprotein) by viral proteases, produced by translation of the single genomic ORF. In the present context, 〈〈 gene 〉〉 signifies a region of the genomic ORF coding for a protein released as an independent molecule by viral proteases. Potyviral 〈〈 genes 〉〉 or cistrons are referred to herein by reference to the designation accepted in the art for the type members of the potyviruses family PVY as shown in figure 20, or their functional equivalent in other members of the family.
(+) sense RNA : In the context of potyviral RNA, 〈〈 (+) sense RNA 〉〉 signifies the genomic RNA of the virus as contained within the virion, which serves directly as a template for translation of a polyprotein. The (+) sense RNA may be encapsidated or not. Naked (+) sense RNA is also infectious.
Replicative or (-) sense RNA: In the context of potyviral RNA, 〈〈 (-) sense or replicative RNA 〉〉 signifies the full-length, non-coding negative strand template RNA produced by the copying of the entire (+) RNA genome by a viral polymerase (NIb). No sub-genomic RNAs are produced in potyviruses. In the context of the present invention, positions of nucleotides in the viral (-) sense RNA are indicated by reference to the corresponding position of the (+) strand, with a (-) in brackets. Reference to genes or cistrons in the (-) strand (which is in fact non-coding) signifies a region or a site in the (-) strand corresponding to that region or site in the (+) strand.
Potyviral RNA : In the present context, this expression encompasses both (+) sense genomic RNA and (-) sense replicative RNA.
Resistance :reduction or prevention of viral infection e.g. absence of symptoms and absence of detectable viral particles, absence or reduction or delay of apparition of symptoms. Symptoms are sometimes atypical. Recovery possible.
Hybridising /complementary : the 〈〈 arms 〉〉 of the ribozyme are said to 〈〈 hybridise 〉〉 to a target RNA sequence or to be 〈〈 complementary 〉〉 to the target region in the potyvirus. Such hybridisation and complementarity is defined herein as an extent of duplex formation sufficient to form a stable enough structure so that cleavage adjacent to the target triplet can occur. Depending on the target sequence, the ribozyme and the secondary structure of the RNA, preferably less than about 5-10, more preferably less than about 3 to 5 and even more preferably less than 1 to 3 bases would be mismatched between the target RNA sequence and one or both hybridising arms of the ribozyme. Over the whole length of the hybridising arm, not more than 10% of mismatches should occur. Accordingly, the present invention extends to ribozymes described herein, to the polyribozymes containing them, and to any or all functional mutants, derivatives, parts, homologues or analogues thereof. Examples of such mutants include single or multiple nucleotide substitutions, deletions and/or additions to the hybridising regions and/or catalytic regions of the ribozymes provided such modified ribozymes are still functional in cleaving the target RNA sequence, and/or conferring resistance to polyviruses.

The present invention therefore extends to a ribozyme having one or a plurality of hybridising domains and catalytic domains, wherein the hybridising domain comprises a sequence of nucleotides substantially complementary to a sequence of nucleotides in a potyvirus RNA in vitro under high stringency conditions and wherein said catalytic region is capable of cleaving said target RNA sequence. This aspect of the present invention is conveniently determined by the extent of cleavage of the target RNA sequence in vitro.

For the purposes of the present invention, the stringency conditions set forth in Sambrook et al, Molecular Cloning : A laboratory Manual, Cold Spring Harbor laboratories, Cold Spring Harbor, NY, USA, pages 387-389 at paragraph 11 are considered high stringent conditions. Alternatively, medium stringency may be used involving 0.25-05 % w/v SDS at 45°C for 2-3 hours. The alternative conditions are applicable depending on concentration, purity and source of nucleic acid molecules.
Hybridising region : the total length of hybridizing arms.
Target region : In the context of potyviral RNA, 〈〈 target region 〉〉 signifies a stretch of ribonucleotides, normally having a length of from approximately 10 bases up to the length of the full potyviral RNA, to which the hybridising region of the ribozymes of the invention hybridise. The target region contains a precise target site at which cleavage can occur when acted upon by the ribozyme.
Target triplet : In the context of hammerhead ribozymes, 〈〈 target triplet 〉〉 signifies the three bases immediately 5' to the cleavage site in the substrate RNA. The target triplet will be noted herein as X°X°X° wherein X° 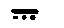 any ribonucleotide, preferably X°UX° wherein X° is any ribonucleotide and U is uracil, or X°UY wherein Y is adenine, cylosine or uracil.
Target sites in(-) sense RNA_: Ribozyme target sites in the potyviral (-) strand replicative RNA are designated by reference to the corresponding cistron (or 〈〈 gene 〉〉) in the (+) strand.

The potyviruses targeted by the ribozymes of the present invention are preferably :
- Zucchini yellow mosaic virus: (ZYMV),
- Watermelon mosaic virus 2: (WMV2),
- Tobacco vein mottling virus: (TVMV),
- Tobacco etch virus: (TEV),
- Potato virus Y: (PVY),
- Pepper mottle virus: (PepMoV),
- Soyabean mosaic virus: (SbMV),
- Papaya ringspot virus: (PRSV),
- Pea seed-borne mosaic virus: (PSbMV),
- Turnip mosaic virus: (TuMV),
- Johnson grass mosaic virus: (JGMV),
- Plum pox virus,: (PPV),
- Maize Dwarf Virus: (MDV).
Strains of ZYMV and WMV2 as well as serologically related viruses are particularly preferred.

Target regions of potyviral RNA which are particularly preferred are those cistrons (and the corresponding regions of (-) sense RNA) which give rise to proteins involved in viral processing, replication, and assembly such as NIa, NIb and CP. These cistrons are located in the 3'-half of the genomic RNA.

According to a preferred embodiment, the nucleic acid molecules of the invention comprise at least one unit 〈〈 R 〉〉 having the general formula :
3' [(X)ₙ - Q - (X')_{n'}]ₚ 5' wherein :
- X and X' each represent, independently, any ribonucleotide ; (X)ₙ and (X')_{n'} represent ribonucleotide sequences substantially complementary to at least part of said target region of potyvirus genomic (+) RNA or to at least part of its (-) strand replicative RNA ;
- n and n' are each equal to or greater than 4 ;
- Q represents the catalytic region of a ribozyme ;
- p ≥ 1.

In the above general formula (X)ₙ and (X')_{n'} represent the complementary sequences or 〈〈 arms 〉〉 of the ribozyme which hybridise to the chosen target region of the potyvirus, (X)ₙ and (X')_{n'} together representing the 〈〈 hybridizing region 〉〉 of the ribozyme.

The values of n and n' are, independently, at least 4, for example from 4 to 4000. Preferred values are from 4 to 50, for example 6 to 50 or 8 to 12 nucleotides for 〈〈 short arm 〉〉 ribozymes. For 〈〈 long arm 〉〉 ribozymes, n and n' normally have values greater than 50, for example 50 to 400, particularly 60 to 300 nucleotides.

In the above general formula, p = 1 for monoribozymes. For polyribozymes, p ≥ 2, for example 2-20 or 4-10. Polyribozymes are particularly preferred.

Preferably, the complementary sequences (X)ₙ and (X')_{n'} are complementary to target regions of potyvirus RNA which are conserved between strains of a given species, and preferably also between different species of potyvirus. In the context of the present invention, 〈〈 conserved 〉〉 signifies that the sequences on at least one side, and preferably on both sides of the cleavage site in the substrate are essentially identical over a length of 20 bases, i.e. have at least 85% identity, and preferably at least 90% identity over 20 bases. Generally speaking, in a conserved target region, the target triplet itself is identically conserved between two viruses. Exceptionally, since the third base in the target triplet X°X°X° remains unpaired after hybridisation of the ribozyme to the target region in the substrate, this base may vary between two viruses in a conserved region without affecting cleavage capacity.

According to yet a further embodiment of the invention, the hybridising arms of the polyribozyme may be derived from at least two different types of virus. Such polyribozymes are referred to hereinafter as 〈〈 chimeric polyribozymes 〉〉. As an example of this type of construction, mixtures of ZYMV and WMV2 derived sequences can be cited. This type of polyribozyme ensures the inactivation of both types of virus, if present together, or allows the use of a single construction in different geographical areas.

According to a preferred embodiment, the catalytic region 〈〈 Q 〉〉 in the above general formula is a hammerhead ribozyme catalytic region as described in European patent EP-B-0 321 201. Such a ribozyme can be represented as a molecule of formula (I): 
3'                                                    5'
(X)n Y                                    Y (X)n'
     A                                    C
     A                                    U
     G          'A                        C
     C  *       G              G          A
     |          |                   X
     Y'         Y''
 wherein, (X)ₙ and (X)_{n'} represent ribonucleotide sequences substantially complementary to an RNA sequence of a potyvirus (either genomic or replicative intermediate) ; an (*) represents a base pair between complementary ribonucleotides ; Y represents any ribonucleotide and each Y may be the same or different ; y' and Y'' represent ribonucleotides of complementary sequence along at least part of their length to allow base pairing between the oligoribonucleotides, or Y' and Y'' together form a single RNA sequence wherein at least part of said sequence comprises a stem formed by base pairing between complementary nucleotides ; and optionally, an additional nucleotide selected from any one of A, G, C or U is inserted after ¹A.

In another embodiment, the ribozyme is as set forth in formula (II) : 
3'                                                    5'
(X)nA                                    Y(X)n'
A                                        C
A                                          U
G              1A                            G
C      *    G        G                         A
Y      *    Y          Y
Y      *    Y
(Y)m   *    (Y)m'
Y           Y
       B
 wherein (X)ₙ and (X)ₙ' represent ribonucleotides sequences substantially complementary to an RNA of a potyvirus ; Y represents any ribonucleotide and each Y residue may be the same or different ; an (*) represents a base pair between complementary ribonucleotides ; n and n' are as previously defined ; B represents a bond, a base pair, a ribonucleotide, or an oligoribonucleotide containing at least 2 ribonocleotides ; m and m' are 1 or more and may be the same or different ; and optionally, an additional nucleotide selected from any one of A, G, C or U is inserted after ¹A.

In a preferred embodiment, the ribozyme is as set forth in formula (III): 
3'                                                    5'
(X)nA                                        Y(X)n'
A                                            C
A                                            U
G                    A                       G
C          *         G                  G         A
A          *         U                  U
G          *         C
G          *         C
A                         C
G                    U
 wherein (X)ₙ and (X)ₙ' represent a ribonucleotide sequence substantially complementary to an RNA of a potyvirus ; Y represents any ribonucleotide and each Y residue may be the same or different ; an (*) represents a base pair between complementary ribonucleotides ; n and n' are as previously defined.

Although the catalytic regions illustrated above have a conserved structure and sequence, it has been observed that some nucleotides may be deleted, inserted, substituted or modified without prejudice to the activity of the ribozyme. The invention comprises the use of these modified catalytic regions in the polyribozyme provided that their catalytic activity is conserved. This activity can be verified by using the tests described below.

For example, one or more nucleotides of the catalytic region II illustrated in formula I, II and III may be replaced by nucleotides containing bases such as adenine, guanine, cytosine, methylcytosine, uracil, thymine, xanthine, hypoxanthine, inosine or other methylated bases. The 〈〈 conserved 〉〉 bases C-G which together form the first base pair of the catalytic loop, can be replaced by U-A.

Other preferred variant hammerhead catalytic regions have the following sequences :
- Mutant ribozyme I :: cugaugaGUCCugaGGACgaa
- Mutant ribozyme II :: cugaugaGUCCcgugaGGACgaa
- Mutant ribozyme III :: cugaugaGUCgugagGACgaa
- Mutant ribozyme IV :: cugaugaGCGgugaCGCgaa.
- Wild type sTobRV :: cugaugaGUCCgugaGGACgaa (Helix II shown in upper case letters)
Because ribozymes act in a sequence-specific manner, it is possible for a target RNA to become resistant to ribozyme-mediated inactivation by just changing one or a few critical based. For the present application, it was also desirable to build resistance genes which would be protect plants against infection by several strains of a virus species. Both the goals of achieving a robust resistance gene , and obtaining protection against many strains, were achieved by selecting the target sites for the ribozymes according to the following criteria :
- sites are chosen which cannot easily mutate without changing the coding capacity (i.e. the amino acids corresponding to a particular codon) ;
- bites are chosen which are located in highly conserved regions.

In a preferred embodiment of the invention, the 3' (X)ₙ sequence of a given R unit and the 5' (X')_{n'} sequence of the adjacent R unit are complementary to sequences which are contiguous in the potyvirus genomic (+) strand RNA or replicative (-) strand RNA.

According to this variant, the complementary sequence may hybridise with the entire length of the target transcript. On the other hand, it may hybridise with only a portion or region of the targeted transcript. The portion or region in question must be long enough to allow the inclusion of at least 1 catalytic region in the corresponding sequence of the polyribozyme. In general, for this variant, the length of the complementary sequence, not counting the catalytic regions (the sum of the hybridising arms), may vary from about 4 to 4000 bases Lengths of 40 to 1000 are particularly preferred.

According to another highly preferred embodiment of the invention, in the polyribozyme having the formula :

R₁-R₂-R₃-------R_{n'}

the 3' hybridising arm (X')ₙ of a given 〈〈 R 〉〉 unit and the 5' hybridising arm (X')_{n'} of the adjacent 〈〈 R 〉〉 unit are complementary to non-contiguous portions of the genome or of the (-)sense RNA transcript. An example of this type of structure is the 〈〈 short arm polyribozyme 〉〉.

The structure of these non-contiguous polyribozymes can be thought of as a sequence complementary to the target sequence in which ribozyme catalytic regions are embedded and which has undergone deletions in the complementary sequence. According to this embodiment, the distance between two catalytic regions in the polyribozyme is normally shorter than the distance between the two corresponding X°X°X° sites in the target sequence. The distance between ribozymes can be reduced significantly with respect to the distance between the corresponding target sites in the substrate, for example, if two adjacent targeted X°UX° sites in the substrate are separated by a distance of 1000 nucleotides, the distance between the catalytic regions of the 〈〈 short arm ribozyme 〉〉 may be as little as 30 or 40 nucleotides. Reductions of over 90% of the length of the hybridising sequence may therefore be made. The minimum length between two adjacent catalytic regions is normally about 8 nucleotides and preferably at least 20. The present inventors have shown that such shortened hybridising sequences do not, surprisingly, prevent the polyribozyme from efficiently cleaving the substrate and can, in fact, in many cases improve the efficiency of the inactivation.

For example, the reduction in the length of the complementary sequence avoids problems associated with the formation of competing or unfavourable secondary structure leading to the inactivation of the ribozyme. Thus, for a given length of ribozyme molecule, more catalytic regions may be inserted targeting more cleavage sites in the substrate.

Furthermore, the use of the short arm polyribozymes means that efficent polyribozymes may be constructed even if only parts of the sequence of the substrate are known. It is sufficient to know the 10 or 15 bases surrounding an X°X°X° target site.

Another important advantage is the possibility to use, as the shortened hybridising arms, exclusively those sequences which are conserved in different viruses, for example viruses of the same type. In this way, different viruses may be inactivated by the same polyribozyme even if the sequences between the conserved regions are totally dissimilar. These viruses may be of the potyviridae family or species within the family or different isolates of the same species.

Finally, the smaller molecule allows an easier and biologically more economical synthesis. The functional nature of the 〈〈 short arm 〉〉 ribozymes of the invention proves that, in a polyribozyme, the different catalytic regions can act independently of each other.

Long-arm ribozymes, having arm lengths of at least 50 nucleotides, either contiguous or non-contiguous, are also preferred embodiments of the invention. examples are shown in Table 8 below.

Any one polyribozyme of the invention may include a mixture of 〈〈 long arm 〉〉 and 〈〈 short arm 〉〉 ribozymes, and also of 〈〈 contiguous 〉〉 and 〈〈 non-contiguous 〉〉 ribozymes. Alternatively, it may include only long arm ribozymes, or only short arm, or only contiguous, etc... The structure of the ribozyme can therefore be optimised by testing usinq the in vitro - in vivo and transient assay described below.

According to a further variant of the invention, the polyribozymes may include, between two consecutive 〈〈 R 〉〉 units, a non-complementary sequence (A) which has a length of at least 1 base, and which is non-complementary to the (+) or (-)sense potyviral RNA.

The non-complementary sequence of the polyribozyme may have a precise function, for example, it may be constituted by a coding sequence which can be used to select transformants or also a sequence containing a ribozyme which acts on a substrate other than the primary target of the DNA virus or which is cis acting on a part of the polyribozyme It typically contains a polylinker for cloning, or an adaptor to facilitate constructions of chimeric polyribozyme. The non-complementary sequence 〈〈 A 〉〉 usually has a length comprised between about 2 and 500 bases, for example 20 to 100 based. When there is a plurality of non-complementary sequences, they may together constitute as much as about 90% of the length of the polyribozyme, more preferably 50%.

Preferred ribozymes of the present invention have multiple catalytic domains (〈〈 polyribozyme 〉〉) targeting different regions or different RNA strands of the potyvirus. According to their construction (long arm or short arm, contiguous or not contiguous), the polyribozyme may act as a series of individual monoribozyme or may act as an unimolecule simultaneously agains each of its target sites.

In particular, according to a preferred variant, in a polyribozyme, two ribozyme units R1 and R2 target either, different cistrons (or 〈〈 genes 〉〉) on the same RNA strand, or they target the same or different cistrons on different RNA strands. Specifically, such a ribozyme comprises at least two of said R units, 〈〈 R₁ 〉〉 and 〈〈 R₂ 〉〉, wherein the complementary sequences (X)ₙ and (X')_{n'} of R₁ are complementary to sections of a first target region, %₁, of the potyvirus genomic (+) RNA or of the replicative (-) strand RNA, region Z₁ being within or comprising a first cistron or untranslated region (UTR), and X and X' of R₂ are complementary to sections of a second target region, Z₂, of the potyvirus genomic RNA or (-) strand replicative intermediate, region Z₂ being within or comprising a second cistron or untranslated region, Z₁ and Z₂ being either different cistrons or untranslated regions, on the same RNA strand, or being the same or different cistrons or untranslated regions on different strands, of the potyvirus RNA.

Preferred examples of this type of polyribozyme are those whose targets are all on the (+) strand, but within different cistrons, or alternatively, all on the (-) strand, but within regions corresponding to different cistrons or untranslated regions. Another example of this type of ribozyme is a polyribozyme which targets regions on different RNA strands of the potyvirus, the regions corresponding to (+) and (-) forms of the same cistron.

A further preferred polyribozyme of the invention is one comprising at least two R units, 〈〈 R₁ 〉〉 and 〈〈 R₂ 〉〉, wherein the complementary sequences (X)ₙ and (X')_{n'} of R₁ are complementary to sections of a target region Z₁ within a given gene or untranslated region of the potyvirus genomic (+) strand RNA or of the corresponding region of the replicative (-) strand RNA, and the hybridising sequences (X) and (X') of R₂ are complementary to a target region Z₂ within the same gene or untranslated region as that of Z₁, on the same RNA strand, provided that if the gene is the capsid protein gene, the hybridising sequences X and X' of both R₁ and R₂ are complementary to the replicative (-) strand RNA.

Preferred target regions for the polyribozymes of the invention are regions within or comprising the cistron giving rise to the capsid protein, the NIa cistron, the NIb cistron, the 3' untranslated region of a potyvirus, or the corresponding sequences in the replicative (-) strand RNA.

Specific examples of ribozymes of the invention are the following :
- ribozymes comprising at least two R units, R₁ and R₂, wherein the complementary sequences (X)ₙ and (X')_{n'} of R₁ and of R₂ are respectively complementary to regions within the CP cistron (either (+) or (-) strand and the 3' non-translated region of the genomic (+) strand RNA ;
- ribozymes comprising at least two R units, R₁ and R₂, wherein the complementary sequences (X)ₙ and (X')_{n'} of R₁ and R₂ are respectively complementary to regions within the region corresponding to the CP cistron and the NIb cistron, or to the NIb cistron and the Nia cistron, of the replicative (-) strand RNA ;
- ribozymes comprising at least two R units, R₁ and R₂, wherein the complementary sequences (X)ₙ and (X')_{n'} of R₁ are complementary to the CP cistron of the genomic (+) strand RNA, and the hybridising regions of R₂ are complementary to all or a portion of the region corresponding to the CP cistron on the replicative (-) strand RNA ;
- ribozymes comprising at least three R units, R₁, R₂ and R₃, wherein the complementary sequences (X)ₙ and (X')_{n'} of R₁ are complementary to regions within the CP cistron or the 3'NT region of the genomic (+) strand RNA, the complementary sequences (X)ₙ and (X')_{n'} of R₂ are complementary to regions within the region corresponding to the NIb cistron on the replicative (-) strand RNA and the complementary sequences (X)ₙ and (X')_{n'} of R₃ are complementary to regions within the region corresponding to the NIa or CP cistrons on the replicative (-) strand RNA ;
- ribozymes wherein the complementary sequences (X)ₙ and (X')_{n'} of R₁ and R₂ are complementary to regions within the CP gene on the replicative (-) strand RNA.

Further examples of preferred ribozymes are indicated in table 8 below.

In addition to the choice of target site(s), target region(s) and catalytic region(s), a further variant can be obtained by incuding in the ribozyme construction, all or part of a 3' non-coding region of a potyvirus, preferably WMV2, to control intracellular localisation of the ribozyme gene. This may help to direct the ribozyme RNA to the cellular compartment where the potyviral RNA might be. The enhanced functioning of ribozymes carrying the 3' non-coding region is suspected by analogy to the observations in animal cells (Sullenger and Cooh 1993, Science vol. 262 p 1566-1569) that a ribozyme against a retrovirus was much more efficient when it was co-localised with the target, using a retroviral vector to express it.

The 3' non-translated sequence usually contains all the untranslated region of the virus and may also contain the last 10 to 20 codons of the protein and the first 10 to 15 residues of the viral poly A tail.

The ribozymes of the present invention can be tested for their capacity to cleave a substrate RNA, and / or to confer resistance to potyvirus infection, by in vitro, in vivo or transient assays as described below in examples 8, 9 and 10 respectively.

The in vitro test involves incubating a suitable RNA substrate, uniformly labeled with a radioactive label, with the ribozyme under test, prepared by in vitro transcription. Incubation is carried out at varying temeratures as described in example 8. The capacity of the ribozyme to cleave the substrate at the expected target sites is shown by carrying out an autoradiogram on a polyacrylamide gel.

The ribozymes and genetic constructs of the present invention can be conveniently tested in vivo using virus-sensitive plants. The analysis may be qualitative such as by screening for severity of symptoms, or quantitative where samples are analysed, for example, by measuring the level of virus replicating in infected plants using procedures such as ELISA, or alternatively, the level of ribozyme RNA, or the level of target RNA. A combination of qualitative or quantitative analyses may also be employed.

The genetic nature of the virus-resistant transgenic plants can be determined by carrying out a segregation analysis. The goal of this test is to determine genetic linkage of the ribozyme gene and virus resistance. Self-fertilisation, or a cross with a non transformed genotype can be carried out on a primary descendant to obtain T₁. Subsequently, T₁ plants are inoculated with the potyvirus by mechanical inoculaion or natural inoculation or by using aphids fed on infected plants. Plants are scored for resistance to infection. A segregation ratio of 3:1 (resistant : susceptible) indicates a single locus acting as a heterozygous trait (〈〈 dominant 〉〉) ; a segregation ratio of 1:3 indicates a single locus acting as a heterozygous trait (〈〈 recessive 〉〉), and a segregation ratio of >3:1 indicates a plurality of loci, acting independently of each other, and conferring resistance as a single copy as a heterozygous trait.

The ribozymes of the invention can also be tested using a transient transformation assay as described below in Example 10. The target is linked to a reporter gene such as LUC. DNA encoding the substrate-reporet gene hybrid is co-introduced into a plant cell along with a plasmid encoding the ribozyme under test. The level of expression of the reporter gene is measured. Expression is decreased when the ribozyme interacts with the target.

The present invention also extends to genetic constructs comprising the ribozymes herein described. These genetic constructs generally comprise a vector sequence as a vehicle for transferring the ribozyme to a host cell and a promoter sequence to direct transcription of the ribozyme and a terminator to signal termination of transcription. A preferred promoter includes but is not limited to a 35S Cauliflower Mosaic Virus (CaMV) or to a double 35S CaMV promoter. The CaMV promoter is described by Odell et al, Nature 313 : 810-812, 1985. The ribozymes are generally in the complementary form such that upon transcription, the ribozyme is produced. The genetic constructs of the present invention are used to generate transgenic plants capable of constitutively, developmentally or inducibly (e.g. in response to certain stimuli) expressing the ribozymes herein described. Most preferred transgenic cells and plants are rendered substantially resistant to potyvirus infection.

The invention also extends to a method for producing transgenic plants which exhibit at least some resistance to infection by potyviruses, and to the plants which can be obtained thereby. According to this aspect of the present invention, there is provided a method of generating trangenic plants exhibiting reduced symptoms following exposure to potyviruses compared to non-transgenic plants, said method comprising transforming plant cells with a genetic construct encoding a ribozyme as hereinbefore described capable of cleaving potyvirus RNA, regenerating plants from said transformed cells, self-fertilising said regenerated plants and obtaining seeds there from and then growing transgenic plants from said seeds.

All of the known means for introducing foreign DNA into plants may be used for example Agrobacterium, electroporation, protoplast fusion, bombardment with a particle gun, or penetration of DNA into cells such as pollen, the microspore, the seed and the immature embryo, viral vectors such as the Geminiviruses or the satellite viruses. Agrobacterium tumefaciens and rhizogenes constitute the preferred means. In this case, the sequence coding for the polyribozyme is introduced into a suitable vector together with all of the regulatory sequences necessary such as promoters, terminators, etc... as well as any sequence necessary for selecting the transformants.

The invention also extends to progeny, reproductive material and seeds from the transgenic plants. The ribozymes may be expressed constitutively, developmentally or inducibly in the whole plant, or in specific tissues of the plant.

As examples of plants which can be rendered resistant to potyvirus infection by the present invention, mention can be made of plants belonging to the families : Cucurbitaceae (e.g. courgette melon), Chenopodiaceae (e.g. beet), Cruciferaceae, Graminaceae (e.g. oat, wheat, sugar cane, barley, ryegrass), Leguminoseae (e.g. bean, pea, soya, peanut), Ombelliferaceae (e.g. carrot, celery, parsnip), Rosaceae (e.g. plum), Solanaccae (e.g. potato, tomato, tobacco, pepper).

The present invention also extends to the use of the claimed ribozymes in plant cells or plants, in association with other virus resistance genes or systems, either to potyviruses or to other viruses, for example, anti-CMV ribozymes (CMV signifies 〈〈 cucumber mosaic virus), or coat-protein mediated systems for virus resistance such as coat protein antisense or sense (translated, or translation deficient) RNA. According to this variant of the invention, the plant may comprise cells which are transformed both by the ribozyme sequences of the invention and by the sequences providing associated protection. Expression of the different sequences providing protection may be simultaneous or otherwise.

According to a further embodiment of the invention, there is provided a nucleic acid molecule which consists of a fragment of the WMV2. viral replicative (-)RNA, which has been shown to be particularly amenable to cleavage or inactivation by ribozymes, and can thus be used us an artificial target molecule. Indeed, the unexpected discovery that the sequence from the negative strand of the virus around site 1747 is specially susceptible to gene inactivation opens useful possibilities to use this sequence as a ribozyme or antisense target not only for protecting plants against viruses but for other uses as well.

This molecule, and its derivatives will be referred to hereinafter as the 〈〈 artificial target 〉〉 molecule.

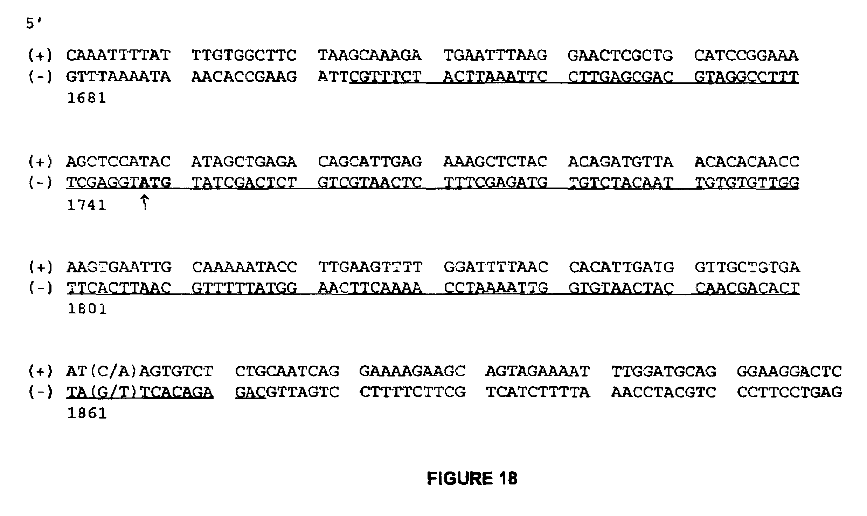

The artificial target molecules, of the invention consist of either nucleotides 1704 to 1873 of the (-) strand of the sequence illustrated in figure 18, or of at least 20, for example between 40 and 170, consecutive nucleotides of the 1704-1873 fragment, including the ATG shown at Position 1748-1750 in Figure 18. The artificial target molecules may also consist of a sequence having at least 75% identity and preferably at least 85% or even 95% identity with the underlined sequence of Figure 18, or a fragment thereof hving at least 20 nucleotides.

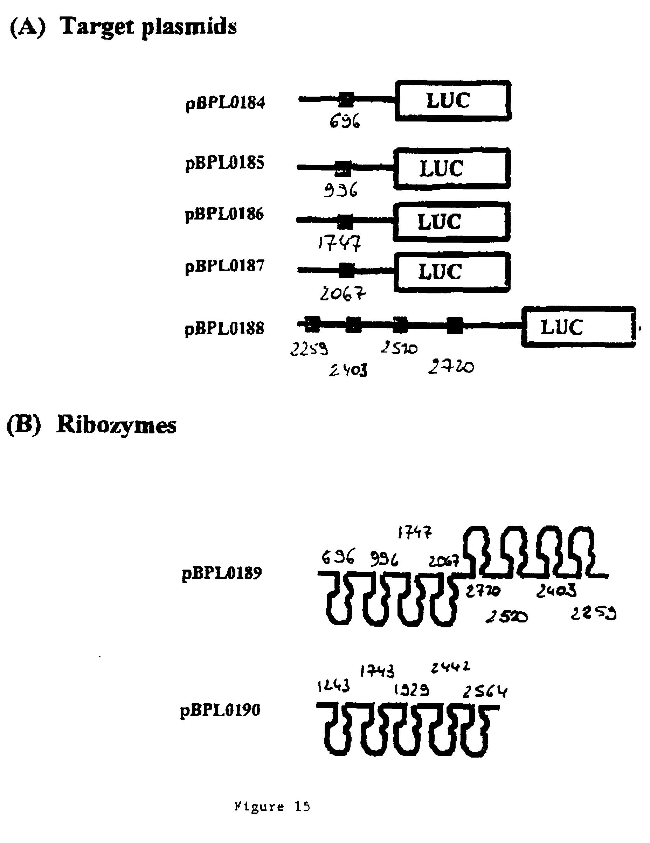

The artififcial target sequence present on pBPL 0186 as described in figure 15, is made of approximately 180 bases of viral sequence from the minus strand of the viral genome, in the region coding on the plus strand for the replicase NIb gene. The sequence is made of 125 bases in 5' of the A of the GUA ribozyme cleavage site arid 44 bases in 3' of the A. The A is the complementary base to the T shown as base n° 1748 on figure 18. Part of this sequence is present on many RNA viruses. For example around this cleavage site in WMV2 :
Potato Virus Y has 20 out of 22 bases identical to WMV2 (90.9%)
Potato Virus A has 22 out of 23 bases identical to WMV2 (95.6%)
Pea Seedborne Mosaic Virus has 20 out of 23 bases identical to WMV2 (86.95%)
Human Cytomegalovirus has 17 out of 23 bases identical to WHV2 (73.9%)
Influenza Virus has 17 out of 19 bases identical to WMV2 (89.5%).

This region is therefore a good target for molecules designed against those viruses.

The invention also relates to larger nucleic acid molecules containing the artificial target sequences of the invention with the exception of known full length viral (-)RNA which naturally contains the sequence.

Preferred examples of molecules comprising the artificial target sequences are molecules in which the artificial target sequence is transcriptionally or translationally fused to a second nucleic acid molecule.

The artificial target sequence and its derivatives is thus appended to a gene of interest (hereafter called 〈〈 the useful gene 〉〉 or 〈〈 gene of interest 〉〉), in 5' or in 3' of the open reading frame or coding sequence of the useful gene, so that it does not prevent the function of the useful gene and makes this gene easily controllable by a ribozyme (such as those described earlier), or antisense, directed against the artificial target.

The term 〈〈 coding sequence 〉〉 in this context includes those sequences active as RNA like tRNA, rRNA, etc. in addition to those sequences coding for proteins. The appended sequence may also be a regulatory sequences, sequences encoding functional RNA such as rRNA, tRNA, mRNA etc.

The 5' fusion can be transcriptional, or translational. Similarly the 3' fusion can be transcriptional or translational. The precise nature of the fusion depends on the functionality of the useful gene. For example, in the case of a gene coding for a protein, a translational fusion can be used if the aminoacids added to the protein (in NH₂ or COOH terminus as the case may be) do not prevent its function.

The artificial target sequence can be smaller than the sequence described above, as demonstrated by the inhibitory activity of short arm ribozymes pairing with only a very small stretch of that sequence. For example the artificial target sequence can be reduced to 40 bases on each side of the A of the GUA, or to 10 bases in 5' and 12 bases in 3' of the A, or even to 8 bases on each side of the A, if a slightly lower efficiency of the ribozyme is acceptable.

Optimal length can be found by routine experiment.

The high activity of ribozymes against the artificial target sequence in the experiments described can probably be explained in part by its accessibility to inhibitor RNA molecules. Therefore, it is possible to use not only ribozymes but also antisense RNA against the artificial target, albeit with a lower efficiency.

The fusion between the gene of interest and the artificial target can now be used as a target for ribozymes or antisense RNA. The inactivation of the useful gene containing the artificial target can be caused by the ribozyme gene brought in through a cross, or by the tissue specific expression of the ribozyme gene if the ribozyme gene is under control of a tissue specific promoter. Alternatively, it can be caused by the inducible expression of the ribozyme gene if the ribozyme gene is under control of an inducible promoter. By inducible is meant chemically inducible (by a natural or an artificial molecule) or inducible by environmental factors such as light, drought, stress, pathogen attack and other stimuli. Inducible and tissue specific promoters are well known.

The advantage of controlling gene expression this way is that it makes use of a known ribozyme gene, already shown to function properly. Therefore the need to experiment to find the best molecule for inactivating the useful gene is reduced.

As an example of a useful gene, we can describe the case of the gene A9-PR-Glucanase (Worrall et al, The Plant Cell, 1992, vol. 4, p 759-771). This gene is used to cause artificial male sterility (AMS) in transgenic plants. AMS plants are used as the female parent in the cross to produce hybrid seeds. When the hybrid plant obtained is grown for its fruits, it is often desirable to restore its fertility to guarantee the optimal fruit set. To facilitate the restoration of the AMS caused by the A9-PR-Glucanase gene, the gene can be engineered by fusing to it the artificial target sequence described above so that the gene function is not affected. It is then possible to use a ribozyme against the artificial target as a restorer of the male sterility. The ribozyme gene is introduced in the male parent where it has no effect (since there is no target sequence in that plant). The hybrid plant resulting from the cross between the AMS female plant and the male restorer plant will be fully fertile since the ribozyme brought in by the male parent will inactivate the AMS gene brought in by the female parent. Other AMS gene can be used similarly. The use of the artificial target is not limited to the area of plant improvement. The artificial target could be used with any artificial gene when an efficient inhibition of the gene is necessary in some circumstances. For example, the artificial target could be appended to a therapeutic gene used in a gene therapy treatment in humans or animals. A tissue specific ribozyme gene against the artificial target could then be used to protect some specific tissue against the activity of the therapeutic gene if such activity was deemed undesirable in laid tissue. An inducible ribozyme gene against the artificial target could be used to give the clinician the ability to stop the effect of the therapeutic gene if it was necessary, just by inducing the ribozyme gene. Indeed such systems might be absolutely required for certain gene therapy procedures, perhaps wwhere the expression of toxin genes is involved. A synthetic ribozyme against the artificial target could also be used through extraneaous delivery to control activity of the therapeutic gene in the appropriate tissue or at the appropriate time.

Similarly the artificial target could be used in association with genes used in animal improvement. It could also be used in association with genes used in the food areas for example to improve microorganisms used in food production.
Different aspect, of the invention arc illustrated in the figures :
Figure 1: Location of the primers used to amplify the viral sequences. The amino acid sequence used to design the primers is indicated (sec figure2, 3, and 4 below).
Figure 2: Conserved Sequence in Nib coding region, used to design primer 2. Viruses are mentioned with the reference of the publication for their partial (WMV2 and ZYMV) or complete (all others) genomic sequence. Position on the published sequence is indicated. The standard one letter amino-acid code is used.
Figure 3: Conserved Sequence in NIb coding region, used to design primer 3. Notations are as in Figure 2. CP is Coat Protein.
Figure 4: Conserved Sequence at end of NIa coding region, used to design Primer 4. Notations are as in Figure 2.
Figure 5: Sequence of the cDNA of the 3' region of WMV2 strain Mar6. Bases 1-10 were added during cloning. Viral sequence starts at base 11 and ends at base 2971 where the polyA tail starts.

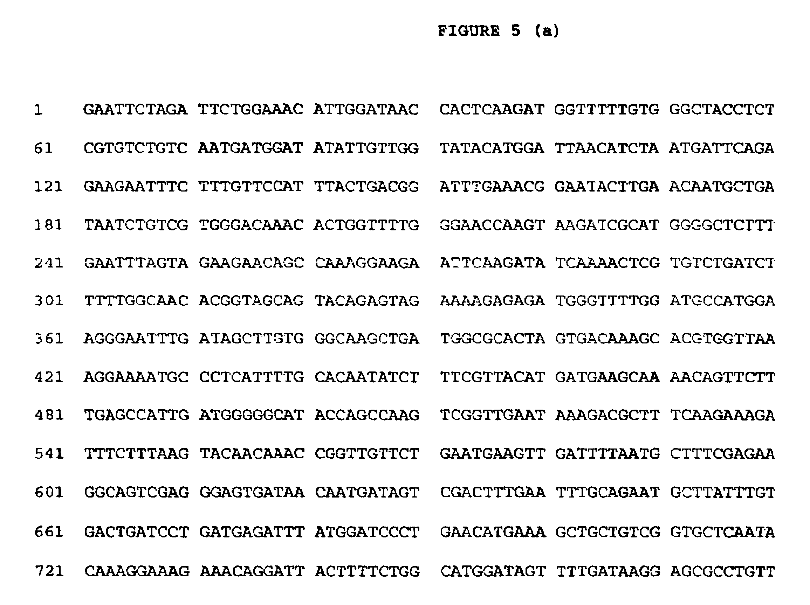

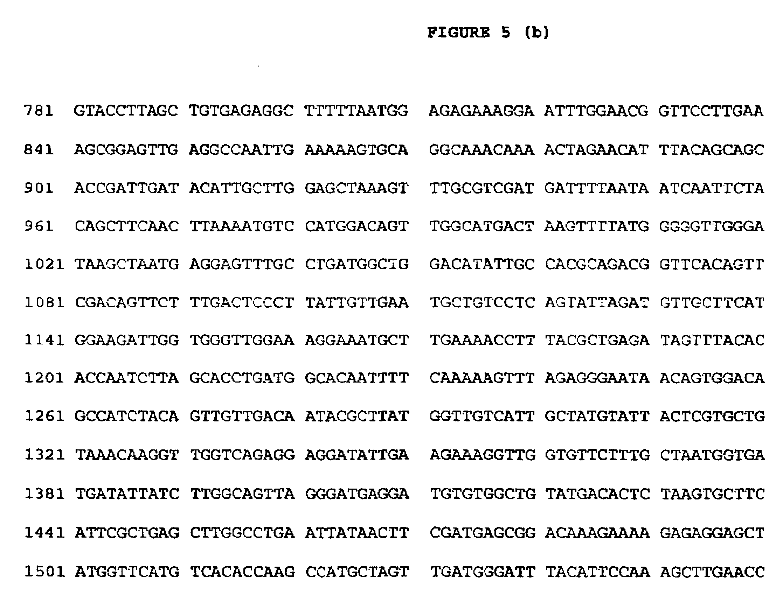

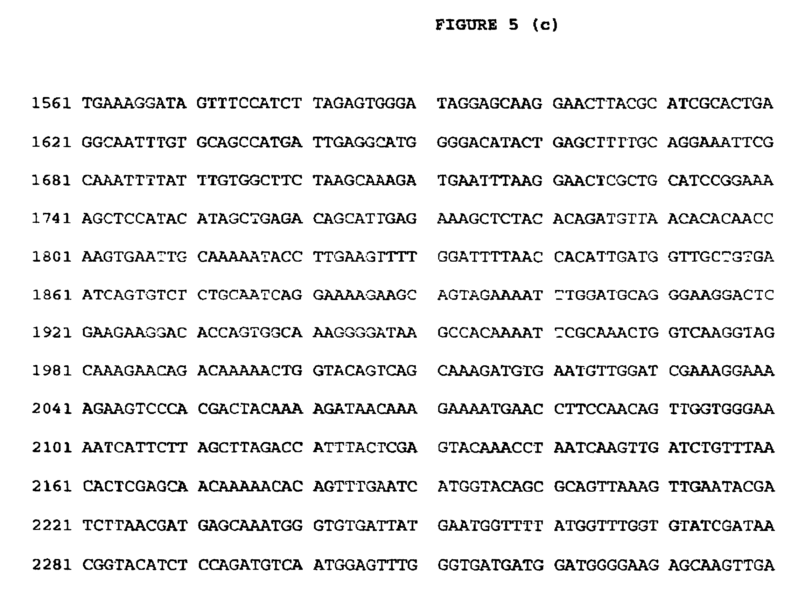

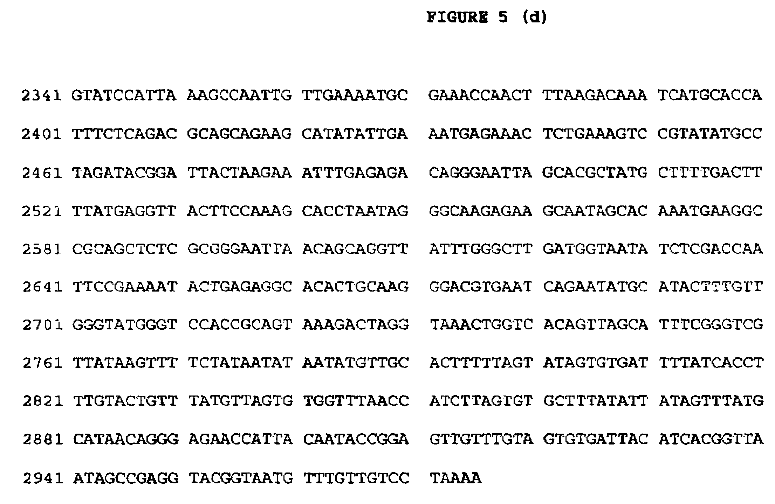

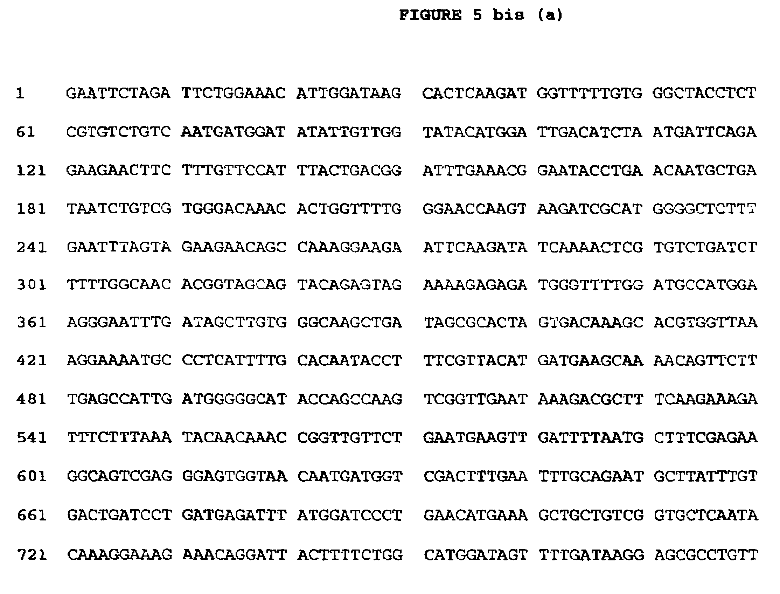

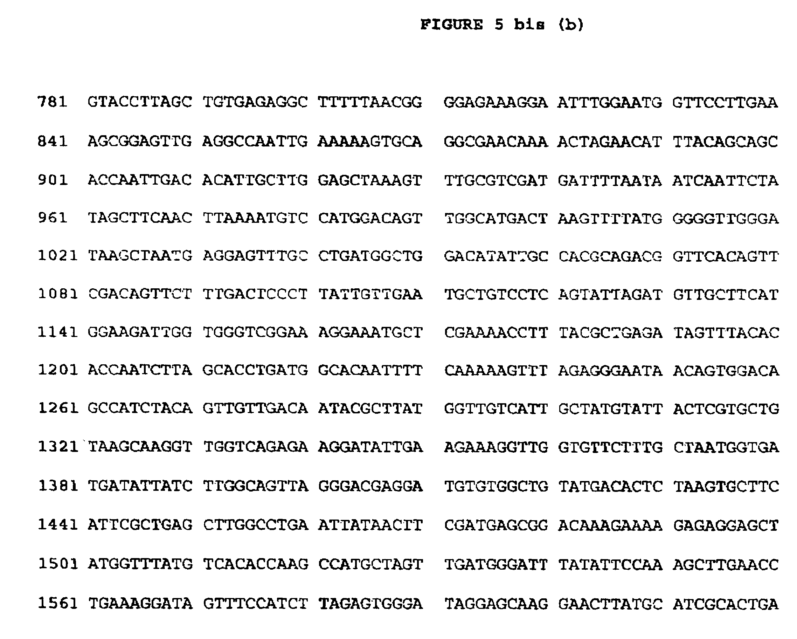

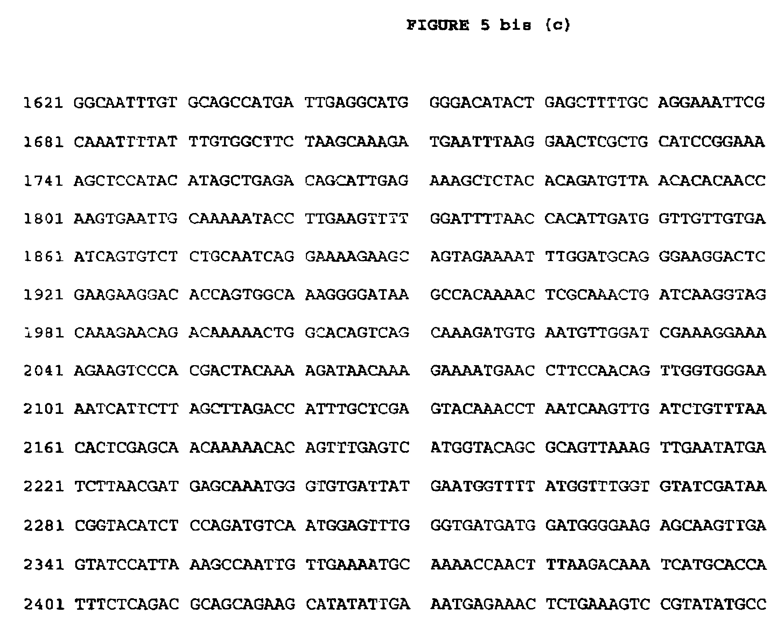

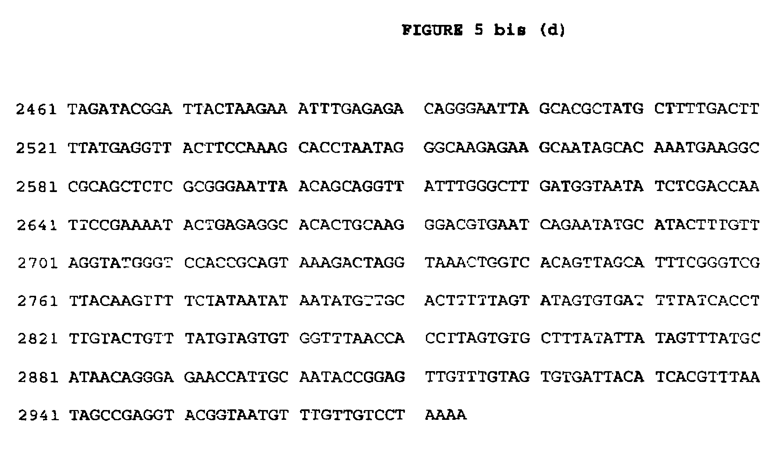

Figure 5 bis: Sequence of the cDNA of the 3' region of WMV2 strain val3. Bases 1-10 were added during cloning. Viral sequence starts at base 11 and ends at base 2970 where the polyA tail starts.

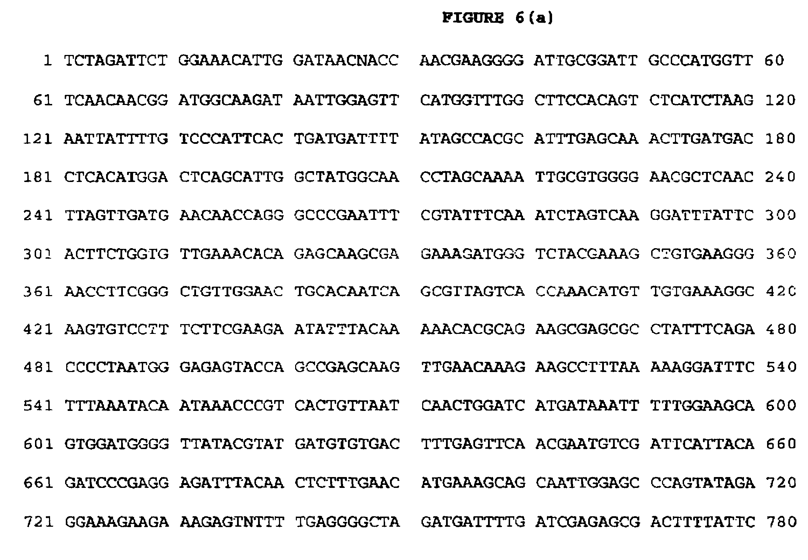

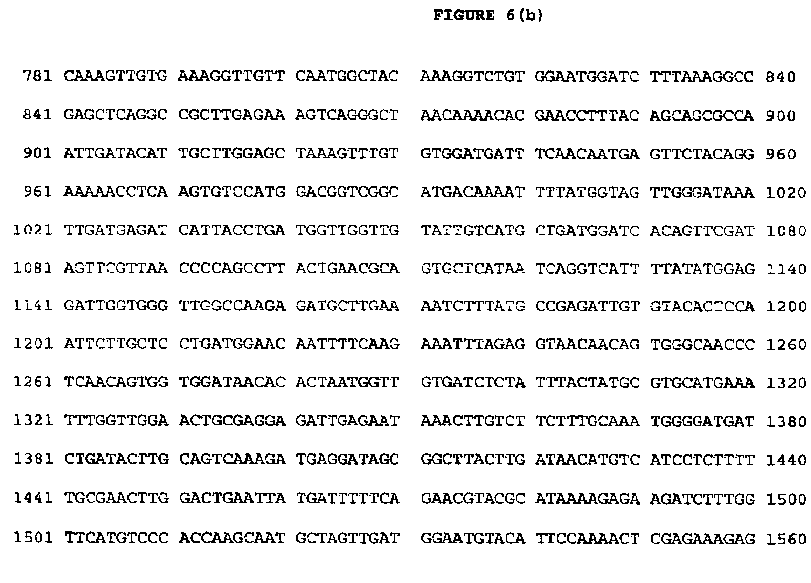

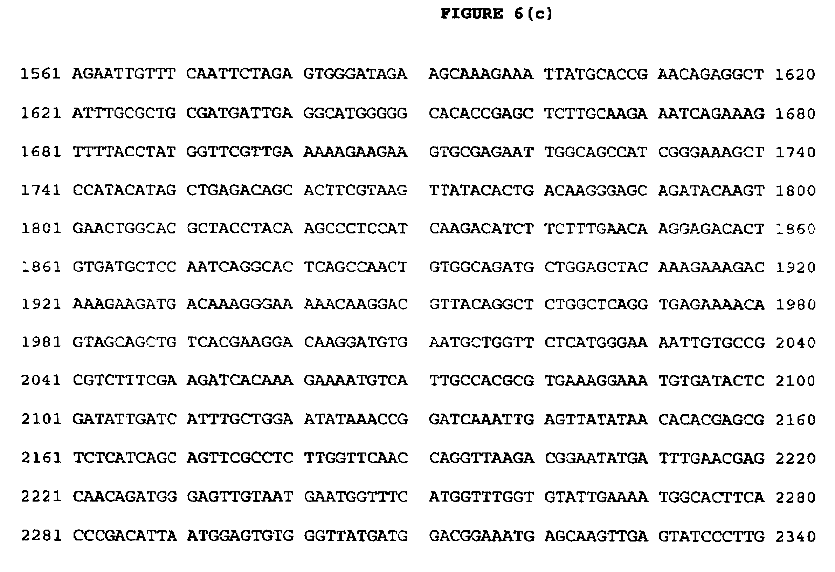

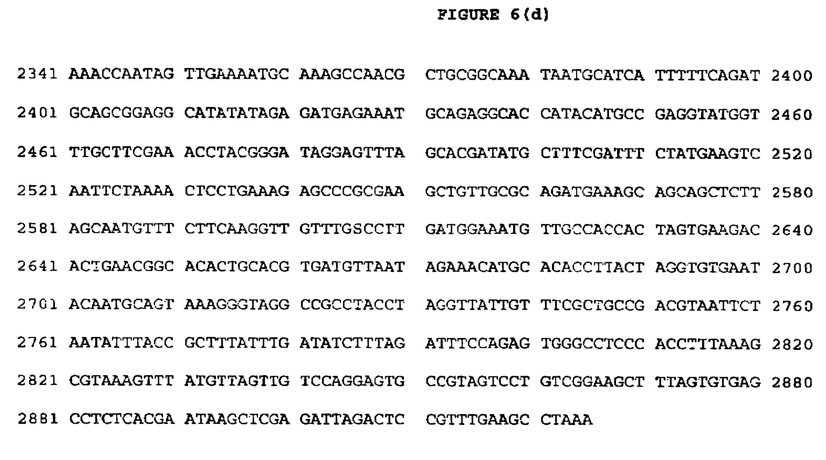

Figure 6: Sequence of the cDNA of the 3' region of ZYMV strain E15. Bases 1-6 were added during cloning. Viral sequence starts at base 7 and ends at base 2922 where the polyA tail starts.

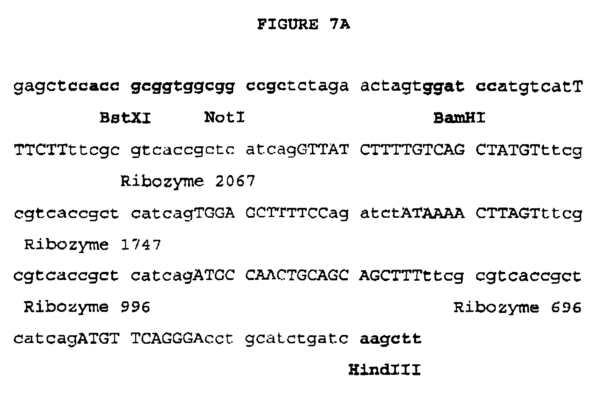

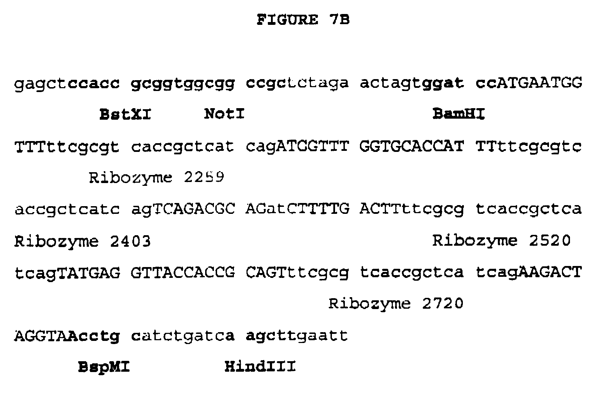

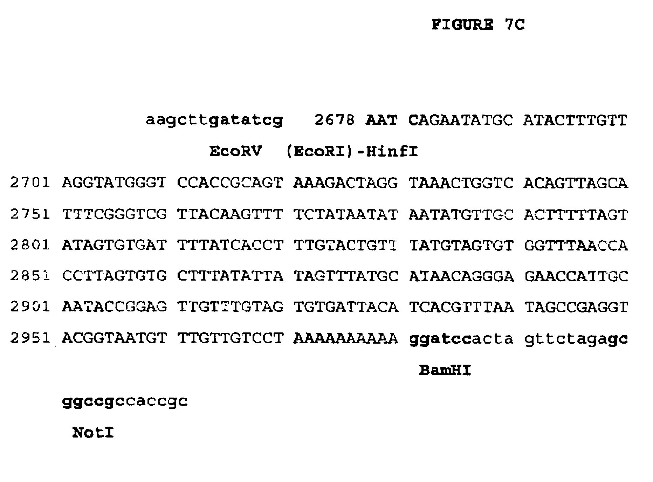

Figure 7 Sequences used to build the short arm ribozyme gene against WMV2.
   A :Short arm ribozyme against the minus strand, cloned into BlueScript SK+ (pBPL4741). Lower case letters are used for the ribozyme catalytic sequence and the bases from polylinkers. Relevant restriction sites are in **bold letters**. The sequence complementary to the ribozyme RNA is shown.
   B :Short arm ribozyme against the plus strand, cloned into BlueScript SK+ (pBPL4742). Notations as above. The sequence complementary to the ribozyme RNA is shown.
   C : Sequence of the cloned 3' UTR of WMV2 Val3 used in the short arm ribozyme gene. Lower case letter show polylinker regions from BlueScript or the BamHI cloning site added by PCR. Cloning sites destroyed during cloning are shown between brackets. Numbering refers to the original sequence determined.

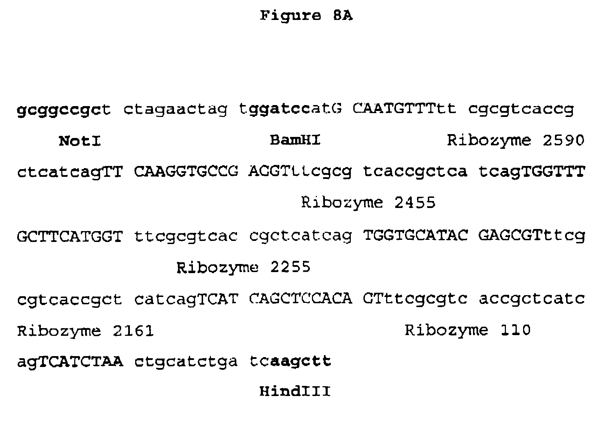

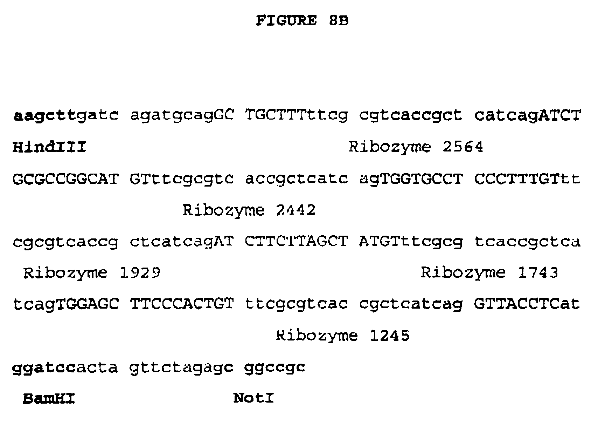

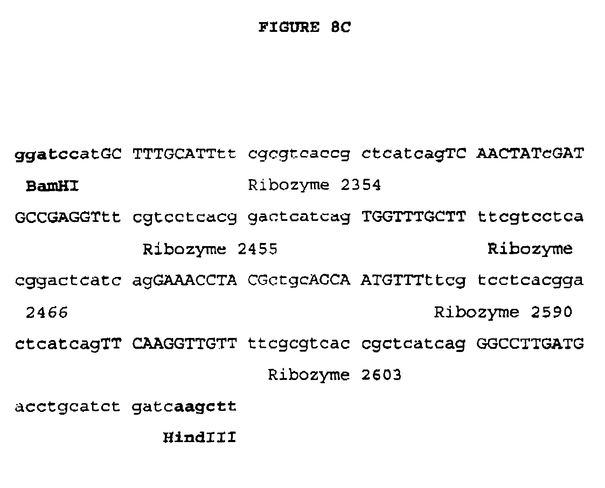

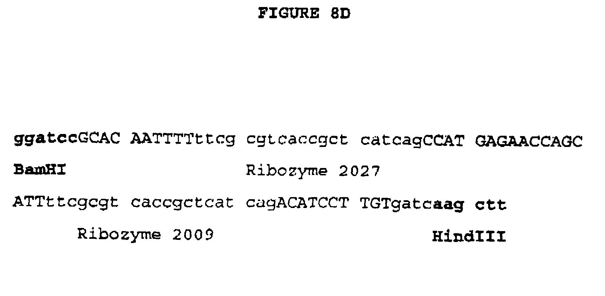

Figure 8: Sequences used to build short arm ribozymes against ZYMV.
   A : First combination against the plus strand, cloned in BlueScript SK+: pBPL4746. Notations as in figure 7. The sequence complementary to the ribozyme RNA is shown.
   B : First combination against the minus strand, cloned in BlueScript SK+: pBPL4747. Notations as above. The sequence complementary to the ribozyme RNA is shown.
   C : Second combination, against both strands, cloned in BlueScriptSK+: pBPL4750. Notations as above. The sequence complementary to the ribozyme RNA is shown.
   D : Third combination, against minus strand, cloned in BlueScriptSK+: pBPL4751. Notations as above. The sequence complementary to the ribozyme RNA is shown.

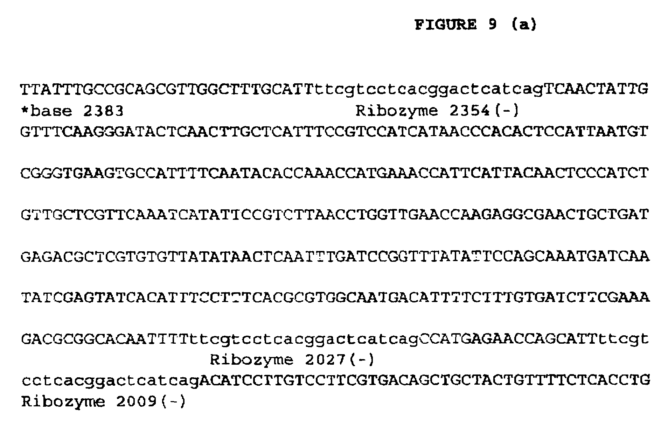

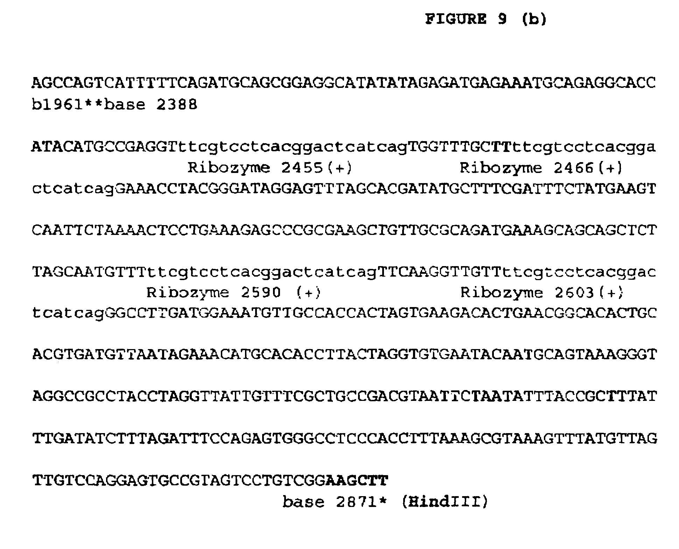

Figure 9: Sequence of the long arm ribozyme gene against ZYMV: pBPL4760.
   Base numbers corresponding to the sequence of the + strand shown in figure 6 are indicated with a * below the base.
   The first 486 bases are targeting the minus strand (between base 2383 and base 1961), the junction is shown by ** under the bases, and the last 568 bases are targeting the + strand (between base 2388 and base 2871).
   Notations as above. The sequence complementary to the ribozyme RNA is shown.

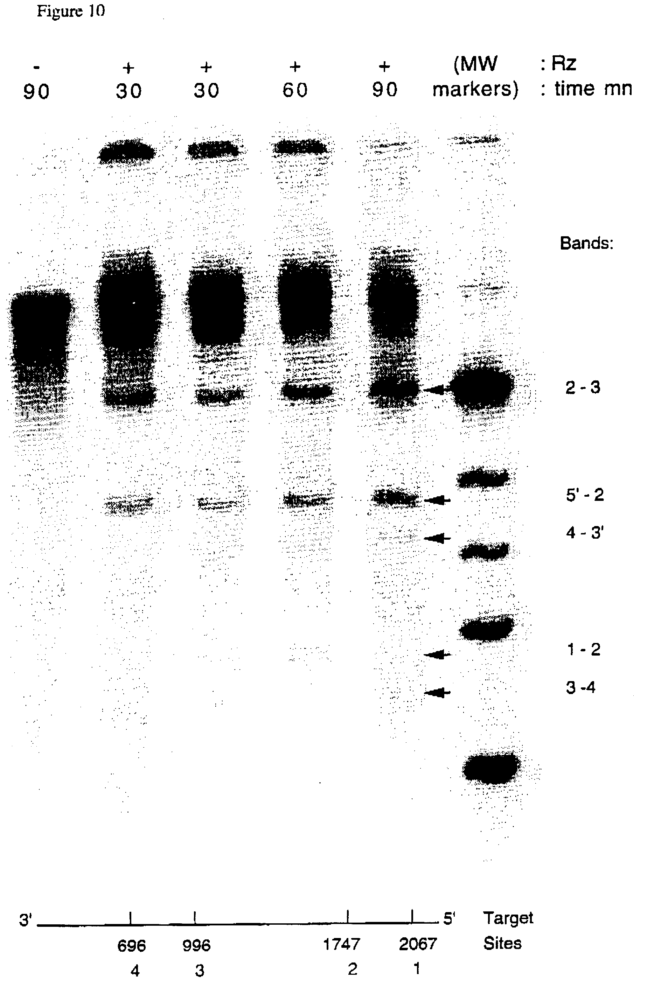

Figure 10 : In vitro assay of ribozyme from pBPL4734.

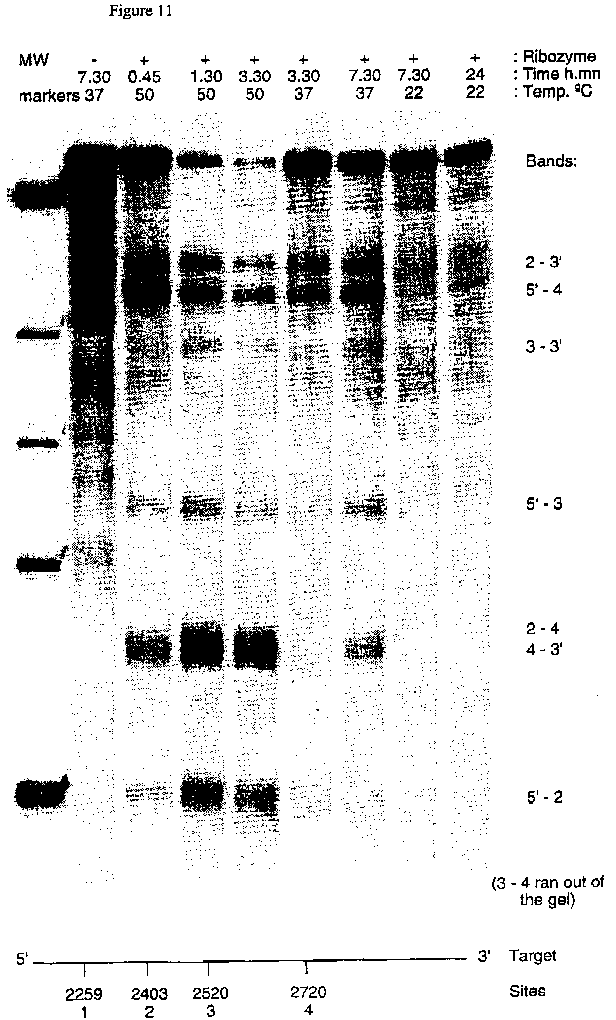

Figure 11 : In vitro assay of ribozyme from pBPL4736.

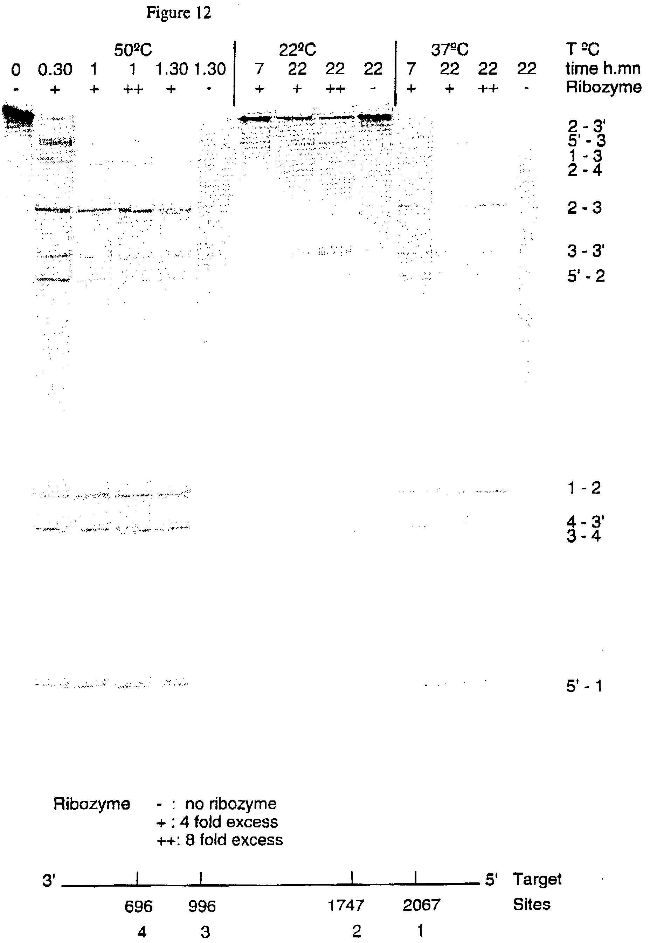

Figure 12 : In vitro assay of ribozyme from pBPL4741.

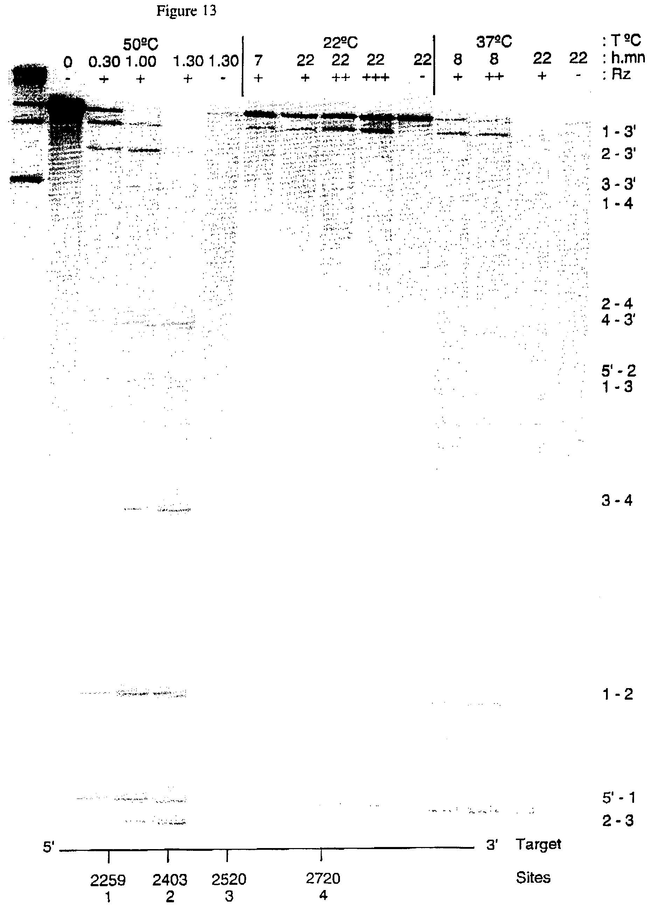

Figure 13 : In vitro assay of ribozyme from pBPL4742.

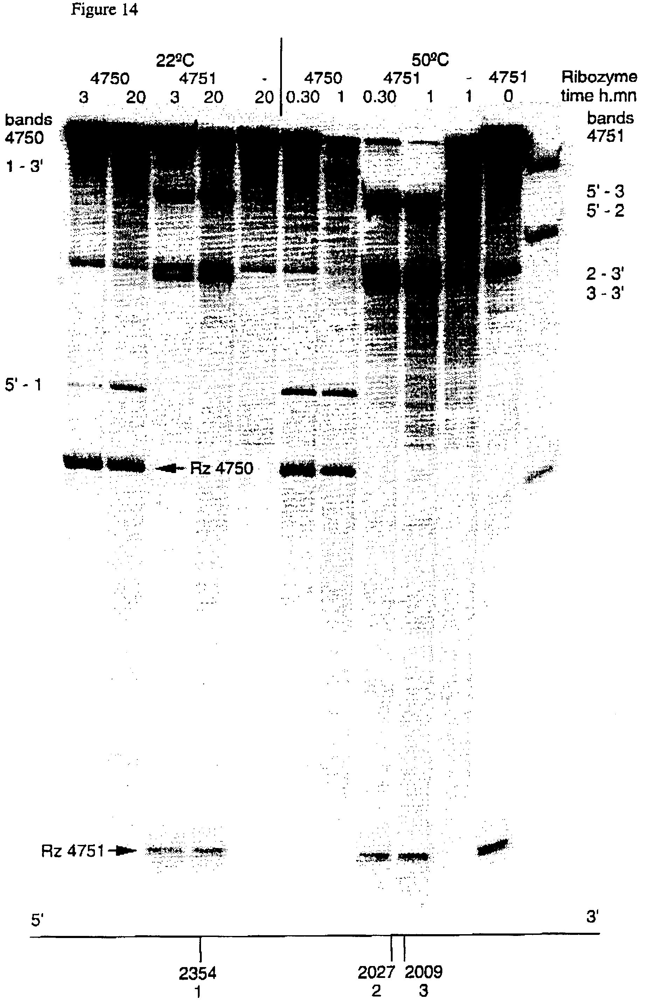

Figure 14 : In vitro assay of ribozyme from pBPL4750 and pBPL4751.
Figure 15 : Plasmids used in the transient assay.
   A : Target plasmids pBPL0184, 0185, 0186 and 0187 contain 190 bp, 210 bp, 170 bp and 180 bp respectively of minus strand sequence of WMV2 cloned in sites NsiI - NcoI of pGLFuz (Sawyer and Birch 1996). Target plasmid pBPL0188 contain 540 bp of the plus strand of WMV2 cloned into sites NsiI - NcoI of pGLFuz.
   B : Ribozyme plasmid pBPL0189 contains a 400 bp insert cloned into the BglII - XhoI sites of a derivative of pGLFuz, encoding an octameric ribozyme against WMV2, targeting sites 696(-), 996(-), 1747(-), 2067(-), 2259(+), 2403 (+), 2520(+) and 2720(+) Ribozyme plasmid pBPL0190 contains a 220 bp insert cloned into the BglII - ShoI sites of a derivative of pGLFuz, encoding a pentameric ribozyme against the minus strand of ZYMV, targeting sites 1243, 1743 (equivalent to 1747 in WMV2), 1929, 2442 and 2564.

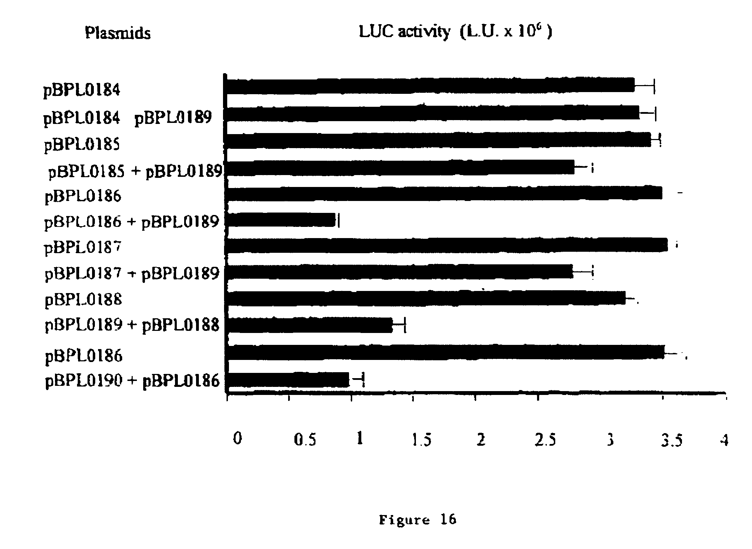

Figure 16 : Transient LUC activity after co-bonbardment of either an octanmeric ribozyme (pBPL0189) or a pentameric ribozyme (pBPL0190) with various plasmids containing different target regions fused 5' to the luciferase gene. Each co-bombardment consisted of 2 µg of ribozyme plasmid (pBPL0189 or pBPL0190) and 2 µg of the target plasmid as indicated. Bars represent standard errors of the mean of three independent experiments.

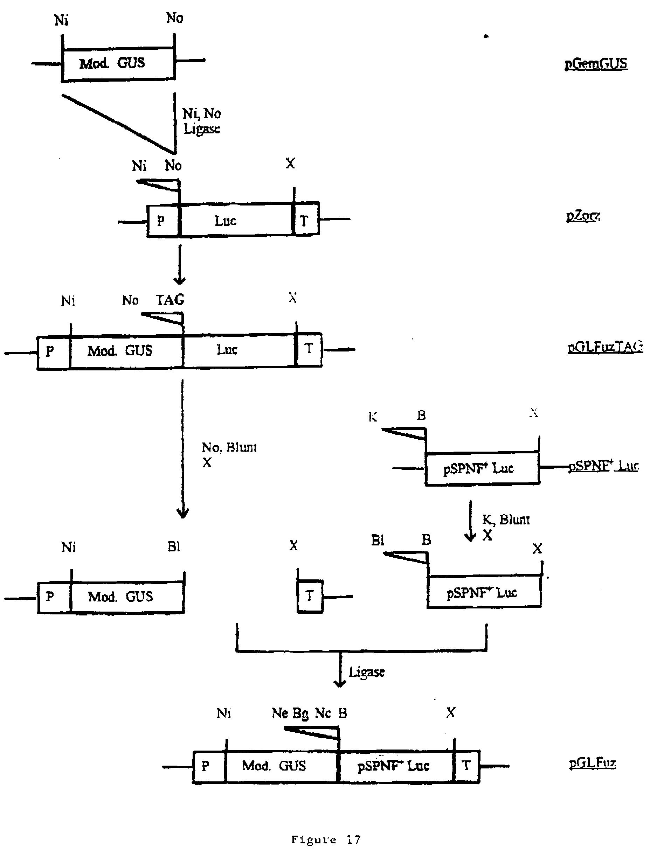

Figure 17 : Construction of pGLFuz vector (Sawyer and Birch 1996). The 1.8 Kb PCR modified uidA gene was inserted as a NsiI - NcoI DNA fragment upstream of the luc gene of pZorz to yield pGLfuzTAG. The luc gene of pGLfuzTAG was removed (by digestion with NotI, treatment with T4 DNA polymerase, then digestion with XhoI) and replaced by the 1.9 Kb luc gene from pSPNF+luc (prepared by digestion with KpnI, treatment with T4 DNA polymerase and digestion with XhoI), yielding the uidA :luc fusion construct pGLFuz. B, GglII; Bl, blund end; K, KpnI; Luc, luc gene from pZorz; Mod. GUS, PCR modified uidA gene; Nc, NcoI; Ne, NheI; Ni, NsiI; No, NotI; p, osa artificial promoter region containing 4ocs Δ35S-Ac leader regions; pSPNF⁺Luc, luc gene from pSPNF'luc; T, ocs 3' polyadenylation region; TAG, stop codon; X, ShoI.
Figure 18 : Sequence of the region of WMV2 strain Mar6 (top line = (+) strand, bottom line = (-) strand) around ribozyme cleavage site 1747 in the (-) strand (indicated in bold type). Underlined sequence (positions 1704 → 1873 in (-) **strand**) represents the full length artificial target sequence which can be linked to a reporter gene in transient ribozyme assays, or to a gene of interest as a ribozyme - regulatable element. Arrow (↑) indicates nucleotide in (-) strand which is immediately 5' of the ribozyme cleavage site. (G/T) at position 1863 in the (-) strand indicates that the naturally occurring G can be replaced with a T to mutate the stop codon at 1864-1862 (TGA). This facilitates in-frame fusion, and can be introduced using appropriate PCR primers.

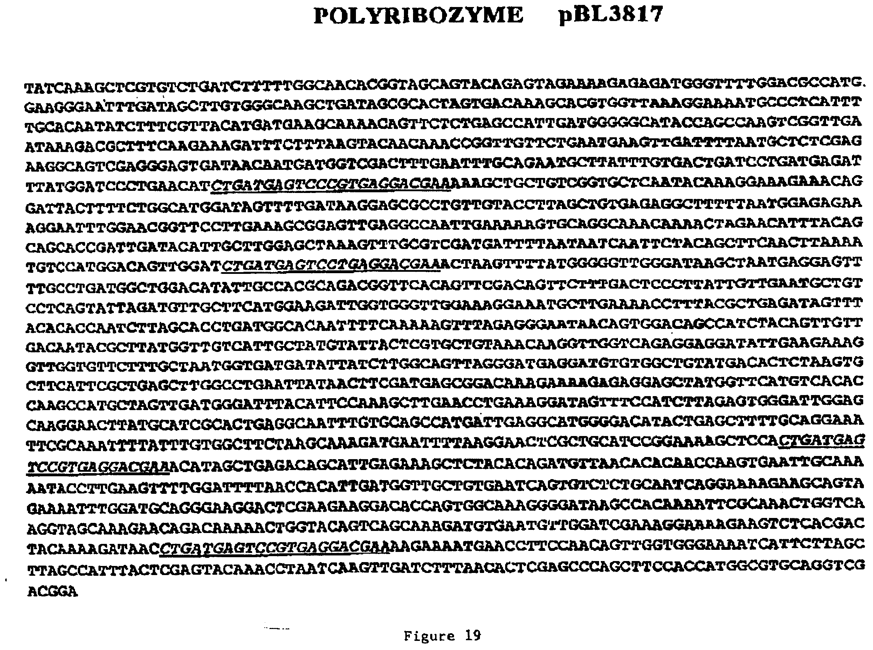

Figure 19 : shows the DNA sequence of pBPL3817 (long arm ribozyme against WMV2 NIb and CP cistrons). The sequence is written from 5' to 3'. The first base corresponds to base number 279 in figure 5. the last viral base is G, 34 bases before the end of the sequence. The ribozyme catalytic regions are underlined, in italics
Figure 20 : shows a the genome organisation of the type member of the potyvirus family, PVY (from Chapter 4, 〈〈 The Potyviridae 〉〉, Ed. Shukla, D.D., Ward, C.W., Brunt, A.A, 1994 CAB International). P1 : First Protein ; HC-Pro :Helper component ; P3 : Third Protein ; 6K1, 6K2 : additional 6kb proteins ; CI : Cylindrical Inclusion Protein ; NIa : Small Nuclear Inclusion protein ; NIb : large Nuclear Inclusion Protein ; CP : Coat Protein

### EXAMPLES

Molecular Biology techniques used are essentially those described in Current Protocols in Molecular Biology (Ausubel F.M. et al. Eds, 1994-1997, John Wiley & Sons, Inc., New York.) except where otherwise. mentioned.

### 1. Cloning and Sequencing the 3' region of genomic RNA of target viruses.

Target viruses for this work are Watermelon Mosaic Virus 2 (WMV2) and Zucchini Yellow Mosaic Virus (ZYMV).

To design ribozymes against these viruses, a partial sequence of the genome of field isolates of these viruses from the Mediterranean region was determined as follows:
a - Infected plant material was harvested, and total RNA extracted.
b - The RNA was used as the template for a Reverse Transcription - Polymerase Chain Reaction (RT-PCR) using primers specific for the 3' terminal region of the potyviral genome.
c - The PCR products were cloned and sequenced.
These three steps are detailed below:
a - Zucchini plants were mechanically inoculated with strains Mar6 or Val3 of WMV2 or strains E9 or E15 of ZYMV. These strains are field isolates from the Mediterranean region. Leaves were harvested and total RNA was extracted.
b - A sequence comparison of all published potyvirus sequences allowed the identification of regions of sequence conservation among potyviruses.

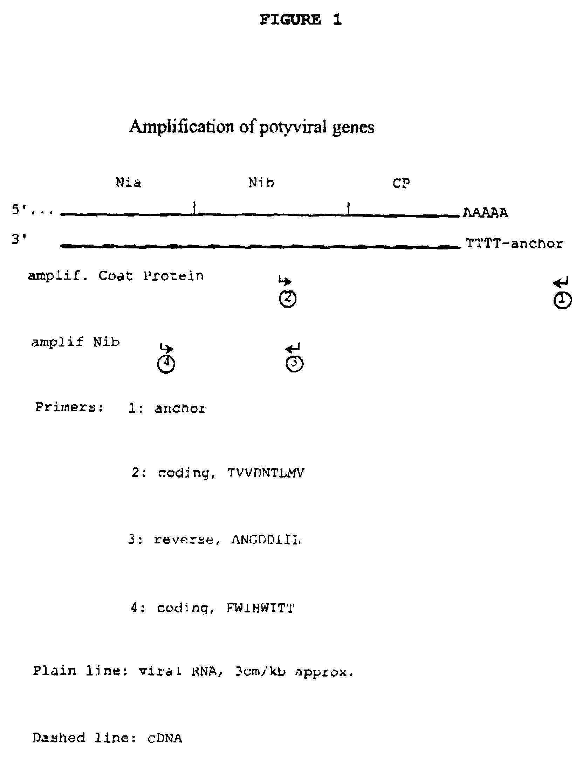

   Figure 1 shows the location of relevant conserved sequences identified on the genome of several potyviruses.

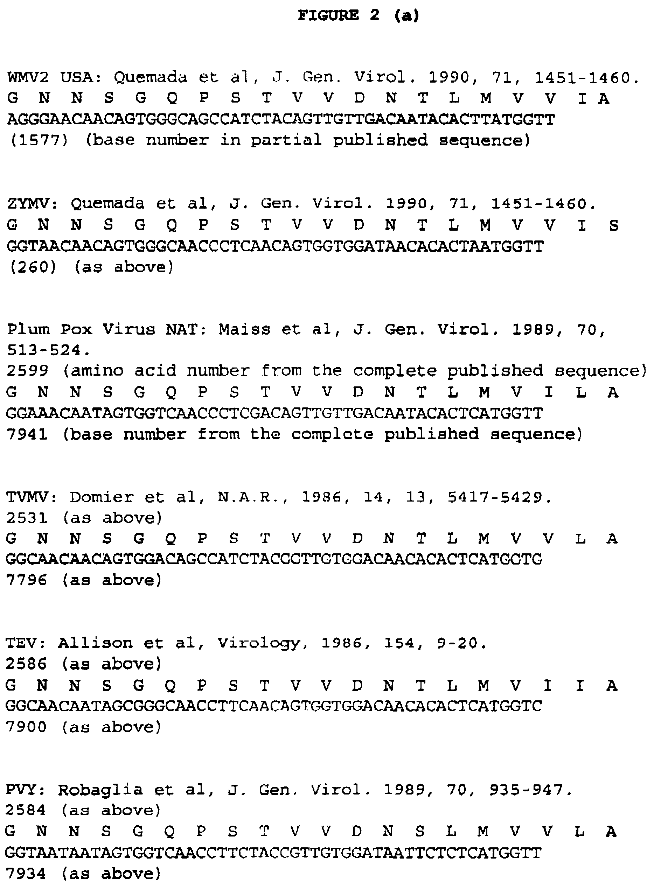

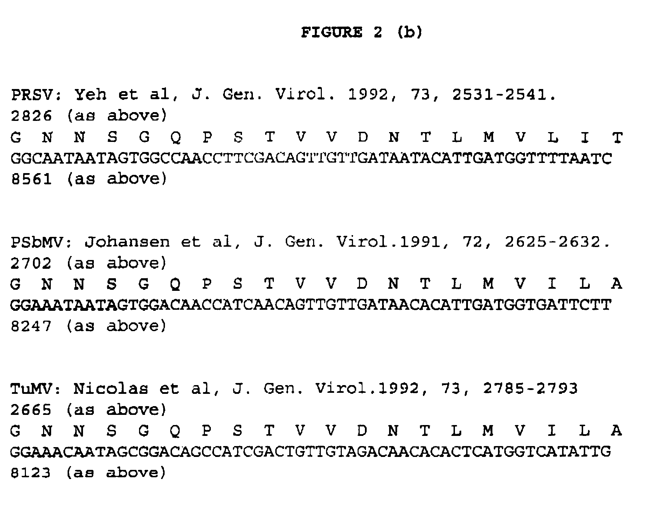

   Figure 2 shows the conserved sequence identified in the region coding for protein NIb used to design primer 2.

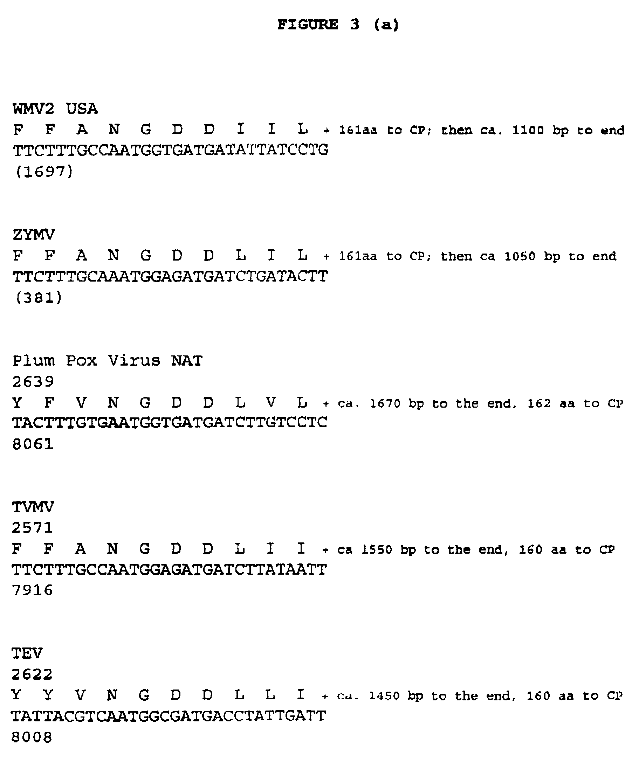

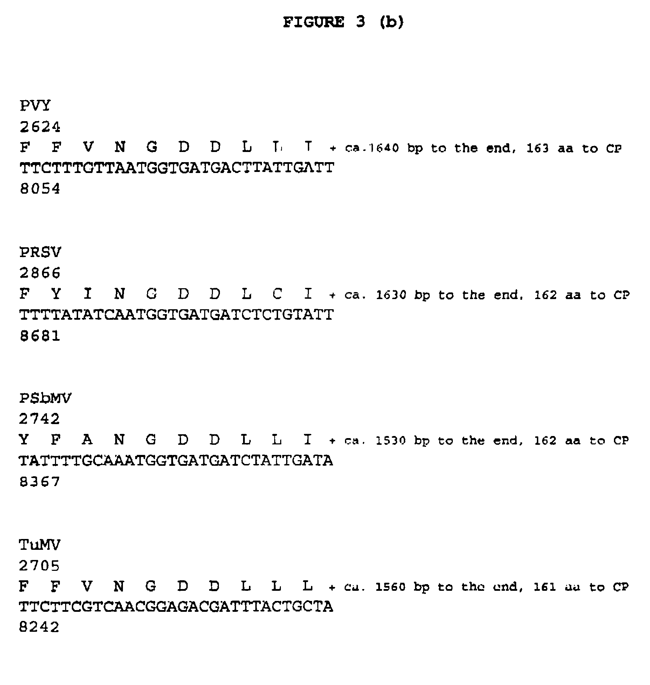

   Figure 3 shows the conserved sequence identified in the region coding for protein NIb used to design primer 3.

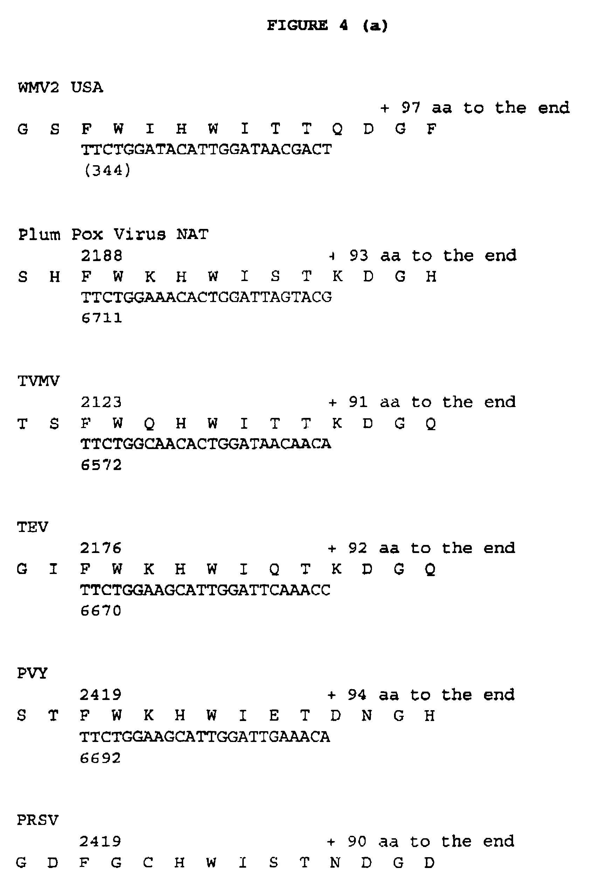

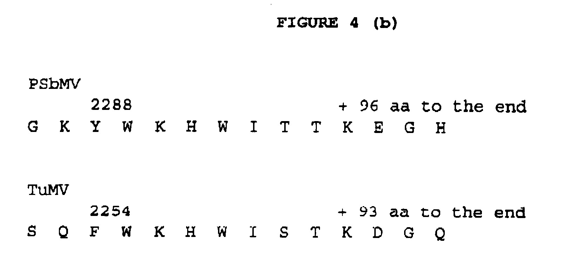

   Figure 4 shows the conserved sequence identified at the end of the region coding for protein Nia, and used to design primer 4.

   Reverse transcription was performed using the primer Poly-T-anchor:
   - Poly-T-anchor:: 5' GGGAGCTCGCTAGCGGATCCT₁₇

   Primer 1, used in the PCR step, is homologous to the anchor region, with extra bases added to facilitate cloning of the PCR products:
   Primer 1: 5' AATTGGGAGCTCGCTAGCGGAT
      The other primers were designed to match the consensus sequence of the conserved regions identified as described above, and some bases were added to facilitate cloning. The primers were moderately degenerate to take into account polymorphism. Multiple bases at a given position are indicated in the sequence by a / : T/G means that both T and G could be present in the primer at a given position. N means that any base could be present.
   Primer 2: 5' AATTGCTAGCAGTT/GGTT/GGACAATACACTT/AATGG
   Primer 3: 5' AATTCTAGAATA/CAT/GATCATCACCATTG/TGC
   Primer 4: 5' AATTCTAGATTCTGGAT/AACATTGGATAA/TC/GNAC
c - PCR products of the predicted size were obtained with primers pairs 1-2. (1.7 kb) and 3-4 (1.3 kb). The PCR products were purified on gel, cut with the appropriate restriction enzymes, cloned into plasmid BlueScript SK+ (commercially available from Stratagene) and the complete nucleotide sequence of the viral fragment was determined using standard methods known in the art. Most of the sequence was determined from two independent PCR products from each RNA template. For WMV2, a PCR product was also obtained with primer pair 1-4. It was used subsequently to build long arm ribozyme genes (see Example 3 below).

The sequence of the 3' region of WMV2 strain Mar6 is described in Figure 5 and the sequence of the 3' region of WMV2 strain Val3 is described in figure 5 bis. The two strains are more than 99% identical.

The sequence of the 3' region of ZYMV strain E15 is described in Figure 6. The E9 strain was more than 99% identical to E15.

### 2. Design of ribozymes against WMV2

Both the plus (+, coding, or genomic) RNA strand of the virus and the minus (-, replication intermediate) RNA strand of the virus were targeted by ribozymes.

The following criteria were used when choosing cleavage sites on the target RNA:

Triplets known to be well cleaved in vitro were preferred (Perriman ct al., 1992):
GUC, GUA, GUU, UUC, UUU.

Triplets where the codons had limited degeneracy were preferred.

This increases the chance that the target sequence will not evolve easily so that the virus overcomes the resistance. Some examples are shown below for illustration, with the cleaved triplet in **Bold**. Many more combinations are possible.
For the plus strand:

| | | | | |
|---|---|---|---|---|
| Aminoacid sequence: | MetSer | MetTyr | MetPhe | Phe |
| Nucleotide sequence: | AUGUCN | AUGUAU/C | AUGUUU/C | UUU/C |

For the minus strand:

| | | | | |
|---|---|---|---|---|
| Aminoacid sequence (+): | MetLys | MetThr | GluLys | Tyr |
| Nucleotide sequence (+): | 5'AUGAAA/G | AUGACN | GAA/GAAA/G | UAU/C |
| Nucleotide sequence (-): | 3'UACUUU/C | UACUGN | CUU/CUUU/C | AUA/G |

Triplets located in a region of sequence conservation were preferred. This would increase the chance that the ribozyme could target multiple strains of the virus or even multiple species of related viruses. It would also decrease the chance that a virus resistant to cleavage arises.

Four sites were selected on the plus strand and four sites were selected on the minus strand. The number used to describe a given ribozyme refers to the base underlined, on the + strand in the following tables, according to the numbering of the sequence presented in figure 5. Cleavage site is in **Bold** and the cleaved bond is indicated by a -.

Sites selected on the minus strand:

| Number | Target sequence Aminoacid sequence | + strand sequence |
|---|---|---|
| 696 | 5' **UUC** - A 3' 5' UGAA 3' | Met Lys |
| 996 | 5' **GUC** - A 3' 5' UGAC 3' | Met Thr |
| 1747 | 5' **GUA** - U 3' 5' AUAC 3' | Tyr |
| 2067 | 5' **UUU** - G 3' 5' CAAA 3' | Thr Lys |

Sites selected on the plus strand:

| Number | Target sequence | Aminoacid sequence |
|---|---|---|
| 2259 | 5' **UUU** - A 3' | Phe |
| 2403 | 5' **UUC** - U 3' | Phe |
| 2520 | 5' **UUU** - U 3' | Phe |
| 2720 | 5' **GUA** - A 3' | Gln Och |

Of these sites, site 2259 was conserved in ZYMV (at base 2249 on figure 6).

Site 1747 was highly conserved among most potyviruses: a comparison of a 23 base segment surrounding the site shows the following identity:
ZYMV: 23/23
Potato Virus Y: 20/22
sugar Cane Mosaic Virus: 19/23
Pea Seed-borne Mosaic Virus: 20/23
Plum Pox Virus: 20/23
Maize Dwarf Mosaic Virus: 20/23
The structure and properties of the ribozymes exemplifying the present invention are indicated below in Table 8.

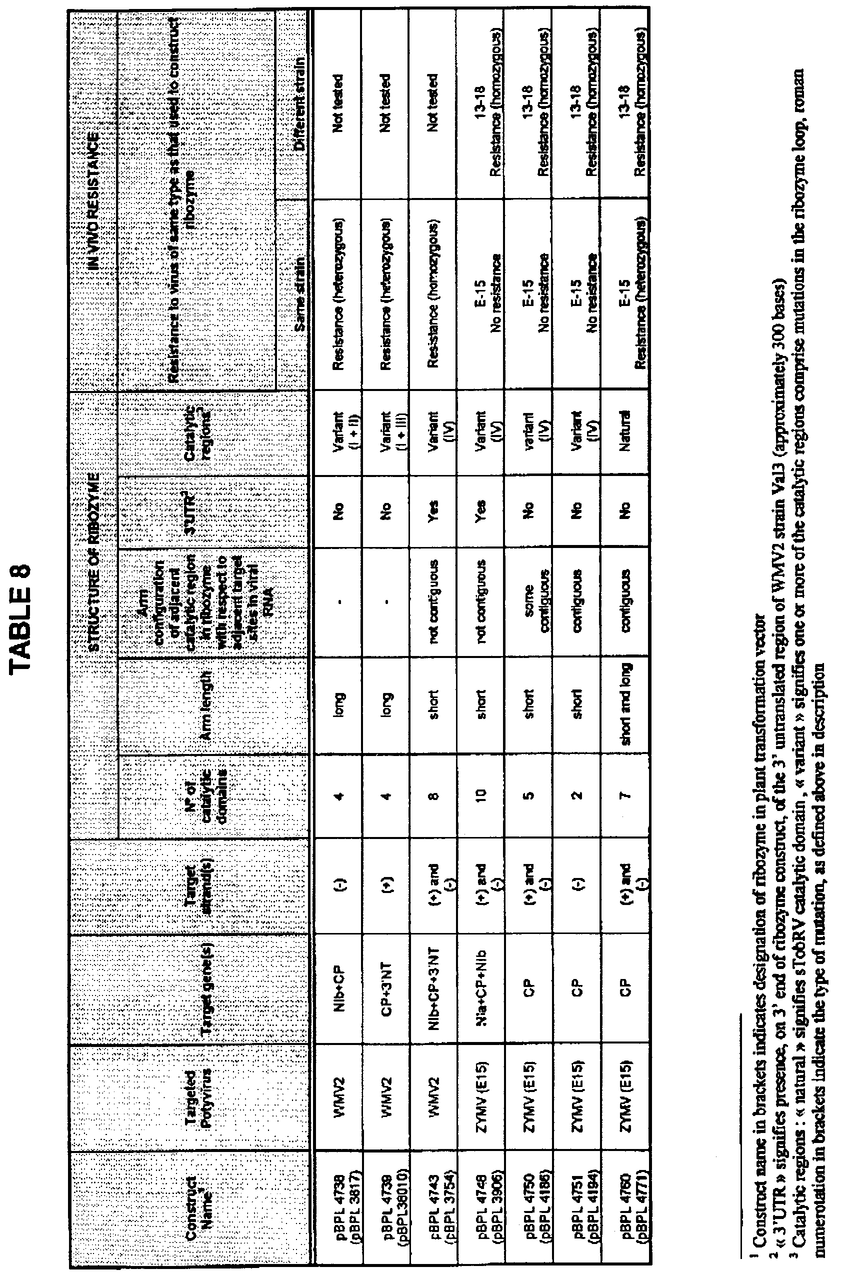

### 3. Construction of a long arm multimeric ribozyme gene against the minus strand of WMV2 (pBPL 4738)

A subclone of the cDNA for the 3' region of WMV2 Mar6, extending from base 269 (EcoRI site) to base 2168 (XhoI site) (see figure 5) was built by internal deletions of a plasmid containing the PCR product obtained with primers 1 and 4 as described in 1. above. This subclonewas used as a template for the next step. Inverse PCR (IPCR) reactions were used to insert a ribozyme catalytic sequence in replacement of the third base of each target triplet, essentially as described in Unit 3.17.3 of Current Protocols in Molecular Biology. The ribozyme catalytic sequence from the satellite of Tobacco Ringspot Virus (sTobRV, Haseloff and Gerlach, 1988) was used.

The primers used are listed below.

Bases in lower case are encoding the ribozyme catalytic sequence. Bases in upper case are complementary to the target region and used to prime the plasmid template. Bases paired with the first 2 bases of the cleaved triplet are in bold. For the first primer pair the sequence of the final product around site 696 is shown, to demonstrate that ligation of the IPCR product will produce a molecule with a functional catalytic sequence inserted in replacement of the third base of the target site.

Rbz696-3': 5'gtgaggacgaaAAAGCTGCTGTCGGTGCTC3'

Rbz696-5': 3'CTAAATACCTAGGGACTTGTAgactactcagg5'

Result for 696: 5'...GAACAUcugaugaguccgugaggacgaaAAACC...3'

Rbz996-3': 5'gtgaggacgaaACTAAGTTTTATGGGGTTGC3'
Rbz996-5': 3'CAGGTACCTGTCAACCGTAgactactcagg5'

Rbz1747-3': 5'gtgaggacgaaACATAGCTGAGACAGCATTG
Rbz1747-5': 3'CGTAGGCCTTTTCGAGGTgactactcagg5'

Rbz2067-3': 5'gtgaggacgaaAAGAAAATCAACCTTCCAACAG3'
Rbz2067-5': 3'GGGTGCTGATGTTTTCTATTGgactactcagg5'

The plasmid template was amplified by IPCR using the first pair of primers. The full length PCR product was purified on a gel, and circularised by ligation. The circular product was transformed into bacteria, and the resulting plasmid was extracted from a bacterial colony and submitted to the second IPCR reaction using the second pair of primers. The procedure was repeated with the third and fourth pair, and the final product was analysed by sequencing the modified regions. A plasmid called pBPL4734 was obtained; it had the correct sequence except for ribozyme 696 which had one extra base in the loop, and ribozyme 996, where a small deletion had taken place. Attempts were made to repair errors in 996 by a new round of IPCR. The final product was called pBPL4738 and had the expected sequence except at sites 696 and 996 where the catalytic sequence is mutated in the ribozyme loop (upper case):
- Mutant ribozyme 696:: cugaugaguccCGUGAggacgaa
- Mutant ribozyme 996 :: cugaugaguccUGAggacgaa
- Wild type sTobRV:: cugaugaguccGUGAggacgaa.

To build a functional ribozyme gene in a plant transformation vector, the multimeric long arm ribozyme sequence was cloned in the plant expression cassette from pBIOS512, made of the enhanced 35S promoter and the nos terminator, and the gene was then inserted in the plant transformation vector pSCK.

Description of pBIOS512: this plasmid was built by replacing the 35S promoter region of plasmid pBIOS3 (Perez et al., 1989) by the enhanced 35S promoter region from pJIT163, a plasmid derived from pJIT60 (described in Guerineau and Mullineaux, 1993) by inserting an NcoI site in the polylinker. The enhanced 35S promoter was isolated from pJIT163 as a SstI - BamHI fragment and cloned into sites SstI - BamHI of pBIOS3 in replacement of the 35S promoter.

Description of pSCK: this plasmid was built by inserting a kanamycin resistance gene into the plant transformation vector pSCV1 (Firek et al., 1993). The kanamycin resistance gene was built by inserting the nptII coding sequence from pCaMVNEO (Fromm et al., 1986) as a BaHI fragment in the BamHI site of pBIOS1 (Perez et al., 1989), resulting in pBIOS1K. The gene was then isolated from pBIOS1K as a EcoRI(Klenow) fragment and inserted into site EcoRV of pSCVl_{,} resulting in pSCK.

The ribozyme sequence was cloned in the expression cassette of pBIOS512, as a EcoRV-SmaI - HindIII fragment in the HindIII (Klenow) site of pBIOS512. The gene was then excised as a EcoICRI - EcoRI(Klenow) fragment and inserted into the SmaI site of pSCK.

The resulting plasmid was called pBPL3817. It contains a T-DNA with a marker gone conferring resistance to kanamycin and the long arm ribozyme gene expressing an RNA with approximately 1900 bases complementary to the minus strand of the virus (the target RNA) and targeting four sites on the target RNA.

### 4. Construction of a long arm multimeric ribozyme gene against the plus strand of WMV2 (pBPL 4739)

A procedure similar to that described in 3. was used to build a ribozyme gene against the + strand. The plasmid template used in the 1PCR reactions extended from base 2164 (site XhoI) to the end of the CDNA (base 2988, including 18 A of the polyA tail) and was obtained by internal deletions of a partial cDNA clone of the 3' region of WMV2.

Inverse PCR reactions were used as described above in 3. using the following primer pairs:
(same notations as above)

Rbz2259+3': 5'gtgaggacgaaAAACCATTCATAATCACACCC3'
Rbz2259+5': 3'CAATAGCTATGTGGTTTGGTAgactactcagg5'

Rbz2403+3': 5'gtgaggacgaaAAATGGTGCATGATTTGTC3'
Rbz2403+5': 3'CGAAGACGACGCAGACTgactactcagg5'

Rbz2520+3':                    5'gtgaggacgaaAAGTCAAAAGCATAGCGTGC3'
Rbz2520+5': 3'CACAAAACCTTCATTGGAGTATgactactcagg5'

Rbz2720+3':                    5'gtgaggacgaaACTGCGGTGGACCCATACC3'
Rbz2720+5': 3'CACTGGTCAAATGGATCAGAAgactactcagg5'

The plasmid obtained was called pBPL4736, and had half of ribozyme 2259 missing, and a one base deletion in the 3 other ribozymes' loop New rounds of IPCR inserted a correct ribozyme for 2259 and corrected ribozyme 2403. The final product still contained some errors which were not considered serious problems. The ribozyme sequence for sites 2259 and 2403 is correct (wild type as described). The ribozyme sequences for sites 2520 and 2720 each have one base deleted:
- Mutant ribozyme in 2520:: cugaugaguccUGAggacgaa
- Mutant ribozyme in 2720:: cugaugagucGUGAGgacgaa
- Wild type sTobRV:: cugaugaguccGUGAggacgaa

These mutations are known not to abolish ribozyme activity. This was confirmed by in vitro assays (see below). This plasmid was called pBPL4739.

The sequence from pBPL4739 was cloned in the expression cassette of pBIOS512 as a BamHI(Klenow) - EcoRI fragment in the HindIII(Klenow) site of pBIOS512. The gene was then transferred to the plant transformation vector pSCK as described above in 3.

The resulting plasmid was called pBPL38010. It contains a T-DNA with a marker gene conferring resistance to kanamycin and the ribozyme gene expressing an RNA with approximately 830 bases complementary to the plus strand of the virus (the target RNA) and targeting four sites on the target RNA.

### 5. Construction of a short arm multimeric ribozyme gene against both strands of WMV2 (pBPL 4743)

In order to build a short arm multimeric ribozyme targeting the sites described above for the long arm ribozymes, synthetic oligonucleotides encompassing the entire sequence of the desired ribozyme gene were purchased. The oligonucleotides were assembled and the desired full length product was obtained using Ligation based PCR (LbPCR) as described by Betzner et al. (1997). The short arm multimeric ribozyme genes against the minus strand and the plus strand were synthesised independently and assembled using restriction sites built at the end of the synthetic sequences.

The ribozyme catalytic sequence used for these constructs was a variant of the sTobRV ribozyme described above, with a 3 base pair helix II instead of the natural 4 base-pair. The ribozyme used for the short multimeric ribozyme was :
cugaugaGCGgugaCGCgaa.
(Helix II shown here in upper case letters)

The pairing regions between the ribozymes and the target (helix I and helix III) was made of 2 arms of 10 to 12 bases (see Haseloff and Gerlach, 1988 for a description of helices I, II, and III).

The sequence of the oligonucleotides used is given below; lower case letters are the ribozyme catalytic sequence, lower italic letters are linker regions, upper case letters are target sequences. All sequences are shown 5' to 3'. The sequence synthesized (shown) is actually the strand complementary to the ribozyme RNA.

Oligonucleotides used for the ribozymes against minus strand: 
WM2067S :
gcagctgctggatccatgTCATTTTCTTtcgcgtcaccgctcatcagGTTATCT
TTTG
- WM1747S :: TCAGCTATGTttcgcgtcaccgctcatcagTGGAGCTTTTC
- WM996S :: *Cagatct*ATAAAACTTAGTttcgcgtcaccgctcatcagATGCCAACTG 
WM696S :
CAGCAGCTTTttcgcgtcaccgctcatcagATGTTCAGGGAcctgcatctgatca
agcttg
 Guide oligonucleotides used to align the full length sequence for the ligation reaction:
- WMGU4S:: assemble WM2067S with WM1747S GAAACATAGCTGACAAAAGATAACCTG
- WMGU5S:: assemble WM1747S with WM996S GTTTTATAGATCTGGAAAAGCTCCAC
- WMGU6S:: assemble WM996S with WM696S GAAAAAGCTGCTGCAGTTGGCATC Oligonucleotides used for the ribozymes against plus strand: 
WM2259S:
gcagctgctggatccATGAATGGTTTttcgcgtcaccgctcatcagATGGTTTGG
- WM2403S:: TGCACCATTTttcgcgtcaccgctcatcagTCAGACGCAG*at*
- WM2520S:: CTTTTGACTTttcgcgtcaccgctcatcagTATGAGGTTAC 
WM2720S:

CACCGCAGTttcgcgtcaccgctcatcagAAGACTAGGTAAcctgcatctgatca

agcttg
 Guide oligonucleotides used to align the full length sequence for the ligation reaction:
- WMGU1S:: assemble WM2259S with WM2403S GAAAAATGGTGCACCAAACCATCTG
- WMGU2S:: assemble WM2403S with WM2520S CGAAAAGTCAAAAGATCTGCGTCTGAC
- WMGU3S:: assemble WM2520S with WM2720S AAACTGCGGTGGTAACCTCATACTG Primers used to amplify the assembled ligated oligonucleotides by PCR:
- WMPC1S:: AATTGCAGCTGCTGGATCCATG
- WMPC2S:: AATTCAAGCTTGATCAGATGCAG

The PCR products were digested with BamHI and HindIII and cloned into BlueScript SK+. The ribozyme against the minus strand gave plasmid pBPL4741, and the ribozyme against the plus strand gave pBPL4742. The sequences of the relevant regions are on figure 7. Restriction sites used for further cloning are indicated.

The 2 ribozymes were combined by inserting the BstXI - BspMI fragment of pBPL4742 in sites BstXI - BamHI of pBPL4741, yielding pBPL4743.

The 3' non coding region of WMV2 strain Val3 (3' UTR) was then cloned in 3' of the multimeric ribozyme sequence. This was done as an attempt to increase the efficacy of the ribozyme by co-compartmentalisation with the target viral RNA. If 3' end sequences of RNA are involved in cellular localisation of RNA, as is the case with many RNAs, it was hoped that by providing the ribozyme RNA with a 3' sequence similar to that of its target, the 2 molecules would be present in the same cellular compartment.

The 3' UTR region of WMV2 Val3 was cloned from the original cDNA (see sequence in figure 5 bis) as a HinfI (at base 2678) - BamHI (from the anchor region in primer 1, see 1. above) fragment into EcoRI - BamHI of BlueScript SK+. The fragment contains bases 2678 to 2970 of strain Val3 cDNA, which contains the last 14 codons of the cDNA followed by the complete untranslated region. This sequence (shown in figure 7) was then inserted as an EcoRV - NotI fragment into BamHI(Klenow) - NotI sites of pBPL4743, giving pBPL4744.

The now complete sequence was then transferred as a BamHI - Hind III fragment in sites BamHI - HindIII of pBIOS512 to create a functional gene, as described above in 3. This gene was then cloned in the SmaI site of pSCK as described above in .3., yielding pBPL3754.

pBPL3754 contains a T-DNA with a marker gene conferring resistance to kanamycin and the ribozyme gene expressing an RNA approximately 700 bases long. This RNA contains 8 regions of homology to the plus or minus strand of the virus. Each region is between 20 and 22 bases long, and is split in the middle by the ribozyme catalytic sequence. In addition the ribozyme RNA has a 3' region containing approximately 300 bases identical to the viral 3' end.

### 6. Construction of several short arm multimeric ribozyme genes against ZYMV

Site selection will be described using the same notations as in 2. above. The numbering refers to the sequence shown on figure 6.

Sites selected on the minus strand:

| Number | Target sequence + strand sequence | Aminoacid sequence |
|---|---|---|
| 1245 | 5' **GUU** - G 3' 5' CAAC 3' | Asn |
| 1743 | 5' **GUA** - U 3' 5' AUAC 3' | Tyr |
| 1929 | 5' **GUC** - A 3' 5' UGAC 3' | Asp |
| 2009 | 5' **UUC** - A 3' 5' UGAA 3' | ValAsn |
| 2027 | 5' **UUC** - C 3' 5' GGAA 3' | GlyLys |
| 2354 | 5' **UUU** - U 3' 5' AAAA 3', | GluAsn |
| 2442 | 5' **GUA** - U 3' 5' AUAC 3' | Tyr |
| 2564 | 5' **UUC** - A 3' 5' UGAA 3' | MetLys |

Sites selected on the plus strand:

| Number | Target sequence | Aminoacid sequence |
|---|---|---|
| 110 | 5' **GUC** - U 3' | Val |
| 2161 | 5' **GUC** - U 3' | AlaSer |
| 2255 | 5' **GUU** - U 3' | Val |
| 2455 | 5' **GUA** - U 3' | ArgTyr |
| 2466 | 5' **UUC** - G 3' | LeuArg |
| 2590 | 5' **UUC** - U 3' | ValSer |
| 2603 | 5' **UUU** - G 3' | Phe |

Several combinations of short arm ribozymes were built using synthetic oligonucleotides. The procedure was similar to the procedure described in 5. above.

### 6.1. Construction of pBPL 4748

The first combination contained 10 ribozyme activities, 5 against each strand:
1245 - 1743 - 1929 - 2442 - 2564 against the minus strand (plasmid pBPL4747)
110 - 2161 - 2255 - 2455 - 2590 against the plus strand (plasmid pBPL4746)

The ribozyme catalytic sequence used for these constructs was the same variant of the sTobRV ribozyme as described in 5. above, with a 3 base-pair GCG helix II instead of the natural 4 base-pair.

The pairing regions between the ribozymes and the target (helix I and helix III) was made of 2 arms of between 8 and 11 bases.

Sequence of the relevant region of pBPL4746 and pBPL4747 is shown on figure 8.

The sequences were assembled together with the 3'UTR of WMV2 Val3 (shown on figure 7), by a tripartite ligation: the EcoRV(de phosphorylated) - NotI fragment containing the 3'UTR and the BamHI(Klenow) - HindIII(Klenow) fragment from pBPL4747 were ligated together in pBPL4746 cut with BamHI (Klenow) and NotI (both sites de phosphorylated). The resulting plasmid was called pBPL4748 and contained (from HindIII to BamHI) the sequence for the ribozymes against the plus strand, followed by the ribozymes against the minus strand followed by the 3'UTR. The complete sequence was then cloned as a BamHI - HindIII fragment in the expression cassette of pBIOS512 as described in 3. above, and the functional gene was then cloned into the plant transformation vector pSCK as described in 3. above yielding pBPL3906.

Plasmid pBPL3906 contains a T-DNA with a marker gene conferring resistance to kanamycin and the ribozyme gene expressing an RNA approximately 750 bases long. This RNA contains 10 regions of homology to the plus or minus strand of ZYMV. Each region is between 16 and 20 bases long, and is split in the middle by the ribozyme catalytic sequence. In addition the ribozyme RNA has a 3' region containing approximately 300 bases identical to the non-translated 3' end of WMV2 Val3.

### 6.2 Construction of pBPL4750

A second combination was made using the following ribozymes:
2354 (-) - 2455 (+) - 2466 (+) - 2590 (+) - 2603 (+).
The synthetic sequence cloned into BlueScript SK+ yielded pBPL4750.

This was an attempt to capitalise from the clustering of sites 2455-2466 and 2590-2603.

The ribozyme catalytic sequence used for these construct was the natural sTobRV hammerhead ribozyme except for 2354 and 2603 where the same variant of the sTobRV ribozyme as described in 5. above, with a 3 base-pair GCG helix II instead of the natural 4 base-pair was used.

The pairing regions between the ribozymes and the target (helix I and helix III) was made of 2 arms of between 8 and 12 bases. Adjacent ribozymes 2455-2466 and 2590-2603 had one arm in common.

The relevant sequence of pBPL4750 is shown on figure 8. The ribozyme sequence was cloned directly as a BamHI - HindIII fragment in the expression cassette of pBIOS512 as described in 3. above, and the functional gene was then cloned into the plant transformation vector pSCK as described in 3. above yielding pBPL4186.

Plasmid pBPL4186 contains a T-DNA with a marker gene conferring resistance to kanamycin and the ribozyme gene expressing an RNA approximately 250 bases long. This RNA contains 3 regions of homology to the plus and minus strand of ZYMV. One region of 21 bases is split in the middle by one ribozyme catalytic sequence. The other 2 regions of 31 and 32 bases are each split in three parts by two ribozyme sequences.

### 6.3. Construction of pBPL 4751

A third combination, was made using a double ribozyme against sites 2009-2027 (both minus strand). The synthetic sequence cloned into BlueScript SK+ yielded pBPL475l.

The ribozyme catalytic sequence used for this construct was the same variant of the sTobRV ribozyme as described in 5. above, with a 3 base-pair GCG helix II instead of the natural 4 base-pair.

The pairing regions between the ribozymes and the target (helix I and helix III) was made of 2 arms of 10 and 17 bases for each ribozyme. The 17 base arm was shared between the 2 ribozymes. In effect the total pairing region was 37 bases (10 + 17 + 10), interrupted in 2 places by the 2 ribozyme sequences

The relevant sequence of pBPL4751 is shown on figure 8. The ribozyme sequence was cloned directly as a BamHI - HindIII fragment in the expression cassette of pBIOS512 as described in 3. above, and the functional gene was then cloned into the plant transformation vector pSCK as described in 3. above yielding pBPL4194.

Plasmid pBPL4194 contains a T-DNA with a marker gene conferring resistance to kanamycin and the ribozyme gene expressing an RNA approximately 100 bases long. This RNA contains 1 region of homology to the minus strand of ZYMV. The region is 37 bases long, and is split in three parts by the 2 ribozyme catalytic sequences.

### 7. Construction of a long arm multimeric ribozyme gene against ZYMV

A single gene, expressing one polyribozyme RNA targeting several sites on both strands was made.

The sites chosen were (see 6. above for numbering):
2009 (-) , 2027 (-) , 2354 (-) , 2455 (+) , 2466 (+) , 2590 (+) and 2603 (+).

To create the appropriate template, two fragments from the cDNA of the 3' region of ZYMV were cloned in opposite orientation as follows (see figure 6 for sequence).

The cDNA clone obtained from ZYMV E15 with PCR primers 1 and 2 (as described in 1. above) was cut with NsiI (at base 2383) and SacII (in the polylinker, next to 5'end of the cDNA), than treated with Klenow and do phosphorylated. The large fragment was then purified and used as a vector in the subsequent ligation. The same plasmid was cut with NsiI (at base 2383) and AlwNI (at base 1955), then treated with Klenow, and the 470 base fragment was purified and used as the insert in the subsequent ligation. The clones obtained were screened for the appropriate orientation, and the selected plasmid was called pBPL0157. This plasmid contains the sequence between bases 2383 and 1961 linked to the sequence between bases 2388 and 2922. The ligation places side by side the region 3' of 1961 and the region 3' of 2383. When a promoter is placed in the appropriate orientation, the transcription product of this sequence will be complementary to the minus strand of ZYMV between base 1961 and 2383, and complementary to the plus strand of ZYMV between base 2388 and 2922.

Ribozyme catalytic sequences were inserted at the positions mentioned above using a sequence of IPCR reactions on template plasmid pBPL0157, following the procedure described for WMV2 in 2. above. The ribozyme catalytic sequence used for this construct was the natural sTobRV hammerhead ribozyme as described previously.

The insertion of the catalytic sequence targeting site 2354 (-) proved difficult, as if this ribozyme sequence was toxic to E. coli or affected plasmid replication.

The plasmid containing the 7 ribozyme sequences was called pBPL4760. Sequence of the ribozyme gene in pBPL4760 is shown in figure 9.

The complete sequence was recovered using HindIII (which cleaves at base 2866) and SacI(Klenow) (from the polylinker) and the fragment was cloned in the expression cassette of pBIOS512 in sites HindIII - SalI (Klenow). This had the effect to remove from the region targeting the plus strand the last 51 nucleotides. The functional gene was then transferred to the plant transformation vector pSCK as described previously, yielding plasmid pBPL4771.

Plasmid pBPL4771 contains a T-DNA with a marker gene conferring resistance to kanamycin and the long arm ribozyme gene expressing an RNA with approximately 900 bases complementary to the plus and minus strand of ZYMV RNA and targeting seven sites on the target RNAs.

### 8. In vitro assay of the ribozymes

Ribozyme activity was assayed in vitro by incubating an appropriate target RNA prepared by in vitro transcription and uniformly labelled with radioactive UTP, with the ribozyme RNA prepared by in vitro transcription. The protocol is described in detail below and the result for each ribozyme are also shown.

### Target RNA preparation.

The plasmids used for target RNA synthesis were all derived from the plasmids containing the original PCR amplified viral sequences described in 1. above. The T3 and T7 promoters present in the BlueScript vector were used to transcribe the plus or the minus strand of the cloned fragment as appropriate. In vitro transcription using T3 RNA Polymerase or T7 RNA Polymerase from Promega or New England Biolabs was done following the suppliers instructions (for example in Promega Protocols and Application Guide, available from the Promega company, Madison, Wisconsin, USA), in the presence of radioactive α³²p-UTP. The template plasmid was linearised with an appropriate restriction enzyme prior to transcription. The target RNA was quantitated by measuring the incorporation of radiolabel using the DE81 filter binding assay as described in the Promega Protocols. Target RNA was stored as a precipitate under ethanol at -20°C, and resuspended in water at between 0.01 and 0.1 pmol/µl when needed. Specific activity was usually between 20,000 and 200,000 cpm/pmol.

### Ribozyme RNA preparation

The plasmids described above in sections 3, 4, 5, 6 and 7, made of the various polyribozyme sequences cloned into BlueScript, were used as templates for in vitro transcription reactions as described above, but using only non radioactive nucleotides. A pilot reaction with a small amount of radioactive tracer was done separately to quantitate the synthesised RNA, using the DE81 filter binding assay. Ribozyme RNA was stored as a precipitate under ethanol at -20°C and resuspended in water at between 0.1 and 1 pmol/µl when needed.

### Ribozyme reaction

The target RNA and ribozyme RNA were mixed in T7 RNA Polymerase buffer (from New Englands Biolabs) and incubated at various temperature (22°C, 37°C or 50°C). The standard reaction was in 4 µl and contained 0.005 to 0.1 pmol of target, and between 1 and 10 fold molar excess of ribozyme. The reaction was stopped by adding an equal volume of stop-loading buffer (Formamide containing 10mM EDTA and 1mg/ml each Xylene Cyanol and Bromophenol Blue) to the reaction and freezing the sample at -20°C until all samples were ready. The reactions were then boiled for 30 seconds, placed on ice, and loaded on a denaturing polyacrylamide gel in TBE buffer with 7M urea. Polyacrylamide concentration was between 4 and 6 % depending on the expected length of the products. The gel was transferred to a Whatmann 3MM paper, dried and exposed to a PhosphorImager screen. An autoradiogram of the gel was obtained by scanning the screen using a PhosphorImager.

### Long arm ribozyme against the minus strand of WMV2

The in vitro assays of long arm ribozymes proved difficult Since the target and the ribozymes do not readily dissociate prior to loading and/or reassociate in the gel. The data are therefore of poor quality and difficult to interprete.

The ribozyme used was obtained from pBPL4734 (described in 3. above). The target contained 1898 bases of viral sequence, complementary to the sequence from base 2168 (site XhoI) to base 270 (site EcoRI) of WMV2 Mar6, with some sequence from the polylinker of BlueScript KS+ (between the T3 promoter and the XhoI site).

Figure 10 shows the result of one assay. There was 4 fold excess ribozyme and the reaction was done at 50°C for 30, 60, and 90 mn. Products clearly identifiable include the fragments generated by cleaving all four sites (shown by arrows on the gel). However it seems that sites 996 and 1747 were preferentially cleaved.

### Long arm ribozyme against the plus strand of WMV2

The ribozyme used was obtained from pBPL4736 (described in 4. above). The target contained 807 bases of viral sequence, between base 2163 (site XhoI) and base 2970 (start of polyA tail), with the polyA tail and polylinker sequence from BlueScript KS+ (between the T7 promoter and the EcoRI site).

Figure 11 shows the result of one assay. Incubation of target with 4 fold excess ribozyme at three different temperatures showed that site 2259 was not cleaved as expected because of the mutation in that ribozyme (see 4. above). Site 2720 was the fastest cleaver and gave products at 22°C. Site 2403 was next and site 2520 appears the slowest.

When the ribozyme from plasmid pBPL4739 was tested, evidence of cleavage at site 2259 was seen (data not shown).

### Short arm ribozyme against the minus strand of WMV2

The ribozyme used was obtained from pBPL4741 (described in 5. above) The target contained 1880 bases of viral sequence complementary to the sequence bewteen base 2278 (ClaI site) and base 398 (SpeI site), with polylinker sequence in 5' from BlueScript KS+ (betwen the T3 promoter and the ClaI site).

Figure 12 shows the result of on assay. All four sites are cleaved at high temperature. At 22°C, all sites except 696 are cleaved but cleavage is very slow as demonstrated by the very low abundance of double cleaved products.

### Short arm ribozyme against the plus strand of WMV2

The ribozyme used was obtained from pBPL4742 (described in 5. above). The target was the same as described above for the long arm ribozyme.

Figure 13 shows the result of an assay. Note that the molar excess ribozyme over the substrate was 4.5 (+), 9 (++) and 13.5 (+++). All four sites are cleaved at 50°C; with four fold excess ribozyme, cleavage is essentially complete after 1.0 to 1.5 hour. At 22°C, only sites 2259 and 2403 are cleaved, with ribozyme 2259 being the fastest cleaver.

### Short arm ribozyme against ZYMV: first combination (pBPL4746 and pBPL4747)

The 2 polyribozymes showed some cleavage activity at 50°C, but very little at 37°C and none detectable at 22°C.

At 50°C, for the minus strand, all sites were cleaved; site 2442, 1743, and 1245 were cleaved better than 2564 and 1929.

For the plus strand, all sites were cleaved; sites 110, 2590 and 2255 were cleaved faster than 2455 and 2161.

### Short arm ribozyme against ZYMV: second combination (pBPL4750)

The polyribozyme showed cleavage activity on the plus strand at all 4 predicted sites only at 50°C. No activity was detected at 22°C. The same polyribozyme however showed good cleavage activity on the only site present on the minus strand (site 2354) both at 50°C and 22°C.

Figure 14 shows the result of an assay of this ribozyme on the minus strand target. The target contained 1058 bases of viral sequence, complementary to the sequence between base 2633 (SpeI site) and base 1575 (XbaI site) as shown on figure 6, with 39 bases added in 5' (polylinker sequence between the T3 promoter and the XbaI site of BlueScript SK+) and 11 bases added in 3' (polylinker sequence between the XbaI and the BamHI site of SK+; BamHI was used to linearise the plasmid prior to in vitro transcription). For this experiment the labelled ribozyme from the pilot reaction used to quantitate was mixed with the unlabelled ribozyme. The position of the ribozyme band on the gel is shown by an arrow. The ribozyme was used at 5 fold molar excess over the substrate.

### Short arm ribozyme against ZYMV: third combination (pBPL4751)

The ribozyme from pBPL4751 (targeting sites 2009 and 2027 on the minus strand) was assayed on the same target as the ribozyme from pBPL4750.

Figure 14 shows the result of this assay. Both sites were cleaved efficiently at both 22°C and 50°C. The presence of a two bands of equal intensity for the products 5' - 2 and 5' - 3 (see figure 14) suggest that site 2009 is cleaved more readily or faster than 2027: about 50% of cleaved products are cleaved only at 2009.

### 9. Determination of resistance in transgenic plants

### 9.1. Genetic transformation of melon

The method used is described in the following patent applications : French application n°8910848 of 11/08/89, European application n°904022829 of 09/08/90 and United states patent n° 5,422,259 of 06/06/95.

Genetically transformed plants regenerated, checked by PCR amplification for the presence of nptII gene, were transferred into pots containing a mixture of compost and coarse sand (2:1) and placed in culture in a greenhouse. During the first seven days, the plants are protected by a plastic cover in order to maintain a humid atmosphere. The plants are watered daily. The plants are induced to flower, followed by self-fertilization. The seeds (T1 generation) are collected.

### 9 2. Artificial inoculation of WMV2 and ZYMV on melon to detect resistant plants

### Virus strains :

The strain of WMV2 used is called 〈〈 MAR 〉〉.
Two strains of ZYMV pathotype 0 are used:
- one called 〈〈 13-18 〉〉 (pathotype F) is a wilting strain because of the symptoms induced on susceptible cultivars which bear the Fn gene (Flaccida necrosis) (Lecocq and Pitrat, 1984).
- one called 〈〈 E15 〉〉 (pathotype NF) induces vein clearing symptoms for all the susceptible cultivars (Lecocq and Pitrat, 1984) independent of the presence or not of the Fn gene.

### Virus storage :

Leaves showing symptoms (vein clearing) are thinly cut with a razor blade and placed in boxes with calcium chloride to dry them. The boxes are stored at 6°C during a maximum of one year (Bos, 1969).

### Virus multiplication :

Melon plantlets of a susceptible variety at the two leaf stage are inoculated by rubbing the two cotyledons with the virus solution (Marrov, 1967).

### Virus solution :

One gram of leaves showing symptoms or one gram of the stored virus is ground in 4 ml of extraction buffer (1.075 g of disodium hydrogen phosphate, added with 0.2 g of sodium diethyldithiocarbamate for 100 ml of distilled water) added with activated charcoal (0.1 g) and carborundum (0.0875 g) (Marrov, 1967). This virus solution has to be kept at cool temperature.

### Virus inoculation :

Forty-six to forty-eight seeds (Tₗ progeny) per genetic transformation event are sown in 10 cm square pots filled with a potting soil. The temperature is maintained from 18 to 25 °C with a photo period of 12 hours. Mechanical inoculation on melon plantlets is carried out at the first leaf stage (approximately 15 days after sowing) (Marrov, 1967). The plantlets are inoculated in the same way as for virus multiplication.

### Observation of the symptoms :

The symptoms are scored between 2 and 4 weeks after inoculation.
For WMV2:
- susceptible plant: vein banding
- tolerant plant: light vein banding or symptoms appearing late
- resistant plant: no symptoms For ZYMV:
- susceptible plant (absence of the Fn gene): vein clearing with the E15 or the 13-18 strains.
- susceptible plant (presence of the Fn gene): vein clearing with E15 strain and wilting reaction with the 13-18 strain.
- resistant plant: no symptoms either with E15 or 13-18 strain.

In some cases, the control plants do not show symptoms of virus attack due to bad inoculation of the cotyledons. The behaviour of these control plants enables the determination of the value of the pathology test. 〈〈 Escape 〉〉 plants are considered to have escaped from a 〈〈 correct 〉〉 virus inoculation.

### 9.3. Resistance of transformed plants to WMV2 and ZYMV

The transformed plants are demonstrated to be resistant to WMV2 and to ZYMV.

### A. Resistance to WMV2 of melon plants genetically transformed with pBPL3754

With construct pBPL3754, eight genetic transformation events were analysed for WMV2 resistance behaviour (see table 1).

For six genetic transformation events, a resistance to WMV2 was observed. The segregation ratio (susceptible resistant) was 3:1, as expected for a single locus being introduced.

### B. Resistance to WMV2 of melon plants genetically transformed with pBPL38010

With construct pBPL38010, fifteen genetic transformation events were analysed for WMV2 resistance behaviour (see table 2).

For twelve genetic transformation events, a resistance to WMV2 was observed. The segregation ratio (susceptible:resistant) was 3:1 for nine genetic transformation events. For one genetic transformation event, the segregation ratio (susceptible:resistant) was 1:3.

### C. Resistance to WMV2 of melon plants genetically transformed with pBPL3817

With construct pBP13817, twenty-one genetic transformation events were analysed for WMV2 resistance behaviour (see table 3).

For fourteen genetic transformation events, the segregation ratio (susceptible: resistant) was 1:3 and for three genetic transformation events, the segregation ratio (susceptible:resistant) was 3:1.

### D. Resistance to ZYMV of melon plants genetically transformed with pBPL3906

With construct pBPL3906, thirteen genetic transformation events were analysed for ZYMV resistance behaviour (see table 4)

With the strain 13.18, the progeny of ten genetic transformed plants was tested. Four genetic transformation events showed a resistance segregation with a ratio (susceptible:resistant) of 3:1.

With the strain E15, all the progeny tested from nine genetic transformation events were susceptible.

### E. Resistance to ZYMV of melon plants genetically transformed with pBPL4186

With construct pBPL4186, five genetic transformation events were analysed for ZYMV resistance behaviour (see table 5).

One genetic transformation event from the five tested showed a segregation resistance (ratio susceptible:resistant of 3:1) when tested with the strain 13.18.

With the strain E15, the progeny from the three independent genetic transformation events tested were susceptible.

### F. Resistance to ZYMV of melon plants genetically transformed with pBPL4194

With construct pBPL4194, eight genetic transformation events were analysed for ZYMV resistance behaviour (see table 6).

With the strain 13.18, the progeny of four genetic transformation events from the seven tested showed a resistance segregation with a ratio (susceptible:resistant) of 3:1.

With the strain E15, the progeny of the six genetic transformation events tested were susceptible.

### G. Resistance to ZYMV of melon plants genetically transformed with pBPL4771

With construct pBPL4771, six genetic transformation events were analysed for ZYMV resistance behaviour (see table 7).

With the strain 13.18, two genetic transformation events were studied. The progeny of one genetic transformation event showed a segregation ratio (susceptible;resistant) of 3:1 and the other one a ratio of 1:3.

With the strain E15, the progeny of two genetic transformation events from the six tested showed a resistance segregation with a ratio (susceptible:resistant) of 1:3.

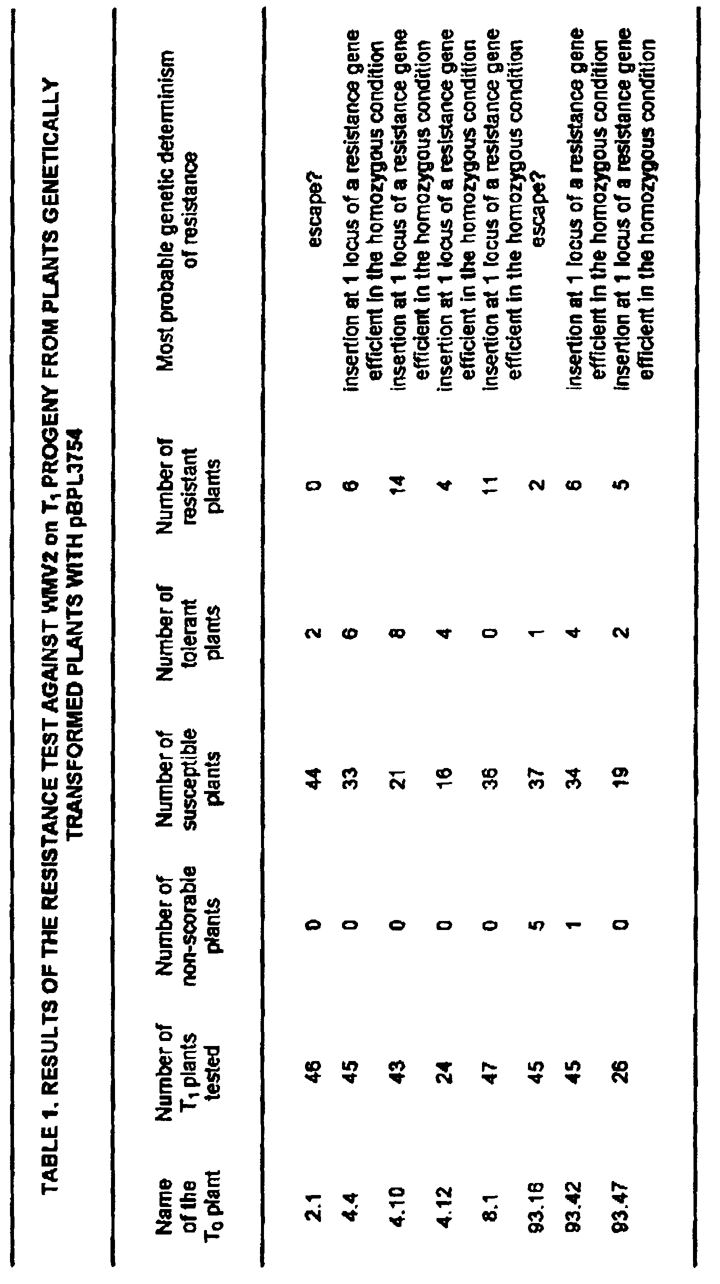

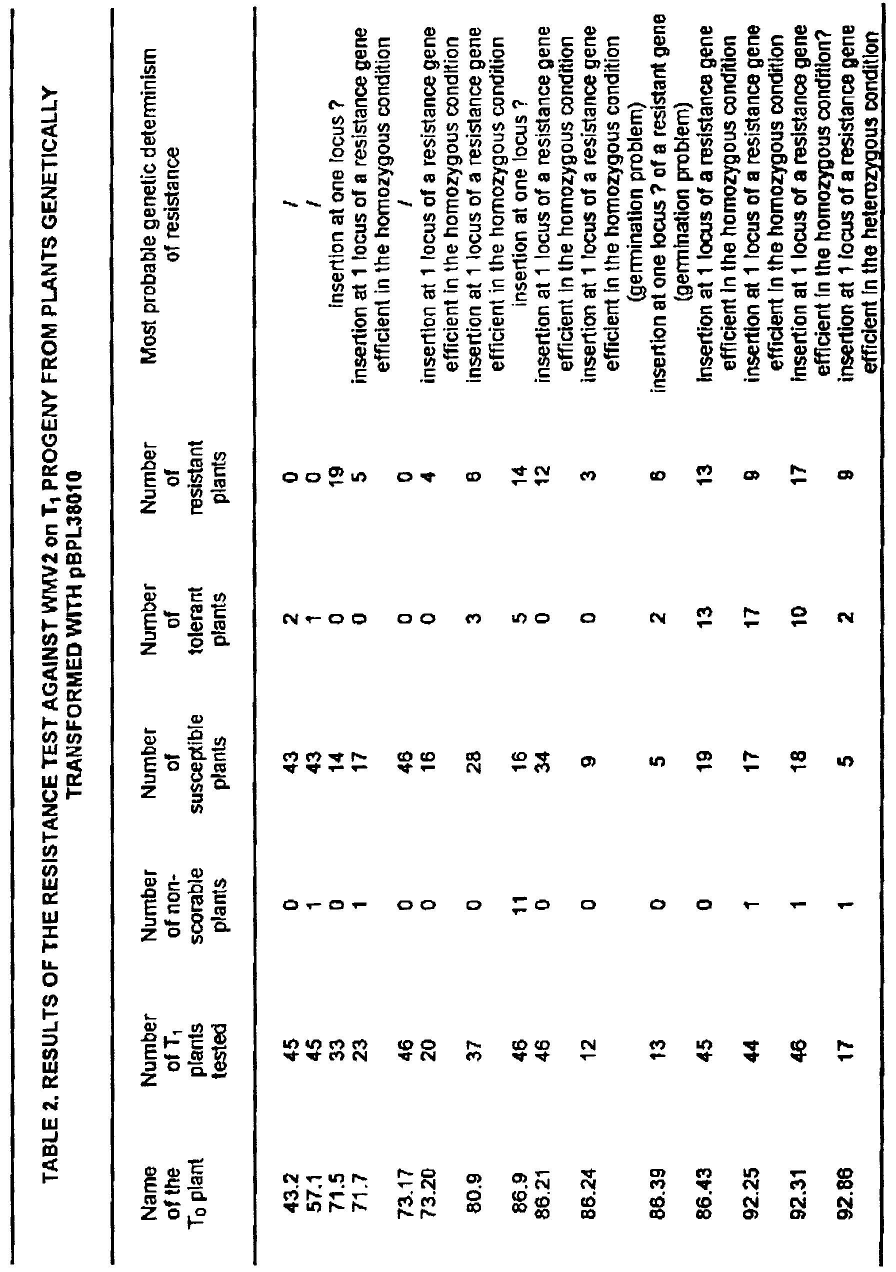

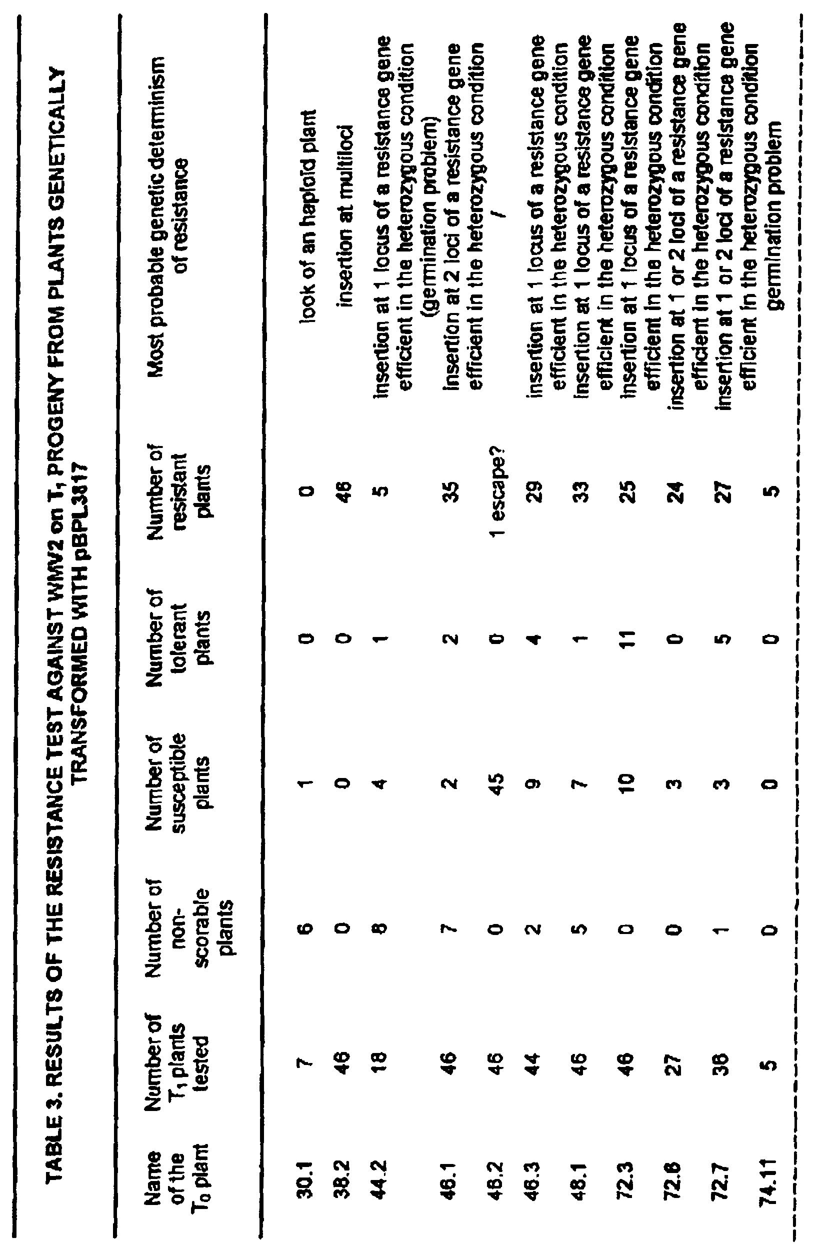

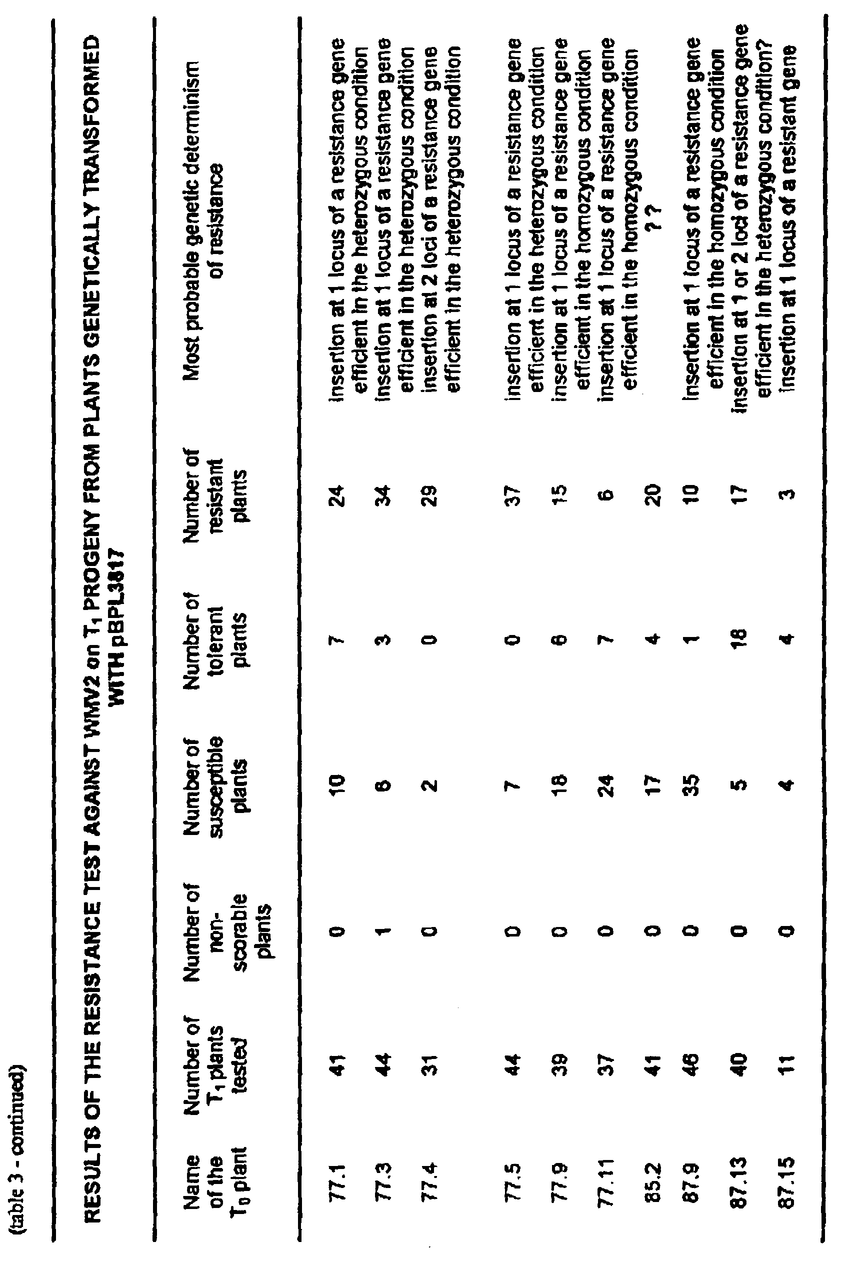

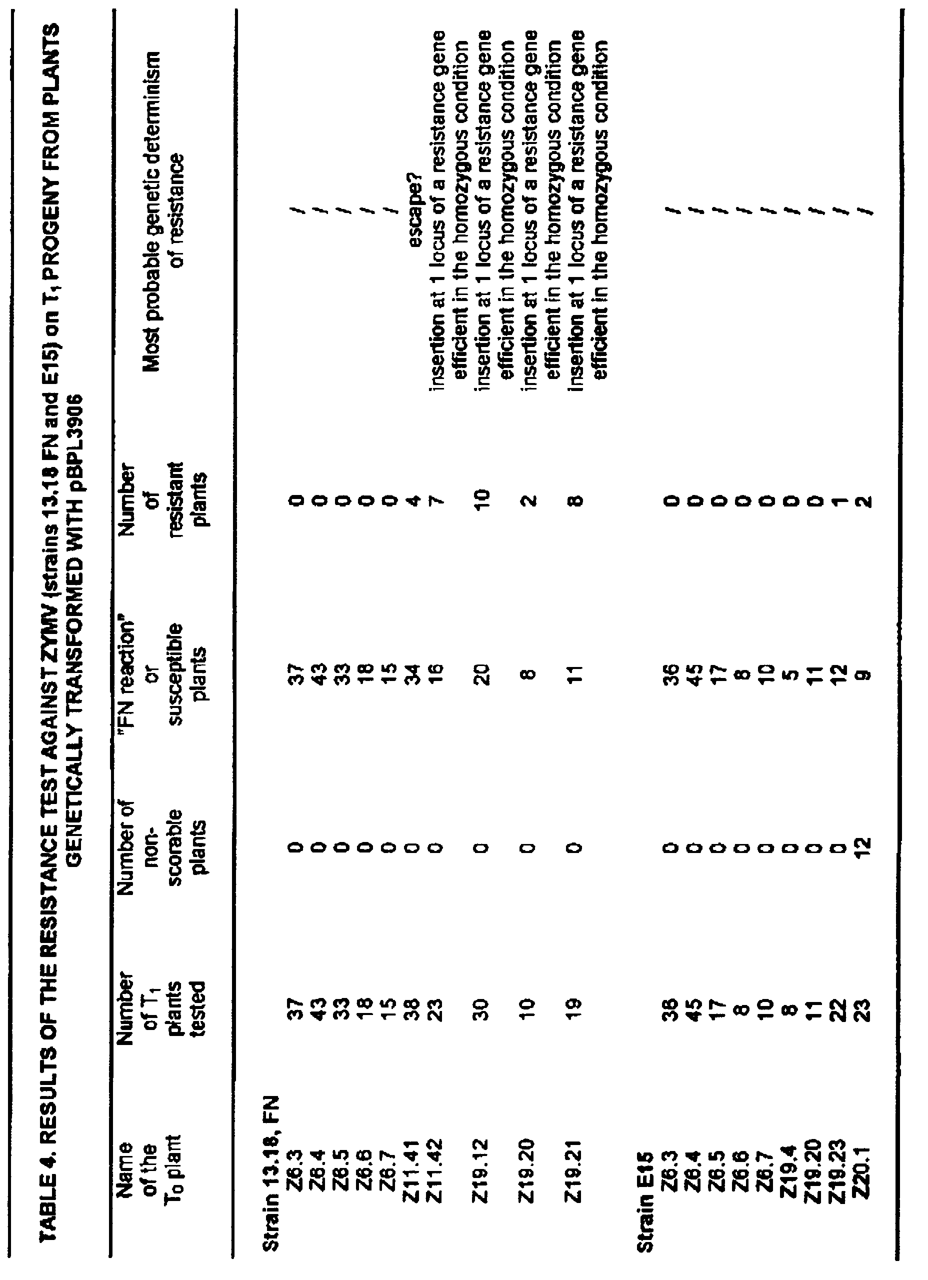

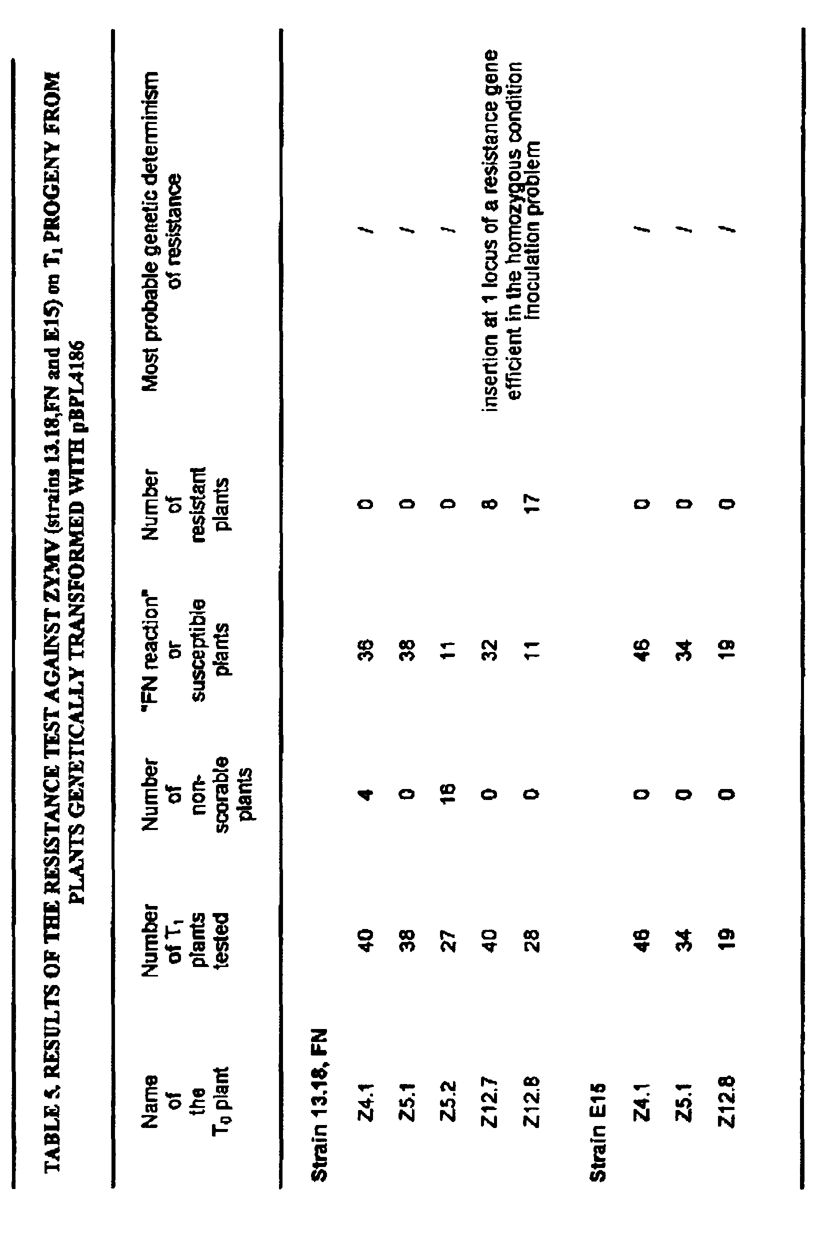

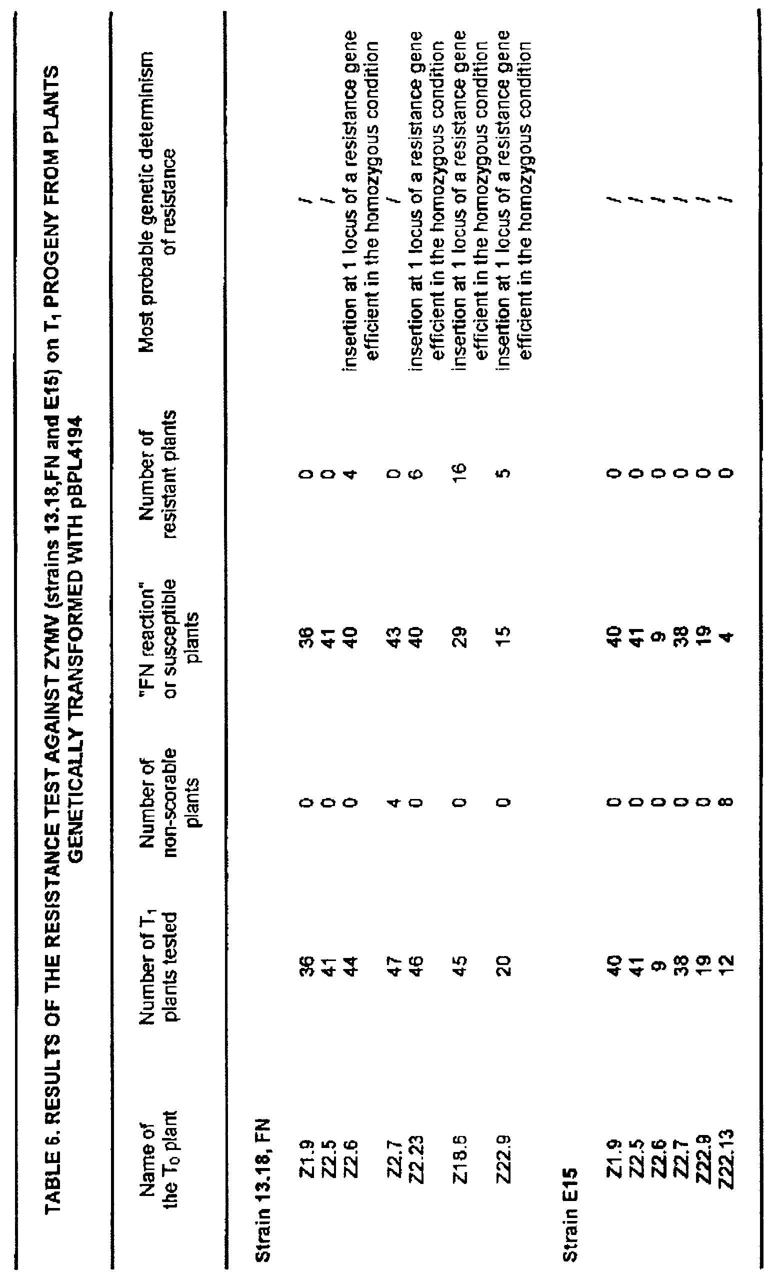

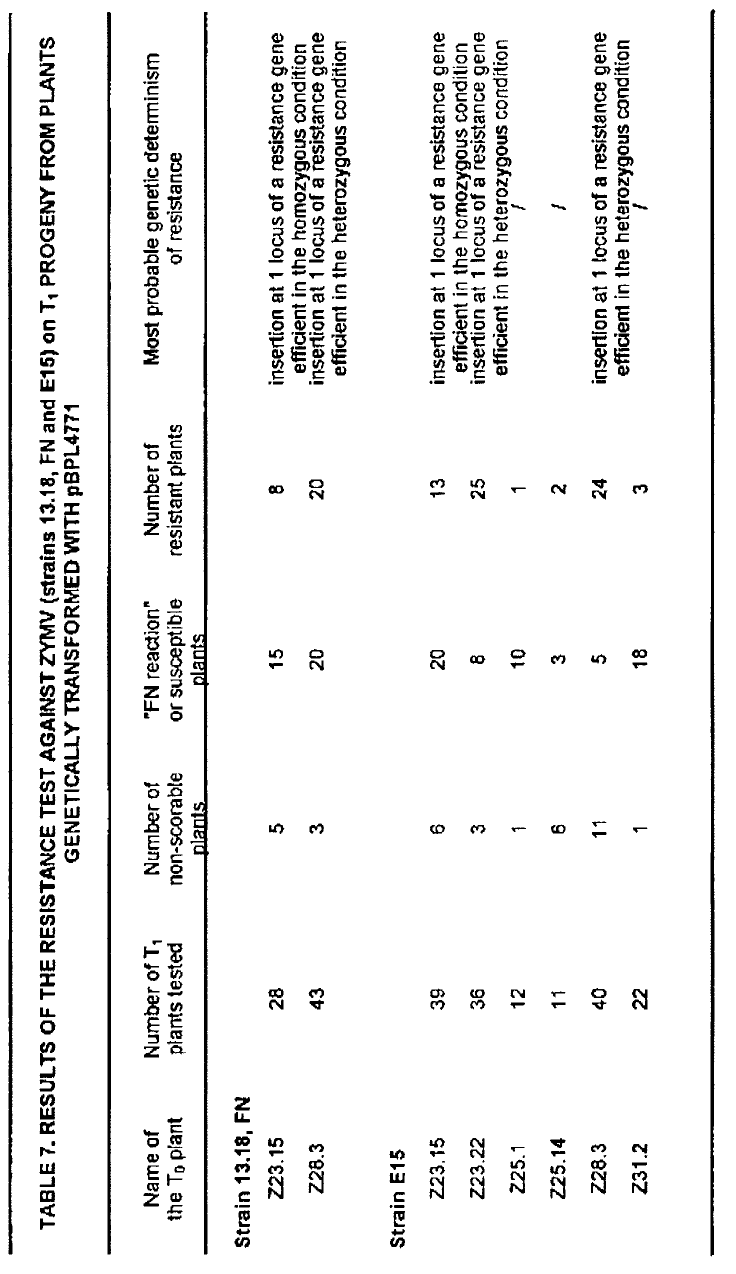

### 10. Transient assay of anti-viral ribozyme genes activity

### 10.1. Description of the system

The transient assay system for in vivo activity of ribozyme or antisense genes was described by Sawyer and Birch (1996). A target reporter gene and the ribozyme gene to be tested are cloned on two separate plasmids and co-introduced into Nicotiana suspension culture cells by particle bombardment. The target reporter gene is an in-frame fusion between the target sequence and the LUC sequence. This in frame fusion encodes a chimeric protein with luciferase activity. 48 hours after the plasmids delivery, the cells are placed under a camera which measures light emission; light emission represents the level of expression of the target reporter gene. The ribozyme gene tested expresses transiently a ribozyme which interacts with the target sequence and decreases the quantity of the chimeric target-LUC mRNA which is detected as a reduced luciferase activiiy.

To measure the activity of various ribozyme genes protecting transgenic melon plants against potyvirus infection, we have built various target reporter genes with viral sequences fused to LUC.

### 10.2.Genes used (see figure 15)

**Target sites on the positive strand** of the virus WMV2 (pBPL0188):

A 540 bases fragment containing four sites targeted by the polyribozyme (2259, 2403, 2520, 2720) was amplified by PCR and cloned in-frame in front of LUC, under control of a modified version of the 35S promoter, using the plasmid pGLFuz (bee figure 17) as described by Sawyer and Birch (1996). Because the most 3' site is located close to the stop codon, it was also necessary to change one base of the viral sequence to remove this stop codon and provide a flanking sequence to this site.

### Target sites on the negative strand of the virus WMV2 (pBPL0184 to 0187):

Because the negative strand is not coding, it was impossible to clone a large sequence containing several ribozyme sites in frame with LUC. Four shorter sequences (between 180 and 220 bases in length) each containing one ribozyme site (696, 996, 1747, 2067) were amplified by PCR and cloned in-frame in front of LUC, under control of the modified version of the 35S promoter in pGLFuz as above.

### Ribozyme gene against WMV2 (pBPL0189):

The short arm polyribozyme targeting four sites on the plus (2259, 2403, 2520 and 2720) and four sites on the minus strand (696, 996, 1747 and 2067) of WMV2 from pBPL4743 was cloned under control of the modified version of the 35S promoter in pGLFuz.

### Ribozyme gene against ZYMV (pBPL0190):

One site on the minus strand (hereafter referred to as 1747) of the virus is present on both WMV2 and ZYMV. The pentameric ribozyme against the minus strand of ZYMV in pBPL4747 is targeting site 1747 amongst others. In this sequence, the ribozyme against site 1747 is identical to the ribozyme present in the construct against WMV2, but the arms are shorter; 8 bases each side. The pentameric ribozyme was cloned under control of the modified version of the 35S promoter in pGLFuz.

### 10.3. Assay and results (see figure 16)

The assays were carried out as described by Sawyer and Birch (1996). For each assay, target and ribozyme plasmid DHA (2µg each, approx. 25% molar excess of ribozyme) were co-precipitated onto tungsten particles and shot onto Nicotiana plumagnifolia cells on filter paper discs (harvested from suspension culture). After bombardment, the cells were cultured on the discs for 48 hours at 28° C in the dark. For luciferase assay, the cells wore placed into a petri dish containing D-luciferin for 30 minutes, then imaged using a liquid nitrogen cooled CCD camera and AT1 software (Wright instruments Ltd, Enfield, UK). Results are shown in figure 16.

The WMV2 polyribozyme (pBPL0189) could inhibit the target containing the four sites on the plus strand (pBPL0188) to an extent (70%) not seen in previous uses of the system with full length antisense when an equal amount of target and inhibitor gene was used (Sawyer and Birch, 1996).

Both ribozymes (pBPL0189 and pBPL0190) could inhibit the target containing site. 1747 (pBPL0186) to an even greater extent (75%). Site 696 (pBPL0184) seemed not amenable to inhibition; this absence of effect on one target shows that the effect observed is not simply caused by an interaction between the 7, plasmids or their promoter. Sites 996 (pBPL0185) and 2067 (pBPL0187) were slightly inhibited.

### 10.4. Comparison with in vitro data obtained previously

Minus strand target (see figure 12):

Site 696 was the site least cleaved in vitro by the WMV2 ribozyme, and was not cleaved at all at 22°C. This is in agreement with the transient assay results.

Sites 1747 and 996 seemed equally cleaved in vitro and 2067 was the site most amenable to cleavage in vitro, particularly at 22°C. This is not fully consistent with the transient assay results suggesting that 1747 is clearly more amenable to cleavage than the 2 others. The comparison of the in vitro and transient in vivo results suggests that the in vitro activity is not a perfect predictor of the transient activity in vivo (unable to rank sites 996, 1747, 2067), but it has some predictive capacity (it could predict that 696 would not be a good target).

Plus strand target (see figure 13);

When the ribozyme was tried in vitro on a target of about 900 bases, site 2259 was somewhat cleaved at 22°C and 37°C (with 4.5 - 13 fold excess ribozyme) and to a lesser extent site 2403; cleavage was far from complete. At 50°C all four sites were cleaved, 2259 then 2403 being apparently the fastest. The transient in vivo assay shows that this ribozyme is active despite the low level of activity observed in vitro at low temperature. ZYMV Ribozyme:

When the ZYMV ribozyme was assayed in vitro on a ZYMV target, site 1747 was not cleaved at 22°C. This result cannot be compared directly with the in vitro assay on WMV2 since the target sequence is significantly different, except immediately around the site itself; it suggests however that shorter arms reduce the activity in vitro. Results of the transient assay suggest that at least for this particular site 1747, the arm length can be reduced with no significant effect on activity in vivo. The predictive capacity of the in vitro assay for this system is therefore not good.

### 10.5. Construction of pGLFuz (see Figure 17):

To create a uidA::luc translational fusion vector (pGLFuz) the uidA gene was first modified by PCR amplification to introduce novel XhoI and NsiI sites upstream of the start codon, replace the stop codon with an alanine codon (TGA -> GCG) and introduce a novel NotI site downstream of this codon. The modified uidA gene PCR product was ligated into pGcmT (Promega). The appropriate recombinant plasmid was designated PGemGUS.

The modified uidA gene from pGemGUS was ligated into pZorz vector as a NsiI NcoI fragment. In the resulting plasmid clone, pGLFuzTAG, the uidA coding region is flanked by an osa promoter and ocs polyadenylation region.

Due to the presence of a stop codon between the uidA and luc genes, preventing a translational fusion, the luc gene in pGLFuzTAG was removed (by digestion with NotI, treatment with T4 DNA polymerase to create blunt ends, then digestion with XhoI) and replaced by the luc gene from pSPNF+luc (Promega) (prepared by digestion with KpnI, treatment with T4 DNA polymerase and digestion with XhoI). The appropriate recombinant plasmid was designated pGLFuz.

For construction of target genes, the uidA coding sequence is replaced by viral sequences. For construction of ribozyme genes, both the uidA and luc coding sequences are replaced by the ribozyme sequences. (see figure 15)

### REFERENCES

Betzner et al., 1997, The Plant Journal, vol. 11, n° 3, p. 101-109.
Bos L (1969). Experience with a collection of plant viruses in leaf material dried and stored over calcium chloride and a discussion of litterature on virus preservation. Medec. Fac. Landbouwwet. Rijksuniv. Gent. 34:875-887
Firek et al., 1993, A wound-induced promoter driving nptII expression limited to dedifferentiated cells at wound sites is sufficient to allow selection of transgenic shoots. Plant Mol. Biol., vol. 22, p 129-142.
Fromm M.E., Taylor L.P. and Walbot V., 1986. Nature, vol. 319, p. 791.
Guerineau F. and Mullineaux P., 1993, Chapter 4 "Plant transformation and expression vectors" in Plant Molecular Biology LABFAX, ed. Croy R.R.D., BIOS scientific Publishers, Blackwell Scientific Publications.
Haseloff and Gerlach, 1988, Simple RNA enzymes with new and highly specific endoribonuclease activities. Nature, vol. 334, p. 585-591.
Lecocq H and Pitrat M (1984). Strains of ZYMV in Muskmelon (*C. melo* L.). Phytopath. Z., 11:165-173
Marrov J (1967). Amélioration des méthodes de transmission mécanique des virus par absorption des inhibiteurs d'infection sur le charbon végétal. C. R. Acad. Agric. Fr. 53:972-981
Pascual Perez, Gérard Tiraby, Jean Kallerhoff and Joel Perret, 1989, Phleomycin resistance as a dominant selectable marker for plant cell transformation. Plant Molecular Biology, vol. 13, p 365-373.
Perriman et al, 1992, Extended target-site specificity for a hammerhead ribozyme. Gene, vol. 113, p. 157-163.
Sawyer B. and Birch R., 1996, A generic transient assay for antisense and ribozyme inhibition in vivo, vol 28, 1996, Proceedings of the Australian society for Biochemistry and Molecular Biology.

## Claims

1. Nucleic acid molecule, called 〈〈 ribozyme 〉〉, having endoribonuclease activity, comprising at least one hybridising region and at least one catalytic region, said hybridising region being complementary to one or more target regions of the genomic (+) RNA of a potyvirus, and/or to one or more target regions of the replicative (-) strand RNA of a potyvirus, with the proviso that the said target region does not consist of the GUC triplet located at position 8122-8124 of the PVYn strain or the equivalent triplet of other potyviruses.

2. Nucleic acid molecule according to claim 1, comprising at least one unit 〈〈 R 〉〉 having the general formula :
3' [(X)ₙ - Q - (X')_{n'}]ₚ 5' wherein :
- X and X' each represent, independently, any ribonucleotide ; (X)ₙ and (X')_{n'} represent ribonucleotide sequences substantially complementary to at least part of said target region of potyvirus genomic (+) RNA or to at least part of its (-) strand replicative RNA ;
- n and n' are each equal to or greater than 4 ;
- Q represents the catalytic region of a ribozyme ;
- p ≥ 1.

3. Nucleic acid molecule according to claim 2 represented by the following formula : 
3′                                                    5′
(X)n Y                                    Y (X)n′
     A                                    C
     A                                    U
     G          1A                        G
     C  *       G               G         A
     |          |                    X
     Y′         Y′′
 wherein, each X represents a ribonucleotide which may be the same or different, (X)ₙ and (X)ₙ' represent ribonucleotide sequences each substantially complementary to one of two RNA sequences in a potyvirus genome or replicative (-) strand RNA, the said two potyvirus sequences being separated from each other by one nucleotide in the genome, or in the replicative (-) strand RNA; an (*) represents a base pair between complementary ribonucleotides ; Y represents any ribonucleotide and each Y may be the same or different ; Y' and Y'' represent ribonucleotides of complementary sequence along at least part of their length to allow base pairing between the oligoribonucleotides, or Y' and Y'' together form a single RNA sequence wherein at least part of said sequence comprises a stem formed by base pairing between complementary nucleotides ; and optionally, an additional nucleotide selected from any one of A, G, C or U is inserted after ¹A.

4. Nucleic acid molecule according to claim 2 or 3 comprising at least two of said R units, 〈〈 R₁ 〉〉 and 〈〈 R₂ 〉〉, wherein the complementary sequences (X)ₙ and (X')_{n'} of R₁ are complementary to sections of a first target region, Z₁, of the potyvirus genomic (+) RNA or of the replicative (-) strand RNA, region Z₁ being within or comprising a first gene or untranslated region (UTR), and X and X' of R₂ are complementary to sections of a second target region, Z₂, of the potyvirus genomic RNA or (-) strand replicative intermediate, region Z₂ being within or comprising a second gene or untranslated region, Z₁ and Z₂ being either different genes or untranslated regions, on the same RNA strand, or being the same or different genes or untranslated regions on different strands of the potyvirus RNA.

5. Nucleic acid molecule according to claim 4 comprising at least two of said R units, 〈〈 R₁ 〉〉 and 〈〈 R₂ 〉〉, wherein the complementary sequences (X)ₙ and (X')_{n'} of R₁ are complementary to a region within or comprising a first gene on the genomic (+) RNA, and X and X' of R₂ are complementary to a region within, or comprising, a second gene on the genomic (+) RNA.

6. Nucleic acid molecule according to claim 4 comprising at least two of said R units, 〈〈 R₁ 〉〉 and 〈〈 R₂ 〉〉, wherein the complementary sequences (X)ₙ and (X')_{n'} of R₁ are complementary to a region within or comprising a region corresponding to a first gene on the replicative (-) strand RNA, and X and X' of R₂ are complementary to a region within or comprising a region corresponding to a second gene one the replicative (-) strand RNA.

7. Nucleic acid molecule according to claim 4 comprising at least two of said R units, 〈〈 R₁ 〉〉 and 〈〈 R₂ 〉〉, wherein the complementary sequences (X)ₙ and (X')_{n'} of R₁ are complementary to a region Z₁ within or comprising a gene on the genomic (+) RNA, and X and X' of R₂ are complementary to a region Z₂ within or comprising a region corresponding to a gene on the replicative (-) strand RNA.

8. Nucleic acid molecule according to claim 7 wherein the complementary sequences of R₁ are complementary to a region Z₁ within or comprising a cistron 〈〈 C 〉〉 on the (+) strand RNA and the complementary sequences of R₂ are complementary to a region Z₂ on the (-) strand within or comprising the region corresponding to the same cistron 〈〈 C 〉〉.

9. Nucleic acid molecule according to claim 7 comprising further R units whose complementary sequences (X)ₙ and (X')ₙ, are complementary to regions within, or comprising, further genes on the genomic (+) strand or on the replicative (-) strand, and/or to regions within the same genes as Z₁ and/or Z₂.

10. Nucleic acid molecule according to claim 2 comprising at least two R units, 〈〈 R₁ 〉〉 and 〈〈 R₂ 〉〉, wherein the complementary sequences (X)ₙ and (X')_{n'} of R₁ are complementary to sections of a target region Z₁ within a given gene or untranslated region of the potyvirus genomic (+) strand RNA or of the corresponding region of the replicative (-) strand RNA, and the hybridising sequences X and X' of R₂ are complementary to a target region Z₂ within the same gene or untranslated region as that of Z₁, on the same RNA strand, provided that if the gene is the capsid protein gene, the hybridising sequences X and X' of both R₁ and R₂ are complementary to the replicative (-) strand RNA.

11. Nucleic acid molecule according to any one of the preceding claims wherein the complementary sequences (X)ₙ and (X')_{n'} of at least one R unit are complementary to regions within or comprising the capsid protein gene, the NIa gene, the NIb gene, the 3' untranslated region of a potyvirus, or the corresponding sequences in the replicative (-) strand RNA.

12. Nucleic acid molecule according to claims 5 and 11 comprising at least two R units, R₁ and R₂, wherein the complementary sequences (X)ₙ and (X')_{n'} of R₁ and of R₂ are respectively complementary to regions within the CP gene and the 3' non-translated region of the genomic (+) strand RNA.

13. Nucleic acid molecule according to claims 6 and 11 comprising at least two R units, R₁ and R₂, wherein the complementary sequences (X)ₙ and (X')_{n'} of R₁ and R₂ are respectively complementary to regions within the CP gene and the NIb gene, or to the NIb gene and the NIa gene, of the replicative (-) strand RNA.

14. Nucleic acid molecule according to claims 8 and 11 comprising at least two R units, R₁ and R₂, wherein the complementary sequences (X)ₙ and (X')_{n'} of R₁ are complementary to the CP gene of the genomic (+) strand RNA, and the hybridising regions of R₂ are complementary to the CP gene on the replicative (-) strand RNA.

15. Nucleic acid molecule according to claims 9 and 11 comprising at least three R units, R₁, R₂ and R₃, wherein the complementary sequences (X)ₙ and (X')_{n'} of R₁ are complementary to region within the CP gene or the 3' non-translated region (3'NT) of the genomic (+) strand RNA, the complementary sequences (X)ₙ and (X')_{n'} of R₂ are complementary to regions within the NIb gene on the replicative (-) strand RNA and the complementary sequences (X)ₙ and (X')_{n'} of R₃ are complementary to regions within the NIa or CP genes on the replicative (-) strand RNA.

16. Nucleic acid molecule according to claims 10 and 11 wherein the complementary sequences (X)ₙ and (X')_{n'} of R₁ and R₂ are complementary to regions within the CP gene on the replicative (-) strand RNA.

17. Nucleic acid molecule according to any one of claims 2 to 16 wherein 〈〈 p 〉〉 has a value from 2 to 20, for example 4 to 10.

18. Nucleic acid molecule according to claim 2 wherein 〈〈 p 〉〉 has a value of 1.

19. Nucleic acid molecule according to any one of claims 2 to 18 wherein each of the complementary sequences (X)ₙ and (X')_{n'} of at least one R unit comprise, independently, qreater than 50 nucleotides, for example 60 to 300 nucleotides.

20. Nucleic acid molecule according to any one of claims 2 to 18 wherein each of the complementary sequences (X)ₙ and (X')_{n'} of at least one R unit comprise, independently, from 4 to 50 nucleotides, for example from 8 to 12 nucleotides.

21. Nucleic acid molecule according to claims 19 and 20 wherein at least a first R unit has complementary sequences (X)ₙ and (X')_{n'} each comprising more than 50 nucleotides, and at least a second R unit has complementary sequences (X)ₙ and (X')_{n'} each comprising from 4 to 50 nucleotides.

22. Nucleic acid molecule according to any one of claims 2 to 21 wherein the 3' (X)ₙ sequence of a given R unit and the 5' (X')_{n'} sequence of the adjacent R unit are complementary to sequences which are contiguous in the potyvirus genomic (+) strand RNA or replicative (-) strand RNA.

23. Nucleic acid molecule according to claim 3 wherein the 3' (X)ₙ sequence of a given R unit and the 5' (X)_{n'} sequence of the adjacent R unit are complementary to non-contiguous portions of the potyvirus genomic (+) strand RNA or replicative (-) strand RNA.

24. Nucleic acid molecule according to any one of claims 2 to 23 wherein at least one nucleotide, or a sequence of nucleotides 〈〈 A 〉〉, non-complementary to the viral genome or the replicative (-) strand RNA, is included between 2 consecutive R units.

25. Nucleic acid molecule according to claim 24 wherein the non-complementary sequence 〈〈 A 〉〉 is a polylinker or a spacer.

26. Nucleic acid molecule according to any one of claims 2 to 25 wherein the complementary sequences (X)ₙ and (X')_{n'} of a first R unit and the complementary sequences (X)ₙ and (X')_{n'} of a second R unit are complementary to target sequences in the genome or in the (-) sense RNA of at least 2 distinct species of potyvirus.

27. Nucleic acid molecule according to claim 1 wherein the catalytic region 〈〈 Y 〉〉 comprises, or is derived from a hairpin ribozyme catalytic region.

28. Nucleic acid molecule according to any one of claims 1 to 26 wherein the hybridising regions are complementary to target regions of one or more potyviruses chosen from the aphid-, mite- and whitefly transmitted potyviruses including
Zucchini yellow mosaic virus (ZYMV),
Watermelon mosaic virus 2 (WMV2),
Tobacco vein mottling virus (TVMV),
Tobacco etch virus (TEV),
Potato virus Y (PVY),
Pepper mottle virus (PepMoV),
Soyabean mosaic virus (SbMV),
Papaya ringspot virus (PRSV),
Pea seed-borne mosaic virus (PSbMV),
Turnip mosaic virus (TuMV),
Johnson grass mosaic virus (JGMV),
Plum pox virus (PPV)
Maize Dwarf Virus (MDV)
Bean common mosaic (BCMV)
Bean yellow mosaic (BYMV)
Carnation vein mottle (CarVMV)
Celery mosaic (CeMV)
Clover yellow vein (ClYVV)
Henbane mosaic (HMV)
Lettuce mosaic (LtMV)
Sugarcane mosaic (SCMV)

29. Nucleic acid molecule according to claim 28 wherein the complementary sequences (X)ₙ and (X')_{n'} are complementary to target regions of ZYMV and/or WMV2, or to mutants or derivatives thereof.

30. Nucleic acid molecule according to any one of claims 1 to 29 further comprising at its 3' end, all or part of the 3' untranslated region of a potyvirus, particularly ZYMV or WMV2.

31. Nucleic acid molecule according to any one of claims 1 to 30, capable of cleaving potyvirus genomic (+) strand RNA or replicative (-) strand RNA thereby reducing viral replication, infection, assembly, cell to cell movement and/or symptoms.

32. Nucleic acid molecule consisting of nucleotides 1704 to 1873 of the (-) strand of the sequence illustrated in Figure 18, or of at least 20 consecutive nucleotides of the said molecule, including the ATG shown at position 1748-1750.

33. Nucleic acid molecule consisting of a sequence having at least 75% identity and preferably at least 85% identity with the molecule according to claim 32, and including the ATG shown at position 1748-1750 of Figure 18.

34. Nucleic acid molecule comprising any of the molecules according to claim 32 or 33, but which is not an entire viral (-) sense replicative RNA.

35. Nucleic acid molecule, comprising a sequence complementary to the molecules of claims 32 or 33.

36. Nucleic acid molecule comprising the molecule of claim 32 or 33 linked as a translational or transcriptional fusion to a second nucleic acid molecule.

37. Nucleic acid molecule according to claim 36 wherein the second nucleic acid molecule comprises a coding sequence, encoding for example a reporter protein, or any other gene of interest.

38. Nucleic acid molecule according to any one of claims 1 to 3 having endoribonuclease activity, wherein said molecule is capable of hybridising to, and cleaving, a molecule according to claims 32-34 or 36-37.

39. Chimeric gene comprising a DNA sequence which is operably linked to 5' and 3' transcription regulation signals functional in plant cells and which, upon transcription, gives rise to one or more nucleic acid molecule(s) according to any one of claims 1 to 38.

40. Vector comprising a nucleic acid molecule according to any one of claims 1 to 38, or a chimeric gone according to claim 39, and optionally a selectable marker gene, reporter gene, and/or functional sequences capable of transferring DNA to a plant cell genome.

41. Plant cells comprising a nucleic acid molecule according to any one of claims 1 to 38, or a chimeric gene according to claim 39, or a DNA sequence which, upon transcription, gives rise to a nucleic acid molecule according to claims 1 to 38.

42. Transgenic plant comprising a nucleic acid according to any one of claims 1 to 31 or a chimeric gene according to claim 39, and capable of reducing replication, infection, cell-to-cell movement and/or assembly of a potyvirus.

43. Transgenic plant comprising a nucleic acid according to any one of claims 32 to 38.

44. Transgenic plant according to claim 42, wherein the plant is chosen from the following generae : Cucurbitaceae, Chenopodiaceae, Cruciferaceae, Graminaceae, Leguminoseae, Ombelliferaceae, Rosaceae, Solanaceae

45. Transgenic plant according to claim 44,, said plant being courgette, melon, beet, oat, wheat, sugar cane, barley, ryegrass, bean, pea, soya, peanut, carrot, celery, parsnip, plum, potato, tomato, tobacco, pepper.

46. Seeds of transgenic plants according to any one of claims 42 to 45.

47. Progeny of transgenic plants according to any one of claims 42 to 45.

48. Fruit of transgenic plants according to any one of claims 42 to 45.

49. Process for conferring resistance to a potyvirus on a plant, comprising introducing into the plant, or into a part of the plant, a nucleic acid comprising, or encoding, a nucleic acid according to any one of claims 1 to 31, optionally followed by regeneration of a transgenic plant exhibiting the required resistance.

50. Method for controlling the functional expression of a nucleic acid molecule by
i) translationally or transcriptionally fusing an artificial target sequence comprising a nucleic acid according to any one of claims 32 to 34, to the nucleic acid whose expression is to be controlled, giving rise to a hybrid gene whose expression and function are not disrupted by the fusion,
ii) allowing the transcription product of the hybrid gene to come into contact with an RNA molecule complementary to at least a part of the artificial target sequence, and capable of hybridising to, and/or cleaving the artificial target.

51. Method according to claim 50 wherein the complementary RNA molecule is a ribozyme or antisense molecule.


SEQUENCE LISTING

(1) GENERAL INFORMATION:

(i) APPLICANT:
(A) NAME: GENE SHEARS PTY LTD
(B) STREET: Suite 1, Building 5 - 105 Dehli Road
(C) CITY: NORTH RYDE
(E) COUNTRY: AUSTRALIA
(F) POSTAL CODE (ZIP): NSW 2113

(ii) TITLE OF INVENTION:
RIBOZYMES CAPABLE OF CONFERRING RESISTANCE TO POTYVIRUS INFECTION, AND
PLANTS EXPRESSING SAID RIBOZYMES.

(iii) NUMBER OF SEQUENCES: 109

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 97402925.8

(2) INFORMATION FOR SEQ ID NO: 1:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC RNA"

(ix) FEATURE:
(A) NAME/KEY: misc_feature
(B) LOCATION:1..21
(D) OTHER INFORMATION:/note= "Mutant ribozyme I, page 17"

(xi) SEQUENCE DESCRIPTION: SEQ ID NO: 1:

CUGAUGAGUC CUGAGGACGA A 21

(2) INFORMATION FOR SEQ ID NO: 2:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic RNA"

(ix) FEATURE:
(A) NAME/KEY: misc_feature
(B) LOCATION:1..23
(D) OTHER INFORMATION:/note= "Mutant ribozyme II, page 17

(xi) SEQUENCE DESCRIPTION: SEQ ID NO: 2:

CUGAUGAGUC CCGUGAGGAC GAA 23

(2) INFORMATION FOR SEQ ID NO: 3:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC RNA"

(ix) FEATURE:
(A) NAME/KEY: misc_feature
(B) LOCATION:1..21
(D) OTHER INFORMATION:/note= "Mutant ribozyme III, page 17"

(xi) SEQUENCE DESCRIPTION: SEQ ID NO: 3:

CUGAUGAGUC GUGAGGACGA A 21

(2) INFORMATION FOR SEQ ID NO: 4:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC RNA"

(ix) FEATURE:
(A) NAME/KEY: misc_feature
(B) LOCATION:1..20
(D) OTHER INFORMATION:/note= "Mutant ribozyme IV, page 17"

(xi) SEQUENCE DESCRIPTION: SEQ ID NO: 4:

CUGAUGAGCG GUGACGCGAA 20

(2) INFORMATION FOR SEQ ID NO: 5:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic RNA"

(ix) FEATURE:
(A) NAME/KEY: misc_feature
(B) LOCATION:1..22
(D) OTHER INFORMATION:/note= "Wild type sTobRV, page 18"

(xi) SEQUENCE DESCRIPTION: SEQ ID NO: 5:

CUGAUGAGUC CGUGAGGACG AA 22

(2) INFORMATION FOR SEQ ID NO: 6:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..21
(D) OTHER INFORMATION:/note= "page 43, poly-T-anchor"

(xi) SEQUENCE DESCRIPTION: SEQ ID NO: 6:

GGGAGCTCGC TAGCGGATCC T 21

(2) INFORMATION FOR SEQ ID NO: 7:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..22
(D) OTHER INFORMATION:/note= "PRIMER 1, page 43"

(xi) SEQUENCE DESCRIPTION: SEQ ID NO: 7:

AATTGGGAGC TCGCTAGCGG AT 22

(2) INFORMATION FOR SEQ ID NO: 8:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(ix) FEATURE:
(A) NAME/KEY: misc_feature
(B) LOCATION:1..33
(D) OTHER INFORMATION:/note= "PRIMER 2, page 43"

(xi) SEQUENCE DESCRIPTION: SEQ ID NO: 8:

AATTGCTAGC AGTKGTKGAC AATACACTWA TGG 33

(2) INFORMATION FOR SEQ ID NO: 9:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..29
(D) OTHER INFORMATION:/note= "primer 3, page 43"

(xi) SEQUENCE DESCRIPTION: SEQ ID NO: 9:

AATTCTAGAA THAKATCATC ACCATTKGC 29

(2) INFORMATION FOR SEQ ID NO: 10:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(ix) FEATURE:
(A) NAME/KEY: misc_feature
(B) LOCATION:1..32
(D) OTHER INFORMATION:/note= "Primer 4, page 43"

(xi) SEQUENCE DESCRIPTION: SEQ ID NO: 10:

AATTCTAGAT TCTGGAWACA TTGGATAWSN AC 32

(2) INFORMATION FOR SEQ ID NO: 11:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..30
(D) OTHER INFORMATION:/note= "RBZ696-3' page 48"

(xi) SEQUENCE DESCRIPTION: SEQ ID NO: 11:

GTGAGGACGA AAAAGCTGCT GTCGGTGCTC 30

(2) INFORMATION FOR SEQ ID NO: 12:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..32
(D) OTHER INFORMATION:/note= "RBZ696-5' page 49"

(xi) SEQUENCE DESCRIPTION: SEQ ID NO: 12:

GGACTCATCA GATGTTCAGG GATCCATAAA TC 32

(2) INFORMATION FOR SEQ ID NO: 13:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..32
(D) OTHER INFORMATION:/note= "RBZ996-3' page 49"

(xi) SEQUENCE DESCRIPTION: SEQ ID NO: 13:

GTGAGGACGA AACTAAGTTT TATGGGGGTT GG 32

(2) INFORMATION FOR SEQ ID NO: 14:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..30
(D) OTHER INFORMATION:/note= "RBZ996-5' page 49"

(xi) SEQUENCE DESCRIPTION: SEQ ID NO: 14:

GGACTCATCA GATGCCAACT GTCCATGGAC 30

(2) INFORMATION FOR SEQ ID NO: 15:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(ix) FEATURE:
(A) NAME/KEY: misc_feature
(B) LOCATION:1..31
(D) OTHER INFORMATION:/note= "RBZ1747-3', page 49"

(xi) SEQUENCE DESCRIPTION: SEQ ID NO: 15:

GTGAGGACGA AACATAGCTG AGACAGCATT G 31

(2) INFORMATION FOR SEQ ID NO: 16:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(ix) FEATURE:
(A) NAME/KEY: misc_feature
(B) LOCATION:1..29
(D) OTHER INFORMATION:/note= "RBZ1747-5' page 49"

(xi) SEQUENCE DESCRIPTION: SEQ ID NO: 16:

GGACTCATCA GTGGAGCTTT TCCGGATGC 29

(2) INFORMATION FOR SEQ ID NO: 17:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..33
(D) OTHER INFORMATION:/note= "RBZ2067-3', page 49"

(xi) SEQUENCE DESCRIPTION: SEQ ID NO: 17:

GTGAGGACGA AAAGAAAATG AACCTTCCAA CAG 33

(2) INFORMATION FOR SEQ ID NO: 18:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..32
(D) OTHER INFORMATION:/note= "RBZ2067-5' page 49"

(xi) SEQUENCE DESCRIPTION: SEQ ID NO: 18:

GGACTCATCA GGTTATCTTT TGTAGTCGTG GG 32

(2) INFORMATION FOR SEQ ID NO: 19:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic RNA"

(ix) FEATURE:
(A) NAME/KEY: misc_feature
(B) LOCATION:1..23
(D) OTHER INFORMATION:/note= "Mutant ribozyme 696 page 49"

(xi) SEQUENCE DESCRIPTION: SEQ ID NO: 19:

CUGAUGAGUC CCGUGAGGAC GAA 23

(2) INFORMATION FOR SEQ ID NO: 20:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic RNA"

(ix) FEATURE:
(A) NAME/KEY: misc_feature
(B) LOCATION:1..21
(D) OTHER INFORMATION:/note= "Mutant ribozyme 996, page 50"

(xi) SEQUENCE DESCRIPTION: SEQ ID NO: 20:

CUGAUGAGUC CUGAGGACGA A 21

(2) INFORMATION FOR SEQ ID NO: 21:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..32
(D) OTHER INFORMATION:/note= "RBZ2259-3', page 51"

(xi) SEQUENCE DESCRIPTION: SEQ ID NO: 21:

GTGAGGACGA AAAACCATTC ATAATCACAC CC 32

(2) INFORMATION FOR SEQ ID NO: 22:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..32
(D) OTHER INFORMATION:/note= "RBZ 2259-5', page 51"

(xi) SEQUENCE DESCRIPTION: SEQ ID NO: 22:

GGACTCATCA GATGGTTTGG TGTATCGATA AC 32

(2) INFORMATION FOR SEQ ID NO: 23:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..30
(D) OTHER INFORMATION:/note= "RBZ2403-3', page 51"

(xi) SEQUENCE DESCRIPTION: SEQ ID NO: 23:

GTGAGGACGA AAAATGGTGC ATGATTTGTC 30

(2) INFORMATION FOR SEQ ID NO: 24:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..28
(D) OTHER INFORMATION:/note= "RBZ2403-5',page 51"

(xi) SEQUENCE DESCRIPTION: SEQ ID NO: 24:

GGACTCATCA GTCAGACGCA GCAGAAGC 28

(2) INFORMATION FOR SEQ ID NO: 25:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..31
(D) OTHER INFORMATION:/note= "RBZ 2520-3', page 52"

(xi) SEQUENCE DESCRIPTION: SEQ ID NO: 25:

GTGAGGACGA AAAGTCAAAA GCATAGCGTG C 31

(2) INFORMATION FOR SEQ ID NO: 26:

(i) SEQUENCE CHARACTERISTICS:
(A) LENGTH: 33 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..33
(D) OTHER INFORMATION:/note= "RBZ 2520-5', page 52"

(xi) SEQUENCE DESCRIPTION: SEQ ID NO: 26:

GGACTCATCA GTATGAGGTT ACTTCCAAAA CAC 33

(2) INFORMATION FOR SEQ ID NO: 27:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..30
(D) OTHER INFORMATION:/note= "RBZ 2720-3', page 52"

(xi) SEQUENCE DESCRIPTION: SEQ ID NO: 27:

GTGAGGACGA AACTGCGGTG GACCCATACC 30

(2) INFORMATION FOR SEQ ID NO: 28:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..32
(D) OTHER INFORMATION:/note= "RBZ 2720-5', page 52"

(xi) SEQUENCE DESCRIPTION: SEQ ID NO: 28:

GGACTCATCA GAAGACTAGG TAAACTGGTC AC 32

(2) INFORMATION FOR SEQ ID NO: 29:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic RNA"

(ix) FEATURE:
(A) NAME/KEY: misc_feature
(B) LOCATION:1..21
(D) OTHER INFORMATION:/note= "Mutant ribozyme 2520 page 52"

(xi) SEQUENCE DESCRIPTION: SEQ ID NO: 29:

CUGAUGAGUC CUGAGGACGA A 21

(2) INFORMATION FOR SEQ ID NO: 30:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic RNA"

(ix) FEATURE:
(A) NAME/KEY: misc_feature
(B) LOCATION:1..21
(D) OTHER INFORMATION:/note= "Mutant ribozyme 2720 page 52"

(xi) SEQUENCE DESCRIPTION: SEQ ID NO: 30:

CUGAUGAGUC GUGAGGACGA A 21

(2) INFORMATION FOR SEQ ID NO: 31:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic RNA"

(ix) FEATURE:
(A) NAME/KEY: misc_feature
(B) LOCATION:1..20
(D) OTHER INFORMATION:/note= "page 53"

(xi) SEQUENCE DESCRIPTION: SEQ ID NO: 31:

CUGAUGAGCG GUGACGCGAA 20

(2) INFORMATION FOR SEQ ID NO: 32:

(i) SEQUENCE CHARACTERISTICS:
(A) LENGTH: 59 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..59
(D) OTHER INFORMATION:/note= "WM2067S, page 54"

(xi) SEQUENCE DESCRIPTION: SEQ ID NO: 32:

GCAGCTGCTG GATCCATGTC ATTTTCTTTT CGCGTCACCG CTCATCAGGT TATCTTTTG 59

(2) INFORMATION FOR SEQ ID NO: 33:

(i) SEQUENCE CHARACTERISTICS:
(A) LENGTH: 41 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..41
(D) OTHER INFORMATION:/note= "WM1747S, page 54"

(xi) SEQUENCE DESCRIPTION: SEQ ID NO: 33:

TCAGCTATGT TTCGCGTCAC CGCTCATCAG TGGAGCTTTT C 41

(2) INFORMATION FOR SEQ ID NO: 34:

(i) SEQUENCE CHARACTERISTICS:
(A) LENGTH: 49 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..49
(D) OTHER INFORMATION:/note= "WM996S page 54"

(xi) SEQUENCE DESCRIPTION: SEQ ID NO: 34:

CAGATCTATA AAACTTAGTT TCGCGTCACC GCTCATCAGA TGCCAACTG 49

(2) INFORMATION FOR SEQ ID NO: 35:

(i) SEQUENCE CHARACTERISTICS:
(A) LENGTH: 61 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(ix) FEATURE:
(A) NAME/KEY: misc_feature
(B) LOCATION:1..61
(D) OTHER INFORMATION:/note= "WM696S, page 54"

(xi) SEQUENCE DESCRIPTION: SEQ ID NO: 35:

CAGCAGCTTT TTCGCGTCAC CGCTCATCAG ATGTTCAGGG ACCTGCATCT GATCAAGCTT 60

G 61

(2) INFORMATION FOR SEQ ID NO: 36:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..27
(D) OTHER INFORMATION:/note= "WMGU4S, page 54"

(xi) SEQUENCE DESCRIPTION: SEQ ID NO: 36:

GAAACATAGC TGACAAAAGA TAACCTG 27

(2) INFORMATION FOR SEQ ID NO: 37:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..26
(D) OTHER INFORMATION:/note= "WMGU5S, page 55"

(xi) SEQUENCE DESCRIPTION: SEQ ID NO: 37:

GTTTTATAGA TCTGGAAAAG CTCCAC 26

(2) INFORMATION FOR SEQ ID NO: 38:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..24
(D) OTHER INFORMATION:/note= "WMGU6S, page 55"

(xi) SEQUENCE DESCRIPTION: SEQ ID NO: 38:

GAAAAAGCTG CTGCAGTTGG CATC 24

(2) INFORMATION FOR SEQ ID NO: 39:

(i) SEQUENCE CHARACTERISTICS:
(A) LENGTH: 55 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..55
(D) OTHER INFORMATION:/note= "WM2259S page 55"

(xi) SEQUENCE DESCRIPTION: SEQ ID NO: 39:

GCAGCTGCTG GATCCATGAA TGGTTTTTCG CGTCACCGCT CATCAGATGG TTTGG 55

(2) INFORMATION FOR SEQ ID NO: 40:

(i) SEQUENCE CHARACTERISTICS:
(A) LENGTH: 42 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(ix) FEATURE:
(A) NAME/KEY: misc_feature
(B) LOCATION:1..42
(D) OTHER INFORMATION:/note= "WM2403S page 55"

(xi) SEQUENCE DESCRIPTION: SEQ ID NO: 40:

TGCACCATTT TTCGCGTCAC CGCTCATCAG TCAGACGCAG AT 42

(2) INFORMATION FOR SEQ ID NO: 41:

(i) SEQUENCE CHARACTERISTICS:
(A) LENGTH: 41 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..41
(D) OTHER INFORMATION:/note= "WM2520S page 55"

(xi) SEQUENCE DESCRIPTION: SEQ ID NO: 41:

CTTTTGACTT TTCGCGTCAC CGCTCATCAG TATGAGGTTA C 41

(2) INFORMATION FOR SEQ ID NO: 42:

(i) SEQUENCE CHARACTERISTICS:
(A) LENGTH: 61 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..61
(D) OTHER INFORMATION:/note= "WM2720S page 55"

(xi) SEQUENCE DESCRIPTION: SEQ ID NO: 42:

CACCGCAGTT TCGCGTCACC GCTCATCAGA AGACTAGGTA ACCTGCATCT GATCAAGCTT 60

G 61

(2) INFORMATION FOR SEQ ID NO: 43:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(ix) FEATURE:
(A) NAME/KEY: misc_feature
(B) LOCATION:1..25
(D) OTHER INFORMATION:/note= "WMGU1S page 55"

(xi) SEQUENCE DESCRIPTION: SEQ ID NO: 43:

GAAAAATGGT GCACCAAACC ATCTG 25

(2) INFORMATION FOR SEQ ID NO: 44:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..27
(D) OTHER INFORMATION:/note= "WMGU2S page 55"

(xi) SEQUENCE DESCRIPTION: SEQ ID NO: 44:

CGAAAAGTCA AAAGATCTGC GTCTGAC 27

(2) INFORMATION FOR SEQ ID NO: 45:

(i) SEQUENCE CHARACTERISTICS:
(A) LENGTH: 25 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..25
(D) OTHER INFORMATION:/note= "WMGU3S page 56"

(xi) SEQUENCE DESCRIPTION: SEQ ID NO: 45:

AAACTGCGGT GGTAACCTCA TACTG 25

(2) INFORMATION FOR SEQ ID NO: 46:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..22
(D) OTHER INFORMATION:/note= "WMPC1S page 56"

(xi) SEQUENCE DESCRIPTION: SEQ ID NO: 46:

AATTGCAGCT GCTGGATCCA TG 22

(2) INFORMATION FOR SEQ ID NO: 47:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..23
(D) OTHER INFORMATION:/note= "WMPC2S page 56"

(xi) SEQUENCE DESCRIPTION: SEQ ID NO: 47:

AATTCAAGCT TGATCAGATG CAG 23

(2) INFORMATION FOR SEQ ID NO: 48:

(i) SEQUENCE CHARACTERISTICS:
(A) LENGTH: 51 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: CDS
(B) LOCATION:1..51
(D) OTHER INFORMATION:/note= "FIGURE 2A, WMV2 USA"

(xi) SEQUENCE DESCRIPTION: SEQ ID NO: 48:

GGG AAC AAC AGT GGG CAG CCA TCT ACA GTT GTT GAC AAT ACA CTT ATG 48
Gly Asn Asn Ser Gly Gln Pro Ser Thr Val Val Asp Asn Thr Leu Met
1               5                   10                  15

GTT 51
Val

(2) INFORMATION FOR SEQ ID NO: 49:

(i) SEQUENCE CHARACTERISTICS:
(A) LENGTH: 17 amino acids
(B) TYPE: amino acid
(D) TOPOLOGY: linear

(ii) MOLECULE TYPE: protein

(xi) SEQUENCE DESCRIPTION: SEQ ID NO: 49:

Gly Asn Asn Ser Gly Gln Pro Ser Thr Val Val Asp Asn Thr Leu Met
1               5                   10                  15

Val

(2) INFORMATION FOR SEQ ID NO: 50:

(i) SEQUENCE CHARACTERISTICS:
(A) LENGTH: 51 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: CDS
(B) LOCATION:1..51
(D) OTHER INFORMATION:/note= "FIGURE 2A, ZYMV"

(xi) SEQUENCE DESCRIPTION: SEQ ID NO: 50:

GGT AAC AAC AGT GGG CAA CCC TCA ACA GTG GTG GAT AAC ACA CTA ATG 48
Gly Asn Asn Ser Gly Gln Pro Ser Thr Val Val Asp Asn Thr Leu Met
1               5                   10                  15

GTT 51
Val

(2) INFORMATION FOR SEQ ID NO: 51:

(i) SEQUENCE CHARACTERISTICS:
(A) LENGTH: 17 amino acids
(B) TYPE: amino acid
(D) TOPOLOGY: linear

(ii) MOLECULE TYPE: protein
(xi) SEQUENCE DESCRIPTION: SEQ ID NO: 51:

Gly Asn Asn Ser Gly Gln Pro Ser Thr Val Val Asp Asn Thr Leu Met
1               5                   10                  15

Val

(2) INFORMATION FOR SEQ ID NO: 52:

(i) SEQUENCE CHARACTERISTICS:
(A) LENGTH: 51 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: CDS
(B) LOCATION:1..51
(D) OTHER INFORMATION:/note= "FIGURE 2A, PLUM POX VIRUS NAT"

(xi) SEQUENCE DESCRIPTION: SEQ ID NO: 52:

GGA AAC AAT AGT GGT CAA CCC TCG ACA GTT GTT GAC AAT ACA CTC ATG 48
Gly Asn Asn Ser Gly Gln Pro Ser Thr Val Val Asp Asn Thr Leu Met
1               5                   10                  15

GTT 51
Val

(2) INFORMATION FOR SEQ ID NO: 53:

(i) SEQUENCE CHARACTERISTICS:
(A) LENGTH: 17 amino acids
(B) TYPE: amino acid
(D) TOPOLOGY: linear

(ii) MOLECULE TYPE: protein
(xi) SEQUENCE DESCRIPTION: SEQ ID NO: 53:

Gly Asn Asn Ser Gly Gln Pro Ser Thr Val Val Asp Asn Thr Leu Met
1               5                   10                  15

Val

(2) INFORMATION FOR SEQ ID NO: 54:

(i) SEQUENCE CHARACTERISTICS:
(A) LENGTH: 51 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: CDS
(B) LOCATION:1..51
(D) OTHER INFORMATION:/note= "FIGURE 2A, TVMV"

(xi) SEQUENCE DESCRIPTION: SEQ ID NO: 54:

GGC AAC AAC AGT GGA CAG CCA TCT ACG GTT GTG GAC AAC ACA CTC ATG 48
Gly Asn Asn Ser Gly Gln Pro Ser Thr Val Val Asp Asn Thr Leu Met
1               5                   10                  15

GTG 51
Val

(2) INFORMATION FOR SEQ ID NO: 55:

(i) SEQUENCE CHARACTERISTICS:
(A) LENGTH: 17 amino acids
(B) TYPE: amino acid
(D) TOPOLOGY: linear

(ii) MOLECULE TYPE: protein

(xi) SEQUENCE DESCRIPTION: SEQ ID NO: 55:

Gly Asn Asn Ser Gly Gln Pro Ser Thr Val Val Asp Asn Thr Leu Met
1               5                   10                  15

Val

(2) INFORMATION FOR SEQ ID NO: 56:

(i) SEQUENCE CHARACTERISTICS:
(A) LENGTH: 51 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: CDS
(B) LOCATION:1..51
(D) OTHER INFORMATION:/note= "FIGURE 2A, TEV"

(xi) SEQUENCE DESCRIPTION: SEQ ID NO: 56:

GGC AAC AAT AGC GGG CAA CCT TCA ACA GTG GTG GAC AAC ACA CTC ATG 48
Gly Asn Asn Ser Gly Gln Pro Ser Thr Val Val Asp Asn Thr Leu Met
1               5                   10                  15

GTC 51
Val

(2) INFORMATION FOR SEQ ID NO: 57:

(i) SEQUENCE CHARACTERISTICS:
(A) LENGTH: 17 amino acids
(B) TYPE: amino acid
(D) TOPOLOGY: linear

(ii) MOLECULE TYPE: protein
(xi) SEQUENCE DESCRIPTION: SEQ ID NO: 57:

Gly Asn Asn Ser Gly Gln Pro Ser Thr Val Val Asp Asn Thr Leu Met
1               5                   10                  15

Val

(2) INFORMATION FOR SEQ ID NO: 58:

(i) SEQUENCE CHARACTERISTICS:
(A) LENGTH: 51 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: CDS
(B) LOCATION:1..51
(D) OTHER INFORMATION:/note= "FIGURE 2A, PVY"

(xi) SEQUENCE DESCRIPTION: SEQ ID NO: 58:

GGT AAT AAT AGT GGT CAA CCT TCT ACC GTT GTG GAT AAT TCT CTC ATG 48
Gly Asn Asn Ser Gly Gln Pro Ser Thr Val Val Asp Asn Ser Leu Met
1               5                   10                  15

GTT 51
Val

(2) INFORMATION FOR SEQ ID NO: 59:

(i) SEQUENCE CHARACTERISTICS:
(A) LENGTH: 17 amino acids
(B) TYPE: amino acid
(D) TOPOLOGY: linear

(ii) MOLECULE TYPE: protein
(xi) SEQUENCE DESCRIPTION: SEQ ID NO: 59:

Gly Asn Asn Ser Gly Gln Pro Ser Thr Val Val Asp Asn Ser Leu Met
1               5                   10                  15

Val

(2) INFORMATION FOR SEQ ID NO: 60:

(i) SEQUENCE CHARACTERISTICS:
(A) LENGTH: 57 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: CDS
(B) LOCATION:1..57
(D) OTHER INFORMATION:/note= "FIGURE 2B, PRSV"

(xi) SEQUENCE DESCRIPTION: SEQ ID NO: 60:

GGC AAT AAT AGT GGC CAA CCT TCG ACA GTT GTT GAT AAT ACA TTG ATG 48
Gly Asn Asn Ser Gly Gln Pro Ser Thr Val Val Asp Asn Thr Leu Met
1               5                   10                  15

GTT TTA ATC 57
Val Leu Ile

(2) INFORMATION FOR SEQ ID NO: 61:

(i) SEQUENCE CHARACTERISTICS:
(A) LENGTH: 19 amino acids
(B) TYPE: amino acid
(D) TOPOLOGY: linear

(ii) MOLECULE TYPE: protein
(xi) SEQUENCE DESCRIPTION: SEQ ID NO: 61:

Gly Asn Asn Ser Gly Gln Pro Ser Thr Val Val Asp Asn Thr Leu Met
1               5                   10                  15

Val Leu Ile

(2) INFORMATION FOR SEQ ID NO: 62:

(i) SEQUENCE CHARACTERISTICS:
(A) LENGTH: 57 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: CDS
(B) LOCATION:1..57
(D) OTHER INFORMATION:/note= "FIGURE 2B, PSbMV"

(xi) SEQUENCE DESCRIPTION: SEQ ID NO: 62:

GGA AAT AAT AGT GGA CAA CCA TCA ACA GTT GTT GAT AAC ACA TTG ATG 48
Gly Asn Asn Ser Gly Gln Pro Ser Thr Val Val Asp Asn Thr Leu Met
1               5                   10                  15

GTG ATT CTT 57
Val Ile Leu

(2) INFORMATION FOR SEQ ID NO: 63:

(i) SEQUENCE CHARACTERISTICS:
(A) LENGTH: 19 amino acids
(B) TYPE: amino acid
(D) TOPOLOGY: linear

(ii) MOLECULE TYPE: protein
(xi) SEQUENCE DESCRIPTION: SEQ ID NO: 63:

Gly Asn Asn Ser Gly Gln Pro Ser Thr Val Val Asp Asn Thr Leu Met
1               5                   10                  15

Val Ile Leu

(2) INFORMATION FOR SEQ ID NO: 64:

(i) SEQUENCE CHARACTERISTICS:
(A) LENGTH: 57 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: CDS
(B) LOCATION:1..57
(D) OTHER INFORMATION:/note= "FIGURE 2B, TuMV"

(xi) SEQUENCE DESCRIPTION: SEQ ID NO: 64:

GGA AAC AAT AGC GGA CAG CCA TCG ACT GTT GTA GAC AAC ACA CTC ATG 48
Gly Asn Asn Ser Gly Gln Pro Ser Thr Val Val Asp Asn Thr Leu Met
1               5                   10                  15

GTC ATA TTG 57
Val Ile Leu

(2) INFORMATION FOR SEQ ID NO: 65:

(i) SEQUENCE CHARACTERISTICS:
(A) LENGTH: 19 amino acids
(B) TYPE: amino acid
(D) TOPOLOGY: linear

(ii) MOLECULE TYPE: protein
(xi) SEQUENCE DESCRIPTION: SEQ ID NO: 65:

Gly Asn Asn Ser Gly Gln Pro Ser Thr Val Val Asp Asn Thr Leu Met
1               5                   10                  15

Val Ile Leu

(2) INFORMATION FOR SEQ ID NO: 66:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: CDS
(B) LOCATION:1..30
(D) OTHER INFORMATION:/note= "FIGURE 3A, WMV2 USA"

(xi) SEQUENCE DESCRIPTION: SEQ ID NO: 66:

TTC TTT GCC AAT GGT GAT GAT ATT ATC CTG 30
Phe Phe Ala Asn Gly Asp Asp Ile Ile Leu
1               5                   10

(2) INFORMATION FOR SEQ ID NO: 67:

(i) SEQUENCE CHARACTERISTICS:
(A) LENGTH: 10 amino acids
(B) TYPE: amino acid
(D) TOPOLOGY: linear

(ii) MOLECULE TYPE: protein
(xi) SEQUENCE DESCRIPTION: SEQ ID NO: 67:

Phe Phe Ala Asn Gly Asp Asp Ile Ile Leu
1               5                   10

(2) INFORMATION FOR SEQ ID NO: 68:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: CDS
(B) LOCATION:1..30
(D) OTHER INFORMATION:/note= "FIGURE 3A, ZYMV"

(xi) SEQUENCE DESCRIPTION: SEQ ID NO: 68:

TTC TTT GCA AAT GGA GAT GAT CTG ATA CTT 30
Phe Phe Ala Asn Gly Asp Asp Leu Ile Leu
1               5                   10

(2) INFORMATION FOR SEQ ID NO: 69:

(i) SEQUENCE CHARACTERISTICS:
(A) LENGTH: 10 amino acids
(B) TYPE: amino acid
(D) TOPOLOGY: linear

(ii) MOLECULE TYPE: protein
(xi) SEQUENCE DESCRIPTION: SEQ ID NO: 69:

Phe Phe Ala Asn Gly Asp Asp Leu Ile Leu
1               5                   10

(2) INFORMATION FOR SEQ ID NO: 70:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: CDS
(B) LOCATION:1..30
(D) OTHER INFORMATION:/note= "FIGURE 3A, PLUM POX VIRUS NAT"

(xi) SEQUENCE DESCRIPTION: SEQ ID NO: 70:

TAC TTT GTG AAT GGT GAT GAT CTT GTC CTC 30
Tyr Phe Val Asn Gly Asp Asp Leu Val Leu
1               5                   10

(2) INFORMATION FOR SEQ ID NO: 71:

(i) SEQUENCE CHARACTERISTICS:
(A) LENGTH: 10 amino acids
(B) TYPE: amino acid
(D) TOPOLOGY: linear

(ii) MOLECULE TYPE: protein
(xi) SEQUENCE DESCRIPTION: SEQ ID NO: 71:

Tyr Phe Val Asn Gly Asp Asp Leu Val Leu
1               5                   10

(2) INFORMATION FOR SEQ ID NO: 72:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(ix) FEATURE:
(A) NAME/KEY: CDS
(B) LOCATION:1..30
(D) OTHER INFORMATION:/note= "FIGURE 3A, TVMV"

(xi) SEQUENCE DESCRIPTION: SEQ ID NO: 72:

TTC TTT GCC AAT GGA GAT GAT CTT ATA ATT 30
Phe Phe Ala Asn Gly Asp Asp Leu Ile Ile
1               5                   10

(2) INFORMATION FOR SEQ ID NO: 73:

(i) SEQUENCE CHARACTERISTICS:
(A) LENGTH: 10 amino acids
(B) TYPE: amino acid
(D) TOPOLOGY: linear

(ii) MOLECULE TYPE: protein
(xi) SEQUENCE DESCRIPTION: SEQ ID NO: 73:

Phe Phe Ala Asn Gly Asp Asp Leu Ile Ile
1               5                   10

(2) INFORMATION FOR SEQ ID NO: 74:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid
(A) DESCRIPTION:   /desc = "synthetic DNA"

(ix) FEATURE:
(A) NAME/KEY: CDS
(B) LOCATION:1..30
(D) OTHER INFORMATION:/note= "FIGURE 3A, TEV"

(xi) SEQUENCE DESCRIPTION: SEQ ID NO: 74:

TAT TAC GTC AAT GGC GAT GAC CTA TTG ATT                                    30
Tyr Tyr Val Asn Gly Asp Asp Leu Leu Ile
1               5                   10

(2) INFORMATION FOR SEQ ID NO: 75:

(i) SEQUENCE CHARACTERISTICS:
(A) LENGTH: 10 amino acids
(B) TYPE: amino acid
(D) TOPOLOGY: linear

(ii) MOLECULE TYPE: protein
(xi) SEQUENCE DESCRIPTION: SEQ ID NO: 75:

Tyr Tyr Val Asn Gly Asp Asp Leu Leu Ile
1               5                   10

(2) INFORMATION FOR SEQ ID NO: 76:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: CDS
(B) LOCATION:1..30
(D) OTHER INFORMATION:/note= "FIGURE 3B, PVY"

(xi) SEQUENCE DESCRIPTION: SEQ ID NO: 76:

TTC TTT GTT AAT GGT GAT GAC TTA TTG ATT 30
Phe Phe Val Asn Gly Asp Asp Leu Leu Ile
1               5                   10

(2) INFORMATION FOR SEQ ID NO: 77:

(i) SEQUENCE CHARACTERISTICS:
(A) LENGTH: 10 amino acids
(B) TYPE: amino acid
(D) TOPOLOGY: linear

(ii) MOLECULE TYPE: protein
(xi) SEQUENCE DESCRIPTION: SEQ ID NO: 77:

Phe Phe Val Asn Gly Asp Asp Leu Leu Ile
1               5                   10

(2) INFORMATION FOR SEQ ID NO: 78:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: CDS
(B) LOCATION:1..30
(D) OTHER INFORMATION:/note= "FIGURE 3B, PRSV"

(xi) SEQUENCE DESCRIPTION: SEQ ID NO: 78:

TTT TAT ATC AAT GGT GAT GAT CTC TGT ATT 30
Phe Tyr Ile Asn Gly Asp Asp Leu Cys Ile
1               5                   10

(2) INFORMATION FOR SEQ ID NO: 79:

(i) SEQUENCE CHARACTERISTICS:
(A) LENGTH: 10 amino acids
(B) TYPE: amino acid
(D) TOPOLOGY: linear

(ii) MOLECULE TYPE: protein
(xi) SEQUENCE DESCRIPTION: SEQ ID NO: 79:

Phe Tyr Ile Asn Gly Asp Asp Leu Cys Ile
1               5                   10

(2) INFORMATION FOR SEQ ID NO: 80:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: CDS
(B) LOCATION:1..30
(D) OTHER INFORMATION:/note= "FIGURE 3B, PSbMV"

(xi) SEQUENCE DESCRIPTION: SEQ ID NO: 80:

TAT TTT GCA AAT GGT GAT GAT CTA TTG ATA                30
Tyr Phe Ala Asn Gly Asp Asp Leu Leu Ile
1               5                   10

(2) INFORMATION FOR SEQ ID NO: 81:

(i) SEQUENCE CHARACTERISTICS:
(A) LENGTH: 10 amino acids
(B) TYPE: amino acid
(D) TOPOLOGY: linear

(ii) MOLECULE TYPE: protein
(xi) SEQUENCE DESCRIPTION: SEQ ID NO: 81:

Tyr Phe Ala Asn Gly Asp Asp Leu Leu Ile
1               5                   10

(2) INFORMATION FOR SEQ ID NO: 82:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: CDS
(B) LOCATION:1..30
(D) OTHER INFORMATION:/note= "FIGURE 3B, TuMV"

(xi) SEQUENCE DESCRIPTION: SEQ ID NO: 82:

TTC TTC GTC AAC GGA GAC GAT TTA CTG CTA 30
Phe Phe Val Asn Gly Asp Asp Leu Leu Leu
1               5                   10

(2) INFORMATION FOR SEQ ID NO: 83:

(i) SEQUENCE CHARACTERISTICS:
(A) LENGTH: 10 amino acids
(B) TYPE: amino acid
(D) TOPOLOGY: linear

(ii) MOLECULE TYPE: protein
(xi) SEQUENCE DESCRIPTION: SEQ ID NO: 83:

Phe Phe Val Asn Gly Asp Asp Leu Leu Leu
1               5                   10

(2) INFORMATION FOR SEQ ID NO: 84:

(i) SEQUENCE CHARACTERISTICS:
(A) LENGTH: 14 amino acids
(B) TYPE: amino acid
(C) STRANDEDNESS: unknown
(D) TOPOLOGY: unknown

(ii) MOLECULE TYPE: protein

(ix) FEATURE:
(A) NAME/KEY: Protein
(B) LOCATION:1..14
(D) OTHER INFORMATION:/note= "FIGURE 4A, WMV2 USA"

(xi) SEQUENCE DESCRIPTION: SEQ ID NO: 84:

Gly Ser Phe Trp Ile His Trp Ile Thr Thr Gln Asp Gly Phe
1               5                   10

(2) INFORMATION FOR SEQ ID NO: 85:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 85:

TTCTGGATAC ATTGGATAAC GACT                                        24

(2) INFORMATION FOR SEQ ID NO: 86:

(i) SEQUENCE CHARACTERISTICS:
(A) LENGTH: 14 amino acids
(B) TYPE: amino acid
(C) STRANDEDNESS: unknown
(D) TOPOLOGY: unknown

(ii) MOLECULE TYPE: protein

(ix) FEATURE:
(A) NAME/KEY: Protein
(B) LOCATION:1..14
(D) OTHER INFORMATION:/note= "FIGURE 4A, Plum Pox Virus NAT"

(xi) SEQUENCE DESCRIPTION: SEQ ID NO: 86:

Ser His Phe Trp Lys His Trp Ile Ser Thr Lys Asp Gly His
1               5                   10

(2) INFORMATION FOR SEQ ID NO: 87:

(i) SEQUENCE CHARACTERISTICS:
(A) LENGTH: 24 base pairs
(B) TYPE: nucleic acid

(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 87:

TTCTGGAAAC ACTGGATTAG TACG 24

(2) INFORMATION FOR SEQ ID NO: 88:

(i) SEQUENCE CHARACTERISTICS:
(A) LENGTH: 14 amino acids
(B) TYPE: amino acid
(C) STRANDEDNESS: unknown
(D) TOPOLOGY: unknown

(ii) MOLECULE TYPE: protein

(ix) FEATURE:
(A) NAME/KEY: Protein
(B) LOCATION:1..14
(D) OTHER INFORMATION:/note= "FIGURE 4A, TVMV"

(xi) SEQUENCE DESCRIPTION: SEQ ID NO: 88:

Thr Ser Phe Trp Gln His Trp Ile Thr Thr Lys Asp Gly Gln
1               5                   10

(2) INFORMATION FOR SEQ ID NO: 89:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(xi) SEQUENCE DESCRIPTION: SEQ ID NO: 89:

TTCTGGCAAC ACTGGATAAC AACA 24

(2) INFORMATION FOR SEQ ID NO: 90:

(i) SEQUENCE CHARACTERISTICS:
(A) LENGTH: 14 amino acids
(B) TYPE: amino acid
(C) STRANDEDNESS: unknown
(D) TOPOLOGY: unknown

(ii) MOLECULE TYPE: protein

(ix) FEATURE:
(A) NAME/KEY: Protein
(B) LOCATION:1..14
(D) OTHER INFORMATION:/note= "FIGURE 4A, TEV"

(xi) SEQUENCE DESCRIPTION: SEQ ID NO: 90:

Gly Ile Phe Trp Lys His Trp Ile Gln Thr Lys Asp Gly Gln
1               5                   10

(2) INFORMATION FOR SEQ ID NO: 91:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(xi) SEQUENCE DESCRIPTION: SEQ ID NO: 91:

TTCTGGAAGC ATTGGATTCA AACC                                        24

(2) INFORMATION FOR SEQ ID NO: 92:

(i) SEQUENCE CHARACTERISTICS:
(A) LENGTH: 14 amino acids
(B) TYPE: amino acid
(C) STRANDEDNESS: unknown
(D) TOPOLOGY: unknown

(ii) MOLECULE TYPE: protein

(ix) FEATURE:
(A) NAME/KEY: Protein
(B) LOCATION:1..14
(D) OTHER INFORMATION:/note= "FIGURE 4A, PVY"

(xi) SEQUENCE DESCRIPTION: SEQ ID NO: 92:

Ser Thr Phe Trp Lys His Trp Ile Glu Thr Asp Asn Gly His
1               5                   10

(2) INFORMATION FOR SEQ ID NO: 93:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION:   /desc = "synthetic DNA"

(xi) SEQUENCE DESCRIPTION: SEQ ID NO: 93:

TTCTGGAAGC ATTGGATTGA AACA                                        24

(2) INFORMATION FOR SEQ ID NO: 94:

(i) SEQUENCE CHARACTERISTICS:
(A) LENGTH: 14 amino acids
(B) TYPE: amino acid
(C) STRANDEDNESS: unknown
(D) TOPOLOGY: unknown

(ii) MOLECULE TYPE: protein

(ix) FEATURE:
(A) NAME/KEY: Protein
(B) LOCATION:1..14
(D) OTHER INFORMATION:/note= "FIGURE 4A, PRSV"

(xi) SEQUENCE DESCRIPTION: SEQ ID NO: 94:

Gly Asp Phe Gly Cys His Trp Ile Ser Thr Asn Asp Gly Asp
1               5                   10

(2) INFORMATION FOR SEQ ID NO: 95:

(i) SEQUENCE CHARACTERISTICS:
(A) LENGTH: 14 amino acids
(B) TYPE: amino acid
(C) STRANDEDNESS: unknown

(D) TOPOLOGY: unknown

(ii) MOLECULE TYPE: protein

(ix) FEATURE:
(A) NAME/KEY: Protein
(B) LOCATION:1..14
(D) OTHER INFORMATION:/note= "FIGURE 4B, PSbMV"

(xi) SEQUENCE DESCRIPTION: SEQ ID NO: 95:

Gly Lys Tyr Trp Lys His Trp Ile Thr Thr Lys Glu Gly His
1               5                   10

(2) INFORMATION FOR SEQ ID NO: 96:

(i) SEQUENCE CHARACTERISTICS:
(A) LENGTH: 14 amino acids
(B) TYPE: amino acid
(C) STRANDEDNESS: unknown
(D) TOPOLOGY: unknown

(ii) MOLECULE TYPE: protein

(ix) FEATURE:
(A) NAME/KEY: Protein
(B) LOCATION:1..14
(D) OTHER INFORMATION:/note= "FIGURE 4B, TuMV"

(xi) SEQUENCE DESCRIPTION: SEQ ID NO: 96:

Ser Gln Phe Trp Lys His Trp Ile Ser Thr Lys Asp Gly Gln
1               5                   10

(2) INFORMATION FOR SEQ ID NO: 97:

(i) SEQUENCE CHARACTERISTICS:
(A) LENGTH: 2975 base pairs
(B) TYPE: nucleic acid
(C) STRANDEDNESS: single
(D) TOPOLOGY: linear

(ii) MOLECULE TYPE: cDNA to mRNA

(ix) FEATURE:
(A) NAME/KEY: misc_feature
(B) LOCATION:1..2975
(D) OTHER INFORMATION:/note= "FIGURES 5(a) to 5(d)"

(xi) SEQUENCE DESCRIPTION: SEQ ID NO: 97:

GAATTCTAGA TTCTGGAAAC ATTGGATAAC CACTCAAGAT GGTTTTTGTG GGCTACCTCT 60

CGTGTCTGTC AATGATGGAT ATATTGTTGG TATACATGGA TTAACATCTA ATGATTCAGA 120

GAAGAATTTC TTTGTTCCAT TTACTGACGG ATTTGAAACG GAATACTTGA ACAATGCTGA 180

TAATCTGTCG TGGGACAAAC ACTGGTTTTG GGAACCAAGT AAGATCGCAT GGGGCTCTTT 240

GAATTTAGTA GAAGAACAGC CAAAGGAAGA ATTCAAGATA TCAAAACTCG TGTCTGATCT 300

TTTTGGCAAC ACGGTAGCAG TACAGAGTAG AAAAGAGAGA TGGGTTTTGG ATGCCATGGA 360

AGGGAATTTG ATAGCTTGTG GGCAAGCTGA TGGCGCACTA GTGACAAAGC ACGTGGTTAA 420

AGGAAAATGC CCTCATTTTG CACAATATCT TTCGTTACAT GATGAAGCAA AACAGTTCTT 480

TGAGCCATTG ATGGGGGCAT ACCAGCCAAG TCGGTTGAAT AAAGACGCTT TCAAGAAAGA 540

TTTCTTTAAG TACAACAAAC CGGTTGTTCT GAATGAAGTT GATTTTAATG CTTTCGAGAA 600

GGCAGTCGAG GGAGTGATAA CAATGATAGT CGACTTTGAA TTTGCAGAAT GCTTATTTGT 660

GACTGATCCT GATGAGATTT ATGGATCCCT GAACATGAAA GCTGCTGTCG GTGCTCAATA 720

CAAAGGAAAG AAACAGGATT ACTTTTCTGG CATGGATAGT TTTGATAAGG AGCGCCTGTT 780

GTACCTTAGC TGTGAGAGGC TTTTTAATGG AGAGAAAGGA ATTTGGAACG GTTCCTTGAA 840

AGCGGAGTTG AGGCCAATTG AAAAAGTGCA GGCAAACAAA ACTAGAACAT TTACAGCAGC 900

ACCGATTGAT ACATTGCTTG GAGCTAAAGT TTGCGTCGAT GATTTTAATA ATCAATTCTA 960

CAGCTTCAAC TTAAAATGTC CATGGACAGT TGGCATGACT AAGTTTTATG GGGGTTGGGA 1020

TAAGCTAATG AGGAGTTTGC CTGATGGCTG GACATATTGC CACGCAGACG GTTCACAGTT 1080

CGACAGTTCT TTGACTCCCT TATTGTTGAA TGCTGTCCTC AGTATTAGAT GTTGCTTCAT 1140

GGAAGATTGG TGGGTTGGAA AGGAAATGCT TGAAAACCTT TACGCTGAGA TAGTTTACAC 1200

ACCAATCTTA GCACCTGATG GCACAATTTT CAAAAAGTTT AGAGGGAATA ACAGTGGACA 1260

GCCATCTACA GTTGTTGACA ATACGCTTAT GGTTGTCATT GCTATGTATT ACTCGTGCTG 1320

TAAACAAGGT TGGTCAGAGG AGGATATTGA AGAAAGGTTG GTGTTCTTTG CTAATGGTGA 1380

TGATATTATC TTGGCAGTTA GGGATGAGGA TGTGTGGCTG TATGACACTC TAAGTGCTTC 1440

ATTCGCTGAG CTTGGCCTGA ATTATAACTT CGATGAGCGG ACAAAGAAAA GAGAGGAGCT 1500

ATGGTTCATG TCACACCAAG CCATGCTAGT TGATGGGATT TACATTCCAA AGCTTGAACC 1560

TGAAAGGATA GTTTCCATCT TAGAGTGGGA TAGGAGCAAG GAACTTACGC ATCGCACTGA 1620

GGCAATTTGT GCAGCCATGA TTGAGGCATG GGGACATACT GAGCTTTTGC AGGAAATTCG 1680

CAAATTTTAT TTGTGGCTTC TAAGCAAAGA TGAATTTAAG GAACTCGCTG CATCCGGAAA 1740

AGCTCCATAC ATAGCTGAGA CAGCATTGAG AAAGCTCTAC ACAGATGTTA ACACACAACC 1800

AAGTGAATTG CAAAAATACC TTGAAGTTTT GGATTTTAAC CACATTGATG GTTGCTGTGA 1860

ATCAGTGTCT CTGCAATCAG GAAAAGAAGC AGTAGAAAAT TTGGATGCAG GGAAGGACTC 1920

GAAGAAGGAC ACCAGTGGCA AAGGGGATAA GCCACAAAAT TCGCAAACTG GTCAAGGTAG 1980

CAAAGAACAG ACAAAAACTG GTACAGTCAG CAAAGATGTG AATGTTGGAT CGAAAGGAAA 2040

AGAAGTCCCA CGACTACAAA AGATAACAAA GAAAATGAAC CTTCCAACAG TTGGTGGGAA 2100

AATCATTCTT AGCTTAGACC ATTTACTCGA GTACAAACCT AATCAAGTTG ATCTGTTTAA 2160

CACTCGAGCA ACAAAAACAC AGTTTGAATC ATGGTACAGC GCAGTTAAAG TTGAATACGA 2220

TCTTAACGAT GAGCAAATGG GTGTGATTAT GAATGGTTTT ATGGTTTGGT GTATCGATAA 2280

CGGTACATCT CCAGATGTCA ATGGAGTTTG GGTGATGATG GATGGGGAAG AGCAAGTTGA 2340

GTATCCATTA AAGCCAATTG TTGAAAATGC GAAACCAACT TTAAGACAAA TCATGCACCA 2400

TTTCTCAGAC GCAGCAGAAG CATATATTGA AATGAGAAAC TCTGAAAGTC CGTATATGCC 2460

TAGATACGGA TTACTAAGAA ATTTGAGAGA CAGGGAATTA GCACGCTATG CTTTTGACTT 2520

TTATGAGGTT ACTTCCAAAG CACCTAATAG GGCAAGAGAA GCAATAGCAC AAATGAAGGC 2580

CGCAGCTCTC GCGGGAATTA ACAGCAGGTT ATTTGGGCTT GATGGTAATA TCTCGACCAA 2640

TTCCGAAAAT ACTGAGAGGC ACACTGCAAG GGACGTGAAT CAGAATATGC ATACTTTGTT 2700

GGGTATGGGT CCACCGCAGT AAAGACTAGG TAAACTGGTC ACAGTTAGCA TTTCGGGTCG 2760

TTATAAGTTT TCTATAATAT AATATGTTGC ACTTTTTAGT ATAGTGTGAT TTTATCACCT 2820

TTGTACTGTT TATGTTAGTG TGGTTTAACC ATCTTAGTGT GCTTTATATT ATAGTTTATG 2880

CATAACAGGG AGAACCATTA CAATACCGGA GTTGTTTGTA GTGTGATTAC ATCACGGTTA 2940

ATAGCCGAGG TACGGTAATG TTTGTTGTCC TAAAA 2975

(2) INFORMATION FOR SEQ ID NO: 98:
(i) SEQUENCE CHARACTERISTICS:
(A) LENGTH: 2974 base pairs
(B) TYPE: nucleic acid
(C) STRANDEDNESS: single
(D) TOPOLOGY: linear

(ii) MOLECULE TYPE: cDNA to mRNA

(ix) FEATURE:
(A) NAME/KEY: misc_feature
(B) LOCATION:1..2974
(D) OTHER INFORMATION:/note= "FIGURE 5 bis (a) to (d)"

(xi) SEQUENCE DESCRIPTION: SEQ ID NO: 98:

GAATTCTAGA TTCTGGAAAC ATTGGATAAG CACTCAAGAT GGTTTTTGTG GGCTACCTCT 60

CGTGTCTGTC AATGATGGAT ATATTGTTGG TATACATGGA TTGACATCTA ATGATTCAGA 120

GAAGAACTTC TTTGTTCCAT TTACTGACGG ATTTGAAACG GAATACCTGA ACAATGCTGA 180

TAATCTGTCG TGGGACAAAC ACTGGTTTTG GGAACCAAGT AAGATCGCAT GGGGCTCTTT 240

GAATTTAGTA GAAGAACAGC CAAAGGAAGA ATTCAAGATA TCAAAACTCG TGTCTGATCT 300

TTTTGGCAAC ACGGTAGCAG TACAGAGTAG AAAAGAGAGA TGGGTTTTGG ATGCCATGGA 360

AGGGAATTTG ATAGCTTGTG GGCAAGCTGA TAGCGCACTA GTGACAAAGC ACGTGGTTAA 420

AGGAAAATGC CCTCATTTTG CACAATACCT TTCGTTACAT GATGAAGCAA AACAGTTCTT 480

TGAGCCATTG ATGGGGGCAT ACCAGCCAAG TCGGTTGAAT AAAGACGCTT TCAAGAAAGA 540

TTTCTTTAAA TACAACAAAC CGGTTGTTCT GAATGAAGTT GATTTTAATG CTTTCGAGAA 600

GGCAGTCGAG GGAGTGGTAA CAATGATGGT CGACTTTGAA TTTGCAGAAT GCTTATTTGT 660

GACTGATCCT GATGAGATTT ATGGATCCCT GAACATGAAA GCTGCTGTCG GTGCTCAATA 720

CAAAGGAAAG AAACAGGATT ACTTTTCTGG CATGGATAGT TTTGATAAGG AGCGCCTGTT 780

GTACCTTAGC TGTGAGAGGC TTTTTAACGG GGAGAAAGGA ATTTGGAATG GTTCCTTGAA 840

AGCGGAGTTG AGGCCAATTG AAAAAGTGCA GGCGAACAAA ACTAGAACAT TTACAGCAGC 900

ACCAATTGAC ACATTGCTTG GAGCTAAAGT TTGCGTCGAT GATTTTAATA ATCAATTCTA 960

TAGCTTCAAC TTAAAATGTC CATGGACAGT TGGCATGACT AAGTTTTATG GGGGTTGGGA 1020

TAAGCTAATG AGGAGTTTGC CTGATGGCTG GACATATTGC CACGCAGACG GTTCACAGTT 1080

CGACAGTTCT TTGACTCCCT TATTGTTGAA TGCTGTCCTC AGTATTAGAT GTTGCTTCAT 1140

GGAAGATTGG TGGGTCGGAA AGGAAATGCT CGAAAACCTT TACGCTGAGA TAGTTTACAC 1200

ACCAATCTTA GCACCTGATG GCACAATTTT CAAAAAGTTT AGAGGGAATA ACAGTGGACA 1260

GCCATCTACA GTTGTTGACA ATACGCTTAT GGTTGTCATT GCTATGTATT ACTCGTGCTG 1320

TAAGCAAGGT TGGTCAGAGA AGGATATTGA AGAAAGGTTG GTGTTCTTTG CTAATGGTGA 1380

TGATATTATC TTGGCAGTTA GGGACGAGGA TGTGTGGCTG TATGACACTC TAAGTGCTTC 1440

ATTCGCTGAG CTTGGCCTGA ATTATAACTT CGATGAGCGG ACAAAGAAAA GAGAGGAGCT 1500

ATGGTTTATG TCACACCAAG CCATGCTAGT TGATGGGATT TATATTCCAA AGCTTGAACC 1560

TGAAAGGATA GTTTCCATCT TAGAGTGGGA TAGGAGCAAG GAACTTATGC ATCGCACTGA 1620

GGCAATTTGT GCAGCCATGA TTGAGGCATG GGGACATACT GAGCTTTTGC AGGAAATTCG 1680

CAAATTTTAT TTGTGGCTTC TAAGCAAAGA TGAATTTAAG GAACTCGCTG CATCCGGAAA 1740

AGCTCCATAC ATAGCTGAGA CAGCATTGAG AAAGCTCTAC ACAGATGTTA ACACACAACC 1800

AAGTGAATTG CAAAAATACC TTGAAGTTTT GGATTTTAAC CACATTGATG GTTGTTGTGA 1860

ATCAGTGTCT CTGCAATCAG GAAAAGAAGC AGTAGAAAAT TTGGATGCAG GGAAGGACTC 1920

GAAGAAGGAC ACCAGTGGCA AAGGGGATAA GCCACAAAAC TCGCAAACTG ATCAAGGTAG 1980

CAAAGAACAG ACAAAAACTG GCACAGTCAG CAAAGATGTG AATGTTGGAT CGAAAGGAAA 2040

AGAAGTCCCA CGACTACAAA AGATAACAAA GAAAATGAAC CTTCCAACAG TTGGTGGGAA 2100

AATCATTCTT AGCTTAGACC ATTTGCTCGA GTACAAACCT AATCAAGTTG ATCTGTTTAA 2160

CACTCGAGCA ACAAAAACAC AGTTTGAGTC ATGGTACAGC GCAGTTAAAG TTGAATATGA 2220

TCTTAACGAT GAGCAAATGG GTGTGATTAT GAATGGTTTT ATGGTTTGGT GTATCGATAA 2280

CGGTACATCT CCAGATGTCA ATGGAGTTTG GGTGATGATG GATGGGGAAG AGCAAGTTGA 2340

GTATCCATTA AAGCCAATTG TTGAAAATGC AAAACCAACT TTAAGACAAA TCATGCACCA 2400

TTTCTCAGAC GCAGCAGAAG CATATATTGA AATGAGAAAC TCTGAAAGTC CGTATATGCC 2460

TAGATACGGA TTACTAAGAA ATTTGAGAGA CAGGGAATTA GCACGCTATG CTTTTGACTT 2520

TTATGAGGTT ACTTCCAAAG CACCTAATAG GGCAAGAGAA GCAATAGCAC AAATGAAGGC 2580

CGCAGCTCTC GCGGGAATTA ACAGCAGGTT ATTTGGGCTT GATGGTAATA TCTCGACCAA 2640

TTCCGAAAAT ACTGAGAGGC ACACTGCAAG GGACGTGAAT CAGAATATGC ATACTTTGTT 2700

AGGTATGGGT CCACCGCAGT AAAGACTAGG TAAACTGGTC ACAGTTAGCA TTTCGGGTCG 2760

TTACAAGTTT TCTATAATAT AATATGTTGC ACTTTTTAGT ATAGTGTGAT TTTATCACCT 2820

TTGTACTGTT TATGTAGTGT GGTTTAACCA CCTTAGTGTG CTTTATATTA TAGTTTATGC 2880

ATAACAGGGA GAACCATTGC AATACCGGAG TTGTTTGTAG TGTGATTACA TCACGTTTAA 2940

TAGCCGAGGT ACGGTAATGT TTGTTGTCCT AAAA 2974

(2) INFORMATION FOR SEQ ID NO: 99:

(i) SEQUENCE CHARACTERISTICS:
(A) LENGTH: 2922 base pairs
(B) TYPE: nucleic acid

(C) STRANDEDNESS: single
(D) TOPOLOGY: linear

(ii) MOLECULE TYPE: cDNA to mRNA

(ix) FEATURE:
(A) NAME/KEY: misc_feature
(B) LOCATION:1..2922
(D) OTHER INFORMATION:/note= "FIGURE 6(a) to (d)"

(xi) SEQUENCE DESCRIPTION: SEQ ID NO: 99:

TCTAGATTCT GGAAACATTG GATAACACCA ACGAAGGGGA TTGCGGATTG CCCATGGTTT 60

CAACAACGGA TGGCAAGATA ATTGGAGTTC ATGGTTTGGC TTCCACAGTC TCATCTAAGA 120

ATTATTTTGT CCCATTCACT GATGATTTTA TAGCCACGCA TTTGAGCAAA CTTGATGACC 180

TCACATGGAC TCAGCATTGG CTATGGCAAC CTAGCAAAAT TGCGTGGGGA ACGCTCAACT 240

TAGTTGATGA ACAACCAGGG CCCGAATTTC GTATTTCAAA TCTAGTCAAG GATTTATTCA 300

CTTCTGGTGT TGAAACACAG AGCAAGCGAG AAAGATGGGT CTACGAAAGC TGTGAAGGGA 360

ACCTTCGGGC TGTTGGAACT GCACAATCAG CGTTAGTCAC CAAACATGTT GTGAAAGGCA 420

AGTGTCCTTT CTTCGAAGAA TATTTACAAA AACACGCAGA AGCGAGCGCC TATTTCAGAC 480

CCCTAATGGG AGAGTACCAG CCGAGCAAGT TGAACAAAGA AGCCTTTAAA AAGGATTTCT 540

TTAAATACAA TAAACCCGTC ACTGTTAATC AACTGGATCA TGATAAATTT TTGGAAGCAG 600

TGGATGGGGT TATACGTATG ATGTGTGACT TTGAGTTCAA CGAATGTCGA TTCATTACAG 660

ATCCCGAGGA GATTTACAAC TCTTTGAACA TGAAAGCAGC AATTGGAGCC CAGTATAGAG 720

GAAAGAAGAA AGAGTTTTTG AGGGGCTAGA TGATTTTGAT CGAGAGCGAC TTTTATTCCA 780

AAGTTGTGAA AGGTTGTTCA ATGGCTACAA AGGTCTGTGG AATGGATCTT TAAAGGCCGA 840

GCTCAGGCCG CTTGAGAAAG TCAGGGCTAA CAAAACACGA ACCTTTACAG CAGCGCCAAT 900

TGATACATTG CTTGGAGCTA AAGTTTGTGT GGATGATTTC AACAATGAGT TCTACAGGAA 960

AAACCTCAAG TGTCCATGGA CGGTCGGCAT GACAAAATTT TATGGTAGTT GGGATAAATT 1020

GATGAGATCA TTACCTGATG GTTGGTTGTA TTGTCATGCT GATGGATCAC AGTTCGATAG 1080

TTCGTTAACC CCAGCCTTAC TGAACGCAGT GCTCATAATC AGGTCATTTT ATATGGAGGA 1140

TTGGTGGGTT GGCCAAGAGA TGCTTGAAAA TCTTTATGCC GAGATTGTGT ACACTCCAAT 1200

TCTTGCTCCT GATGGAACAA TTTTCAAGAA ATTTAGAGGT AACAACAGTG GGCAACCCTC 1260

AACAGTGGTG GATAACACAC TAATGGTTGT GATCTCTATT TACTATGCGT GCATGAAATT 1320

TGGTTGGAAC TGCGAGGAGA TTGAGAATAA ACTTGTCTTC TTTGCAAATG GGGATGATCT 1380

GATACTTGCA GTCAAAGATG AGGATAGCGG CTTACTTGAT AACATGTCAT CCTCTTTTTG 1440
CGAACTTGGA CTGAATTATG ATTTTTCAGA ACGTACGCAT AAAAGAGAAG ATCTTTGGTT 1500
CATGTCCCAC CAAGCAATGC TAGTTGATGG AATGTACATT CCAAAACTCG AGAAAGAGAG 1560
AATTGTTTCA ATTCTAGAGT GGGATAGAAG CAAAGAAATT ATGCACCGAA CAGAGGCTAT 1620
TTGCGCTGCG ATGATTGAGG CATGGGGGCA CACCGAGCTC TTGCAAGAAA TCAGAAAGTT 1680
TTACCTATGG TTCGTTGAAA AAGAAGAAGT GCGAGAATTG GCAGCCATCG GGAAAGCTCC 1740
ATACATAGCT GAGACAGCAC TTCGTAAGTT ATACACTGAC AAGGGAGCAG ATACAAGTGA 1800
ACTGGCACGC TACCTACAAG CCCTCCATCA AGACATCTTC TTTGAACAAG GAGACACTGT 1860
GATGCTCCAA TCAGGCACTC AGCCAACTGT GGCAGATGCT GGAGCTACAA AGAAAGACAA 1920
AGAAGATGAC AAAGGGAAAA ACAAGGACGT TACAGGCTCT GGCTCAGGTG AGAAAACAGT 1980
AGCAGCTGTC ACGAAGGACA AGGATGTGAA TGCTGGTTCT CATGGGAAAA TTGTGCCGCG 2040
TCTTTCGAAG ATCACAAAGA AAATGTCATT GCCACGCGTG AAAGGAAATG TGATACTCGA 2100
TATTGATCAT TTGCTGGAAT ATAAACCGGA TCAAATTGAG TTATATAACA CACGAGCGTC 2160
TCATCAGCAG TTCGCCTCTT GGTTCAACCA GGTTAAGACG GAATATGATT TGAACGAGCA 2220
ACAGATGGGA GTTGTAATGA ATGGTTTCAT GGTTTGGTGT ATTGAAAATG GCACTTCACC 2280
CGACATTAAT GGAGTGTGGG TTATGATGGA CGGAAATGAG CAAGTTGAGT ATCCCTTGAA 2340
ACCAATAGTT GAAAATGCAA AGCCAACGCT GCGGCAAATA ATGCATCATT TTTCAGATGC 2400
AGCGGAGGCA TATATAGAGA TGAGAAATGC AGAGGCACCA TACATGCCGA GGTATGGTTT 2460
GCTTCGAAAC CTACGGGATA GGAGTTTAGC ACGATATGCT TTCGATTTCT ATGAAGTCAA 2520
TTCTAAAACT CCTGAAAGAG CCCGCGAAGC TGTTGCGCAG ATGAAAGCAG CAGCTCTTAG 2580
CAATGTTTCT TCAAGGTTGT TTGCCTTGAT GGAAATGTTG CCACCACTAG TGAAGACACT 2640
GAACGGCACA CTGCACGTGA TGTTAATAGA AACATGCACA CCTTACTAGG TGTGAATACA 2700
ATGCAGTAAA GGGTAGGCCG CCTACCTAGG TTATTGTTTC GCTGCCGACG TAATTCTAAT 2760
ATTTACCGCT TTATTTGATA TCTTTAGATT TCCAGAGTGG GCCTCCCACC TTTAAAGCGT 2820
AAAGTTTATG TTAGTTGTCC AGGAGTGCCG TAGTCCTGTC GGAAGCTTTA GTGTGAGCCT 2880
CTCACGAATA AGCTCGAGAT TAGACTCCGT TTGAAGCCTA AA 2922

(2) INFORMATION FOR SEQ ID NO: 100:
(i) SEQUENCE CHARACTERISTICS:
(A) LENGTH: 236 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..236
(D) OTHER INFORMATION:/note= "FIGURE 7A"

(xi) SEQUENCE DESCRIPTION: SEQ ID NO: 100:

GAGCTCCACC GCGGTGGCGG CCGCTCTAGA ACTAGTGGAT CCATGTCATT TTCTTTTCGC 60

GTCACCGCTC ATCAGGTTAT CTTTTGTCAG CTATGTTTCG CGTCACCGCT CATCAGTGGA 120

GCTTTTCCAG ATCTATAAAA CTTAGTTTCG CGTCACCGCT CATCAGATGC CAACTGCAGC 180

AGCTTTTTCG CGTCACCGCT CATCAGATGT TCAGGGACCT GCATCTGATC AAGCTT 236

(2) INFORMATION FOR SEQ ID NO: 101:

(i) SEQUENCE CHARACTERISTICS:
(A) LENGTH: 230 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..230
(D) OTHER INFORMATION:/note= "FIGURE 7B"

(xi) SEQUENCE DESCRIPTION: SEQ ID NO: 101:

GAGCTCCACC GCGGTGGCGG CCGCTCTAGA ACTAGTGGAT CCATGAATGG TTTTTCGCGT 60

CACCGCTCAT CAGATGGTTT GGTGCACCAT TTTTCGCGTC ACCGCTCATC AGTCAGACGC 120

AGATCTTTTG ACTTTTCGCG TCACCGCTCA TCAGTATGAG GTTACCACCG CAGTTTCGCG 180

TCACCGCTCA TCAGAAGACT AGGTAACCTG CATCTGATCA AGCTTGAATT 230

(2) INFORMATION FOR SEQ ID NO: 102:

(i) SEQUENCE CHARACTERISTICS:
(A) LENGTH: 348 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..348
(D) OTHER INFORMATION:/note= "FIGURE 7C"

(xi) SEQUENCE DESCRIPTION: SEQ ID NO: 102:

AAGCTTGATA TCGAATCAGA ATATGCATAC TTTGTTAGGT ATGGGTCCAC CGCAGTAAAG 60

ACTAGGTAAA CTGGTCACAG TTAGCATTTC GGGTCGTTAC AAGTTTTCTA TAATATAATA 120

TGTTGCACTT TTTAGTATAG TGTGATTTTA TCACCTTTGT ACTGTTTATG TAGTGTGGTT 180

TAACCACCTT AGTGTGCTTT ATATTATAGT TTATGCATAA CAGGGAGAAC CATTGCAATA 240

CCGGAGTTGT TTGTAGTGTG ATTACATCAC GTTTAATAGC CGAGGTACGG TAATGTTTGT 300

TGTCCTAAAA AAAAAAGGAT CCACTAGTTC TAGAGCGGCC GCCACCGC 348

(2) INFORMATION FOR SEQ ID NO: 103:

(i) SEQUENCE CHARACTERISTICS:
(A) LENGTH: 227 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "SYNTHETIC DNA"

(ix) FEATURE:
(A) NAME/KEY: misc_feature
(B) LOCATION:1..227
(D) OTHER INFORMATION:/note= "FIGURE 8A"

(xi) SEQUENCE DESCRIPTION: SEQ ID NO: 103:

GCGGCCGCTC TAGAACTAGT GGATCCATGC AATGTTTTTC GCGTCACCGC TCATCAGTTC 60

AAGGTGCCGA GGTTTCGCGT CACCGCTCAT CAGTGGTTTG CTTCATGGTT TCGCGTCACC 120

GCTCATCAGT GGTGCATACG AGCGTTTCGC GTCACCGCTC ATCAGTCATC AGCTCCACAG 180

TTTCGCGTCA CCGCTCATCA GTCATCTAAC TGCATCTGAT CAAGCTT 227

(2) INFORMATION FOR SEQ ID NO: 104:

(i) SEQUENCE CHARACTERISTICS:
(A) LENGTH: 226 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..226
(D) OTHER INFORMATION:/note= "FIGURE 8B"

(xi) SEQUENCE DESCRIPTION: SEQ ID NO: 104:

AAGCTTGATC AGATGCAGGC TGCTTTTTCG CGTCACCGCT CATCAGATCT GCGCCGGCAT 60

GTTTCGCGTC ACCGCTCATC AGTGGTGCCT CCCTTTGTTT CGCGTCACCG CTCATCAGAT 120

CTTCTTAGCT ATGTTTCGCG TCACCGCTCA TCAGTGGAGC TTCCCACTGT TTCGCGTCAC 180

CGCTCATCAG GTTACCTCAT GGATCCACTA GTTCTAGAGC GGCCGC 226

(2) INFORMATION FOR SEQ ID NO: 105:

(i) SEQUENCE CHARACTERISTICS:
(A) LENGTH: 220 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..220
(D) OTHER INFORMATION:/note= "FIGURE 8C"

(xi) SEQUENCE DESCRIPTION: SEQ ID NO: 105:

GGATCCATGC TTTGCATTTT CGCGTCACCG CTCATCAGTC AACTATCGAT GCCGAGGTTT 60

CGTCCTCACG GACTCATCAG TGGTTTGCTT TTCGTCCTCA CGGACTCATC AGGAAACCTA 120

CGCTGCAGCA ATGTTTTTCG TCCTCACGGA CTCATCAGTT CAAGGTTGTT TTCGCGTCAC 180

CGCTCATCAG GGCCTTGATG ACCTGCATCT GATCAAGCTT 220

(2) INFORMATION FOR SEQ ID NO: 106:

(i) SEQUENCE CHARACTERISTICS:
(A) LENGTH: 93 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..93
(D) OTHER INFORMATION:/note= "FIGURE 8D"

(xi) SEQUENCE DESCRIPTION: SEQ ID NO: 106:

GGATCCGCAC AATTTTTTCG CGTCACCGCT CATCAGCCAT GAGAACCAGC ATTTTCGCGT 60

CACCGCTCAT CAGACATCCT TGTGATCAAG CTT 93

(2) INFORMATION FOR SEQ ID NO: 107:

(i) SEQUENCE CHARACTERISTICS:
(A) LENGTH: 1054 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..1054
(D) OTHER INFORMATION:/note= "FIGURE 9(a) and (b)"

(xi) SEQUENCE DESCRIPTION: SEQ ID NO: 107:

TTATTTGCCG CAGCGTTGGC TTTGCATTTT CGTCCTCACG GACTCATCAG TCAACTATTG 60

GTTTCAAGGG ATACTCAACT TGCTCATTTC CGTCCATCAT AACCCACACT CCATTAATGT 120

CGGGTGAAGT GCCATTTTCA ATACACCAAA CCATGAAACC ATTCATTACA ACTCCCATCT 180

GTTGCTCGTT CAAATCATAT TCCGTCTTAA CCTGGTTGAA CCAAGAGGCG AACTGCTGAT 240

GAGACGCTCG TGTGTTATAT AACTCAATTT GATCCGGTTT ATATTCCAGC AAATGATCAA 300

TATCGAGTAT CACATTTCCT TTCACGCGTG GCAATGACAT TTTCTTTGTG ATCTTCGAAA 360

GACGCGGCAC AATTTTTTCG TCCTCACGGA CTCATCAGCC ATGAGAACCA GCATTTTCGT 420

CCTCACGGAC TCATCAGACA TCCTTGTCCT TCGTGACAGC TGCTACTGTT TTCTCACCTG 480

AGCCAGTCAT TTTTCAGATG CAGCGGAGGC ATATATAGAG ATGAGAAATG CAGAGGCACC 540

ATACATGCCG AGGTTTCGTC CTCACGGACT CATCAGTGGT TTGCTTTTCG TCCTCACGGA 600

CTCATCAGGA AACCTACGGG ATAGGAGTTT AGCACGATAT GCTTTCGATT TCTATGAAGT 660

CAATTCTAAA ACTCCTGAAA GAGCCCGCGA AGCTGTTGCG CAGATGAAAG CAGCAGCTCT 720

TAGCAATGTT TTTCGTCCTC ACGGACTCAT CAGTTCAAGG TTGTTTTCGT CCTCACGGAC 780

TCATCAGGGC CTTGATGGAA ATGTTGCCAC CACTAGTGAA GACACTGAAC GGCACACTGC 840

ACGTGATGTT AATAGAAACA TGCACACCTT ACTAGGTGTG AATACAATGC AGTAAAGGGT 900

AGGCCGCCTA CCTAGGTTAT TGTTTCGCTG CCGACGTAAT TCTAATATTT ACCGCTTTAT 960

TTGATATCTT TAGATTTCCA GAGTGGGCCT CCCACCTTTA AAGCGTAAAG TTTATGTTAG 1020

TTGTCCAGGA GTGCCGTAGT CCTGTCGGAA GCTT 1054

(2) INFORMATION FOR SEQ ID NO: 108:

(i) SEQUENCE CHARACTERISTICS:
(A) LENGTH: 239 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: misc_feature
(B) LOCATION:1..239
(D) OTHER INFORMATION:/note= "FIGURE 18(-)"

(xi) SEQUENCE DESCRIPTION: SEQ ID NO: 108:

GTTTAAAATA AACACCGAAG ATTCGTTTCT ACTTAAATTC CTTGAGCGAC GTAGGCCTTT 60

TCGAGGTATG TATCGACTCT GTCGTAACTC TTTCGAGATG TGTCTACAAT TGTGTGTTGG 120

TTCACTTAAC GTTTTTATGG AACTTCAAAA CCTAAAATTG GTGTAACTAC CAACGACACT 180

TATCACAGAG ACGTTAGTCC TTTTCTTCGT CATCTTTTAA ACCTACGTCC CTTCCTGAG 239

(2) INFORMATION FOR SEQ ID NO: 109:

(i) SEQUENCE CHARACTERISTICS:
(A) LENGTH: 2005 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "synthetic DNA"

(ix) FEATURE:
(A) NAME/KEY: misc_feature
(B) LOCATION:1..2005
(D) OTHER INFORMATION:/note= "FIGURE 19"

(xi) SEQUENCE DESCRIPTION: SEQ ID NO: 109:

TATCAAAGCT CGTGTCTGAT CTTTTTGGCA ACACGGTAGC AGTACAGAGT AGAAAAGAGA 60

GATGGGTTTT GGACGCCATG GAAGGGAATT TGATAGCTTG TGGGCAAGCT GATAGCGCAC 120

TAGTGACAAA GCACGTGGTT AAAGGAAAAT GCCCTCATTT TGCACAATAT CTTTCGTTAC 180

ATGATGAAGC AAAACAGTTC TCTGAGCCAT TGATGGGGGC ATACCAGCCA AGTCGGTTGA 240

ATAAAGACGC TTTCAAGAAA GATTTCTTTA AGTACAACAA ACCGGTTGTT CTGAATGAAG 300

TTGATTTTAA TGCTCTCGAG AAGGCAGTCG AGGGAGTGAT AACAATGATG GTCGACTTTG 360

AATTTGCAGA ATGCTTATTT GTGACTGATC CTGATGAGAT TTATGGATCC CTGAACATCT 420

GATGAGTCCC GTGAGGACGA AAAAGCTGCT GTCGGTGCTC AATACAAAGG AAAGAAACAG 480

GATTACTTTT CTGGCATGGA TAGTTTTGAT AAGGAGCGCC TGTTGTACCT TAGCTGTGAG 540

AGGCTTTTTA ATGGAGAGAA AGGAATTTGG AACGGTTCCT TGAAAGCGGA GTTGAGGCCA 600

ATTGAAAAAG TGCAGGCAAA CAAAACTAGA ACATTTACAG CAGCACCGAT TGATACATTG 660

CTTGGAGCTA AAGTTTGCGT CGATGATTTT AATAATCAAT TCTACAGCTT CAACTTAAAA 720

TGTCCATGGA CAGTTGGATC TGATGAGTCC TGAGGACGAA ACTAAGTTTT ATGGGGGTTG 780

GGATAAGCTA ATGAGGAGTT TTGCCTGATG GCTGGACATA TTGCCACGCA GACGGTTCAC 840

AGTTCGACAG TTCTTTGACT CCCTTATTGT TGAATGCTGT CCTCAGTATT AGATGTTGCT 900

TCATGGAAGA TTGGTGGGTT GGAAAGGAAA TGCTTGAAAA CCTTTACGCT GAGATAGTTT 960

ACACACCAAT CTTAGCACCT GATGGCACAA TTTTCAAAAA GTTTAGAGGG AATAACAGTG 1020

GACAGCCATC TACAGTTGTT GACAATACGC TTATGGTTGT CATTGCTATG TATTACTCGT 1080

GCTGTAAACA AGGTTGGTCA GAGGAGGATA TTGAAGAAAG GTTGGTGTTC TTTGCTAATG 1140

GTGATGATAT TATCTTGGCA GTTAGGGATG AGGATGTGTG GCTGTATGAC ACTCTAAGTG 1200

CTTCATTCGC TGAGCTTGGC CTGAATTATA ACTTCGATGA GCGGACAAAG AAAAGAGAGG 1260

AGCTATGGTT CATGTCACAC CAAGCCATGC TAGTTGATGG GATTTACATT CCAAAGCTTG 1320

AACCTGAAAG GATAGTTTCC ATCTTAGAGT GGGATTGGAG CAAGGAACTT ATGCATCGCA 1380

CTGAGGCAAT TTGTGCAGCC ATGATTGAGG CATGGGGACA TACTGAGCTT TTGCAGGAAA 1440

TTCGCAAATT TTATTTGTGG CTTCTAAGCA AAGATGAATT TTAAGGAACT CGCTGCATCC 1500

GGAAAAGCTC CACTGATGAG TCCGTGAGGA CGAAACATAG CTGAGACAGC ATTGAGAAAG 1560

CTCTACACAG ATGTTAACAC ACAACCAAGT GAATTGCAAA AATACCTTGA AGTTTTGGAT 1620

TTTAACCACA TTGATGGTTG CTGTGAATCA GTGTCTCTGC AATCAGGAAA AGAAGCAGTA 1680

GAAAATTTGG ATGCAGGGAA GGACTCGAAG AAGGACACCA GTGGCAAAGG GGATAAGCCA 1740

CAAAATTCGC AAACTGGTCA AGGTAGCAAA GAACAGACAA AAACTGGTAC AGTCAGCAAA 1800

GATGTGAATG TTGGATCGAA AGGAAAAGAA GTCTCACGAC TACAAAAGAT AACCTGATGA 1860

GTCCGTGAGG ACGAAAAGAA AATGAACCTT CCAACAGTTG GTGGAAAAAT CATTCTTAGC 1920

TTAGCCATTT ACTCGAGTAC AAACCTAATC AAGTTGATCT TTAACACTCG AGCCCAGCTT 1980

CCACCATGGC GTGCAGGTCG ACGGA 2005